(19) Europäisches Patentamt
European Patent Office
Office européen des brevets

(11) EP 2 987 787 A1

(12) EUROPEAN PATENT APPLICATION
published in accordance with Art. 153(4) EPC

(43) Date of publication:
**24.02.2016 Bulletin 2016/08**

(21) Application number: **14785925.0**

(22) Date of filing: **18.04.2014**

(51) Int Cl.:
*C07D 215/22* (2006.01)
*A61K 31/4704* (2006.01)
*A61K 45/00* (2006.01)
*A61P 31/04* (2006.01)
*A61P 43/00* (2006.01)
*C07D 401/04* (2006.01)
*C07D 403/04* (2006.01)
*C07D 491/056* (2006.01)
*C07D 498/04* (2006.01)
*C07D 498/06* (2006.01)
*C12N 9/99* (2006.01)

(86) International application number:
**PCT/JP2014/061004**

(87) International publication number:
**WO 2014/171527 (23.10.2014 Gazette 2014/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **19.04.2013 JP 2013088008**
**05.09.2013 JP 2013184213**

(71) Applicant: **Juntendo Educational Foundation**
**Tokyo 113-8421 (JP)**

(72) Inventors:
- **HIRAMATSU Keiichi**
**Tokyo 113-8421 (JP)**
- **MORIMOTO Yuh**
**Tokyo 113-8421 (JP)**
- **BABA Tadashi**
**Tokyo 113-8421 (JP)**
- **HAYAKAWA Isao**
**Tokyo 113-8421 (JP)**

(74) Representative: **Godemeyer Blum Lenze Patentanwälte**
**Partnerschaft mbB - werkpatent**
**An den Gärten 7**
**51491 Overath (DE)**

# (54) NOVEL COMPOUND WITH ANTIBACTERIAL ACTIVITY

(57) A compound represented by the general formula (I) or a salt thereof having a potent antibacterial activity against bacteria that have acquired resistance to quinolones, and a medicament for prophylactic and/or therapeutic treatment of an infectious disease containing the compound or a salt thereof as an active ingredient, as well as a medicament for prophylactic and/or therapeutic treatment of an infectious disease containing a combination of the compound or a salt thereof, and a quinolone.

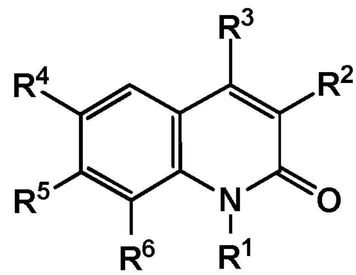

(I)

EP 2 987 787 A1

## Description

### Technical Field

The present invention relates to a novel compound or a salt thereof having a potent antibacterial activity against quinolone-resistant bacteria, and a medicament for prophylactic and/or therapeutic treatment of an infectious disease with a quinolone-resistant-bacterium which comprises said compound or a salt thereof as an active ingredient, and further relates to a medicament for prophylactic and/or therapeutic treatment of an infectious disease which comprises a combination of said compound or a salt thereof and a quinolone.

### Background Art

Quinolones are synthetic antibacterial agents that inhibit the activity of DNA gyrase in the religation step of cleaved DNA strands, thereby causing the DNA replication failure, and death of the bacteria. They have been widely used as drugs for humans as well as for animals.

As for *Staphylococcus aureus,* there are continuously increasing number of multiply antibiotic-resistant strains, of which typical example is the methicillin-resistant *Staphylococcus aureus* (MRSA) that acquired resistance to B-lactam antibacterial agents. It is no exaggeration to say that there is no hospital where MRSA does not exist, and it poses a serious clinical problem. Although quinolones are widely used for the treatment of infection by *Staphylococcus aureus,* many of MRSA strains are resistant to quinolones besides other antibacterial agents.

Vancomycin is available that has long maintained its effectiveness against MRSA,. However, the inventors of the present invention found vancomycin-intermediate *S. aureus* (VISA) in 1996 to which vancomycin therapy was ineffective (see, for example, Non-patent document 1). Since then, VISA has been found in various countries in the world. Moreover, vancomycin-resistant *S. aureus* (VRSA), which is MRSA highly resistant to vancomycin, was found in the U.S. in 2003, and at present, 9 strains in total have been reported from three countries; i.e., the U.S., Iran, and India (see, Non-patent document 2). The quinolones are ineffective also against most of VISA and VRSA strains (see, for example, Non-patent document 3).

Therefore, development of new antibacterial agents that have superior antibacterial activity against quinolone-resistant *Staphylococcus aureus* including MRSA, VISA, and VRSA, as well as those against other quinolone-resistant Gram-positive bacteria is currently strongly desired.

It is understood that the resistance against quinolones is acquired through introduction of mutations at several specific positions of DNA gyrase as the target enzyme of quinolones, which reduce the DNA gyrase inhibitory action of quinolones. As an antibacterial agent effective to such a mutated DNA gyrase, nybomycin is known (refer to Non-patent document 3). Although the antibacterial activity of nybomycin against quinolone-susceptible bacteria is weak, nybomycin shows notably potent antibacterial activity against bacteria that have acquired resistance to quinolones (resistant bacteria having a mutant DNA gyrase including a mutation such as Ser84Leu) (refer to Non-patent document 3). Further, it has also been reported that reversion occurs in DNA gyrase in bacteria that have acquired resistance to nybomycin, and they become susceptible to quinolones (refer to Non-patent document 3). Therefore, if an antibacterial agent that has the property as that of nybomycin can be newly provided, it can be used in combination with a quinolone so that bacteria that have acquired resistance to the quinolone are effectively eliminated by the new antibacterial agent, and at the same time, bacteria that have acquired resistance to the antibacterial agent can be eliminated with the quinolone, and therefore it is expected that an extremely effective treatment of infectious diseases can be achieved by preventing emergence of new resistant bacteria.

### Prior art references

#### Non-patent documents

Non-patent document 1: J. Antimicrob. Chemother., 40(1), pp.135-136, 1997
Non-patent document 2: Antimicrob. Agents Chemother., 53(11), pp.4580-4587, 2009
Non-patent document 3: Int. J. Antimicrob. Agents, 39(6), pp.478-485, 2012

Summary of the Invention

Object to be Achieved by the Invention

An object of the present invention is to provide a novel compound or a salt thereof that has a potent antibacterial activity against bacteria that have acquired resistance to quinolones.

More specifically, the object of the present invention is to provide a novel compound or a salt thereof that has a potent antibacterial activity against bacteria that have acquired resistance to quinolones due to the mutation of DNA gyrase, and a medicament for prophylactic treatment and/or therapeutic treatment of an infectious disease which comprises said compound or a salt thereof as an active ingredient.

Another object of the present invention is to provide an inhibitor that has an inhibitory action against DNA gyrase of quinolone-resistant bacteria, and further object of the present invention is to provide a medicament that can prevent emergence of resistant bacteria when it is used in combination with a quinolone.

Means for Achieving the Object

The following inventions are provided as means for achieving the aforementioned objects.

<1> A compound represented by the following general formula (I), or a salt thereof:

[Formula 1]

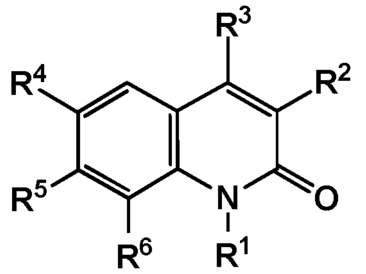

(I)

wherein, in the formula, $R^1$ represents a C1-C6 alkyl group which may have a substituent, a C1-C6 alkenyl group which may have a substituent, or an aryl group which may have a substituent, provided that $R^1$ may bind with $R^6$ to form a partially unsaturated or saturated heterocyclic ring containing one or two or more ring-constituting heteroatoms (the ring may have a substituent); $R^2$ represents hydrogen atom, carboxyl group, or amino group; $R^3$ represents hydroxy group, or a C1-C6 alkyl group which may have a substituent; $R^4$ represents hydrogen atom, a halogen atom, a C1-C6 alkyl group which may have a substituent, or a C1-C6 alkoxy group which may have a substituent, provided that $R^4$ may bind with $R^5$ to form a partially unsaturated or saturated heterocyclic ring containing one or two or more ring-constituting heteroatoms (the ring may have a substituent); $R^5$ represents a C1-C6 alkyl group which may have a substituent, a C1-C6 alkoxy group which may have a substituent, an aryl group which may have a substituent, a heteroaryl group which may have a substituent, or a partially saturated or saturated heterocyclic group which may have a substituent, provided that $R^5$ may bind with $R^6$ to form a partially unsaturated or saturated heterocyclic ring containing one or two or more ring-constituting heteroatoms (the ring may have a substituent); and $R^6$ represents hydrogen atom, a C1-C6 alkyl group which may have a substituent, a C1-C6 alkoxy group which may have a substituent, or a halogen atom.

<2> The compound or a salt thereof according to <1>, wherein, in the general formula (I), $R^1$ is a C1-C6 alkyl group which may have a substituent, provided that $R^1$ may bind with $R^6$ to form a saturated 5- to 7-membered ring containing one or two or more ring-constituting heteroatoms (the ring may have a substituent); $R^2$ is hydrogen atom, carboxyl group, or amino group; $R^3$ is hydroxy group, a C1-C6 alkyl group, a hydroxy(C1-C6 alkyl) group which may have a substituent, or an amino(C1-C6 alkyl) group which may have a substituent; $R^4$ is hydrogen atom, a halogen atom, or a C1-C6 alkyl group which may have a substituent; $R^5$ is an aryl group which may have a substituent, or a partially saturated or saturated heterocyclic group which may have a substituent, provided that $R^5$ may bind with $R^6$ to form a partially unsaturated or saturated 5- to 7-membered ring containing one or two or more ring-constituting heteroatoms (the ring may have a substituent); and $R^6$ is hydrogen atom, a C1-C6 alkyl group which may have a substituent, a C1-C6 alkoxy group which may have a substituent, or a halogen atom.

<3> The compound or a salt thereof according to <1>, wherein, in the general formula (I), $R^1$ is $-CH_3$, $-CH_2CH_3$, or cyclopropyl group, or may bind with $R^6$ to form a saturated 5- or 6-membered heterocyclic ring containing two ring-constituting heteroatoms (the ring may have one C1-C6 alkyl group as a substituent); $R^2$ is hydrogen atom, carboxyl group, or amino group; $R^3$ is hydroxy group, $-CH_3$, $-CH_2CH_3$, $-CH_2OH$, $-CH_2OCH_3$,$-CH_2NH_2$, $-C(O)NH_2$, $-CH_2OCOCH_3$, $-CH_2OCOCH_2CH_3$, $-CH_2OCOCH_2COOH_3$,$-CH_2OCOCH_2CH_2COOH$, $-CH_2OCH_3$, or $-CH_2ONH_2$; $R^4$ is hydrogen atom, fluorine atom, chlorine atom, or $-CH_3$; $R^5$ is phenyl group (the phenyl group may have one or two substituents selected from the group consisting of hydroxy group, amino group, a C1-C6 alkyl group, and aminomethyl group), pyridyl group (the pyridyl group may have one or two substituents selected from the group consisting of hydroxy group, amino group, a C1-C6 alkyl group, and aminomethyl group), piperazinyl group (the piperazinyl group may have one or two substituents selected from the group consisting of hydroxy group, amino group, a C1-C6 alkyl group, and aminomethyl group), piperidinyl group (the piperidinyl group may have one or two substituents selected from the group consisting of hydroxy group, amino group, a C1-C6 alkyl group, and aminomethyl group), or isoindolinyl group (the isoindolinyl group may have one or two substituents selected from the group consisting of hydroxy group, amino group, a C1-C6 alkyl group, and aminomethyl group, and may be partially saturated), or may bind with $R^6$ to form a saturated 5- or 6-membered heterocyclic ring containing two ring-constituting heteroatoms (the ring may have a C1-C6 alkyl group which may have a substituent as a substituent); and $R^6$ is hydrogen atom, a halogen atom, a C1-C6 alkyl group (the alkyl group may be substituted with fluorine atom), or a C1-C6 alkoxy group (the alkoxy group may be substituted with fluorine atom).

<4> An inhibitor against a DNA gyrase of a quinolone-resistant bacterium, which comprises the compound or a salt thereof according to any one of <1> to <3> mentioned above as an active ingredient.

<5> A medicament for prophylactic and/or therapeutic treatment of an infectious disease, which comprises the compound or a salt thereof according to any one of <1> to <3> mentioned above as an active ingredient.

<6> The medicament according to <5>, which is for prophylactic and/or therapeutic treatment of an infectious disease caused by a quinolone-resistant bacterium.

<7> An antibacterial agent comprising the compound or a salt thereof according to any one of <1> to <3> mentioned above.

<8> A medicament comprising a combination of the compound or a salt thereof according to any one of <1> to <3> mentioned above, and a quinolone.

<9> Use of the compound or a salt thereof according to any one of <1> to <3> for manufacture of the medicament according to any one of <5> to <8> mentioned above.

<10> A method for prophylactic and/or therapeutic treatment of an infectious disease of a mammal including human, which comprises the step of administering a prophylactically and/or therapeutically effective amount of the compound or a salt thereof according to any one of <1> to <3> to the mammal.

<11> The method according to <10>, wherein the infectious disease is infection with a quinolone-resistant bacterium.

<12> A method for prophylactic and/or therapeutic treatment of an infectious disease of a mammal including human, which comprises the step of simultaneously or separately administering a prophylactically and/or therapeutically effective amount of the compound or a salt thereof according to any one of <1> to <3>, and a prophylactically and/or therapeutically effective amount of a quinolone to the mammal.

Effect of the Invention

According to the present invention, there are provided a compound or a salt thereof that can show potent antibacterial activity against quinolone-resistant bacteria, and an inhibitor for DNA gyrase of quinolone-resistant bacteria comprising the compound or a salt thereof.

The action of the compound or a salt thereof of the present invention is similar to the action of nybomycin, and it can exhibit notably potent antibacterial activity against bacteria that have acquired resistance to quinolones (resistant bacteria containing a mutant DNA gyrase having a mutation such as Ser84Leu), although the antibacterial activity thereof against bacteria susceptible to quinolones is weaker. Further, in a bacterium that has acquired resistance to the compound or a salt thereof of the present invention, reverse mutation occurs in the DNA gyrase, and thus it becomes susceptible to quinolones. Therefore, with a combination of the compound or a salt thereof of the present invention and a quinolone, bacteria that have acquired resistance to quinolones are effectively eliminated with the antibacterial agent of the present invention, and at the same time, bacteria that have acquired resistance to the antibacterial agent of the present invention can be eliminated with the quinolone, and thus an extremely effective treatment of infectious diseases can be achieved by preventing emergence of new resistant bacteria.

Accordingly, the medicament of the present invention comprising a combination of the compound or a salt thereof of the present invention and a quinolone is useful for a prophylactic and/or therapeutic treatment of an infectious disease caused by either one or both of a quinolone-susceptible bacterium and a quinolone-resistant bacterium, and it can attain high prophylactic and/or therapeutic effect by preventing emergence of quinolone-resistant bacteria.

Brief Description of the Drawings

[Fig. 1] Fig. 1 shows the results of the DNA cleavage assay performed with GyrA(wt) and GyrB(wt) in the absence or presence of the compound J-131CP. "S" represents a substrate plasmid DNA having a superhelix structure, "L" represents linear double-stranded DNA formed by cleavage of the aforementioned substrate plasmid DNA with the DNA gyrase, and "N" represents double-stranded DNA in which one strand of the double-stranded substrate plasmid DNA was cleaved by the DNA gyrase. As a control, levofloxacin (LVX) was used.

[Fig. 2] Fig. 2 shows the results of the DNA cleavage assay performed with GyrA(S84L) and GyrB(wt) in the absence or presence of the compound J-131CP. "S", "L", and "N" have the same meanings as those explained for Fig. 1. As a control, levofloxacin (LVX) was used.

Modes for Carrying out the Invention

In this specification, the alkyl moieties of the "alkyl group" and "a substituent containing an alkyl moiety (alkoxy group and the like)" may be any of linear, branched, and cyclic alkyl groups, or a combination of these. Examples of the C1-C6 alkyl group include methyl group, ethyl group, n-propyl group, isopropyl group, n-butyl group, isobutyl group, sec-butyl group, tert-butyl group, n-pentyl group, isopentyl group, 2-methylbutyl group, 1-methylbutyl group, neopentyl group, 1,2-dimethylpropyl group, 1-ethylpropyl group, n-hexyl group, 4-methylpentyl group, 3-methylpentyl group, and the like, as well as a C3-C8 cycloalkyl group such as cyclopropyl, cyclobutyl, cyclopentyl, and cyclohexyl, cyclopropylmethyl group, cyclopropylbutyl group, and the like, but it is not limited to these examples.

In this specification, as the "alkenyl group", an alkyl group containing one or two or more double bonds can be used. Examples include, for example, vinyl group, prop-1-en-1-yl group, allyl group, isopropenyl group, but-1-en-1-yl group, but-2-en-1-yl group, but-3-en-1-yl group, 2-methylprop-2-en-1-yl group, 1-methylprop-2-en-1-yl group, pent-1-en-1-yl group, pent-2-en-1-yl group, pent-3-en-1-yl group, pent-4-en-1-yl group, 3-methylbut-2-en-1-yl group, 3-methylbut-3-en-1-yl group, hex-1-en-1-yl group, hex-2-en-1-yl group, hex-3-en-1-yl group, hex-4-en-1-yl group, hex-5-en-1-yl group, 2-cyclopropen-1-yl group, 2-cyclobuten-1-yl group, 2-cyclopenten-1-yl group, 3-cyclopenten-1-yl group, 2-cyclohexen-1-yl group, 3-cyclohexen-1-yl group, 1-cyclobuten-1-yl group, 1-cyclopenten-1-yl group, and the like, but it is not limited to these examples.

In this specification, examples of the "aryl group" include monocyclic or condensed polycyclic aromatic hydrocarbon groups, for example, phenyl group, 1-naphthyl group, 2-naphthyl group, anthryl group, phenanthryl group, acenaphthylenyl group, and the like.

In this specification, as the "halogen atom", fluorine atom, chlorine atom, bromine atom, or iodine atom can be used

In this specification, the "heteroaryl group" may be either of a monocyclic heteroaryl group and a condensed polycyclic heteroaryl group, and any aromatic group containing one or two or more ring-constituting heteroatoms may be used. As the "heteroatom", oxygen atom, sulfur atom, nitrogen atom, or the like can be used.

Examples of the "monocyclic heteroaryl group" include, for example, 5- to 7-membered monocyclic heteroaryl groups such as 2-furyl group, 3-furyl group, 2-thienyl group, 3-thienyl group, 1-pyrrolyl group, 2-pyrrolyl group, 3-pyrrolyl group, 2-oxazolyl group, 4-oxazolyl group, 5-oxazolyl group, 3-isoxazolyl group, 4-isoxazolyl group, 5-isoxazolyl group, 2-thiazolyl group, 4-thiazolyl group, 5-thiazolyl group, 3-isothiazolyl group, 4-isothiazolyl group, 5-isothiazolyl group, 1-imidazolyl group, 2-imidazolyl group, 4-imidazolyl group, 5-imidazolyl group, 1-pyrazolyl group, 3-pyrazolyl group, 4-pyrazolyl group, 5-pyrazolyl group, (1,2,3-oxadiazol)-4-yl group, (1,2,3-oxadiazol)-5-yl group, (1,2,4-oxadiazol)-3-yl group, (1,2,4-oxadiazol)-5-yl group, (1,2,5-oxadiazol)-3-yl group, (1,2,5-oxadiazol)-4-yl group, (1,3,4-oxadiazol)-2-yl group, (1,3,4-oxadiazol)-5-yl group, furazanyl group, (1,2,3-thiadiazol)-4-yl group, (1,2,3-thiadiazol)-5-yl group, (1,2,4-thiadiazol)-3-yl group, (1,2,4-thiadiazol)-5-yl group, (1,2,5-thiadiazol)-3-yl group, (1,2,5-thiadiazol)-4-yl group, (1,3,4-thiadiazolyl)-2-yl group, (1,3,4-thiadiazolyl)-5-yl group, (1H-1,2,3-triazol)-1-yl group, (1H-1,2,3-triazol)-4-yl group, (1H-1,2,3-triazol)-5-yl group, (2H-1,2,3-triazol)-2-yl group, (2H-1,2,3-triazol)-4-yl group, (1H-1,2,4-triazol)-1-yl group, (1H-1,2,4-triazol)-3-yl group, (1H-1,2,4-triazol)-5-yl group, (4H-1,2,4-triazol)-3-yl group, (4H-1,2,4-triazol)-4-yl group, (1H-tetrazol)-1-yl group, (1H-tetrazol)-5-yl group, (2H-tetrazol)-2-yl group, (2H-tetrazol)-5-yl group, 2-pyridyl group, 3-pyridyl group, 4-pyridyl group, 3-pyridazinyl group, 4-pyridazinyl group, 2-pyrimidinyl group, 4-pyrimidinyl group, 5-pyrimidinyl group, 2-pyrazinyl group, (1,2,3-triazin)-4-yl group, (1,2,3-triazin)-5-yl group, (1,2,4-triazin)-3-yl group, (1,2,4-triazin)-5-yl group, (1,2,4-triazin)-6-yl group, (1,3,5-triazin)-2-yl group, 1-azepinyl group, 1-azepinyl group, 2-azepinyl group, 3-azepinyl group, 4-azepinyl group, (1,4-oxazepin)-2-yl group, (1,4-oxazepin)-3-yl group, (1,4-oxazepin)-5-yl group, (1,4-oxazepin)-6-yl group, (1,4-oxazepin)-7-yl group, (1,4-thiazepin)-2-yl group, (1,4-thiazepin)-3-yl group, (1,4-thiazepin)-5-yl group, (1,4-thiazepin)-6-yl group, and (1,4-thiazepin)-7-yl group.

Examples of the "condensed polycyclic heteroaryl group" include, for example, 8- to 14-membered condensed

polycyclic heteroaryl groups such as 2-benzofuranyl group, 3-benzofuranyl group, 4-benzofuranyl group, 5-benzofuranyl group, 6-benzofuranyl group, 7-benzofuranyl group, 1-isobenzofuranyl group, 4-isobenzofuranyl group, 5-isobenzofuranyl group, 2-benzo[b]thienyl group, 3-benzo[b]thienyl group, 4-benzo[b]thienyl group, 5-benzo[b]thienyl group, 6-benzo[b]thienyl group, 7-benzo[b]thienyl group, 1-benzo[c]thienyl group, 4-benzo[c]thienyl group, 5-benzo[c]thienyl group, 1-indolyl group, 2-indolyl group, 3-indolyl group, 4-indolyl group, 5-indolyl group, 6-indolyl group, 7-indolyl group, (2H-isoindol)-1-yl group, (2H-isoindol)-2-yl group, (2H-isoindol)-4-yl group, (2H-isoindol)-5-yl group, (1H-indazol)-1-yl group, (1H-indazol)-3-yl group, (1H-indazol)-4-yl group, (1H-indazol)-5-yl group, (1H-indazol)-6-yl group, (1H-indazol)-7-yl group, (2H-indazol)-1-yl group, (2H-indazol)-2-yl group, (2H-indazol)-4-yl group, (2H-indazol)-5-yl group, 2-benzoxazolyl group, 2-benzoxazolyl group, 4-benzoxazolyl group, 5-benzoxazolyl group, 6-benzoxazolyl group, 7-benzoxazolyl group, (1,2-benzisoxazol)-3-yl group, (1,2-benzisoxazol)-4-yl group, (1,2-benzisoxazol)-5-yl group, (1,2-benzisoxazol)-6-yl group, (1,2-benzisoxazol)-7-yl group, (2,1-benzisoxazol)-3-yl group, (2,1-benzisoxazol)-4-yl group, (2,1-benzisoxazol)-5-yl group, (2,1-benzisoxazol)-6-yl group, (2,1-benzisoxazol)-7-yl group, 2-benzothiazolyl group, 4-benzothiazolyl group, 5-benzothiazolyl group, 6-benzothiazolyl group, 7-benzothiazolyl group, (1,2-benzoisothiazol)-3-yl group, (1,2-benzoisothiazol)-4-yl group, (1,2-benzoisothiazol)-5-yl group, (1,2-benzoisothiazol)-6-yl group, (1,2-benzoisothiazol)-7-yl group, (2,1-benzoisothiazol)-3-yl group, (2,1-benzoisothiazol)-4-yl group, (2,1-benzoisothiazol)-5-yl group, (2,1-benzoisothiazol)-6-yl group, (2,1-benzoisothiazol)-7-yl group, (1,2,3-benzoxadiazol)-4-yl group, (1,2,3-benzoxadiazol)-5-yl group, (1,2,3-benzoxadiazol)-6-yl group, (1,2,3-benzoxadiazol)-7-yl group, (2,1,3-benzoxadiazol)-4-yl group, (2,1,3-benzoxadiazol)-5-yl group, (1,2,3-benzothiadiazol)-4-yl group, (1,2,3-benzothiadiazol)-5-yl group, (1,2,3-benzothiadiazol)-6-yl group, (1,2,3-benzothiadiazol)-7-yl group, (2,1,3-benzothiadiazol)-4-yl group, (2,1,3-benzothiadiazol)-5-yl group, (1H-benzotriazol)-1-yl group, (1H-benzotriazol)-4-yl group, (1H-benzotriazol)-5-yl group, (1H-benzotriazol)-6-yl group, (1H-benzotriazol)-7-yl group, (2H-benzotriazol)-2-yl group, (2H-benzotriazol)-4-yl group, (2H-benzotriazol)-5-yl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 3-cinnolinyl group, 4-cinnolinyl group, 5-cinnolinyl group, 6-cinnolinyl group, 7-cinnolinyl group, 8-cinnolinyl group, 2-quinazolinyl group, 4-quinazolinyl group, 5-quinazolinyl group, 6-quinazolinyl group, 7-quinazolinyl group, 8-quinazolinyl group, 2-quinoxalinyl group, 5-quinoxalinyl group, 6-quinoxalinyl group, 1-phthalazinyl group, 5-phthalazinyl group, 6-phthalazinyl group, 2-naphthyridinyl group, 3-naphthyridinyl group, 4-naphthyridinyl group, 2-purinyl group, 6-purinyl group, 7-purinyl group, 8-purinyl group, 2-pteridinyl group, 4-pteridinyl group, 6-pteridinyl group, 7-pteridinyl group, 1-carbazolyl group, 2-carbazolyl group, 3-carbazolyl group, 4-carbazolyl group, 9-carbazolyl group, 2-(α-carbolinyl) group, 3-(α-carbolinyl) group, 4-(α-carbolinyl) group, 5-(α-carbolinyl) group, 6-(α-carbolinyl) group, 7-(α-carbolinyl) group, 8-(α-carbolinyl) group, 9-(α-carbolinyl) group, 1-(β-carbolinyl) group, 3-(β- carbolinyl) group, 4-(β-carbolinyl) group, 5-(β-carbolinyl) group, 6-(β-carbolinyl) group, 7-(β-carbolinyl) group, 8-(β-carbolinyl) group, 9-(β-carbolinyl) group, 1-(γ-carbolinyl) group, 2-(γ-carbolinyl) group, 4-(γ-carbolinyl) group, 5-(γ-carbolinyl) group, 6-(γ-carbolinyl) group, 7-(γ-carbolinyl) group, 8-(γ-carbolinyl) group, 9-(γ-carbolinyl) group, 1-acridinyl group, 2-acridinyl group, 3-acridinyl group, 4-acridinyl group, 9-acridinyl group, 1-phenoxazinyl group, 2-phenoxazinyl group, 3-phenoxazinyl group, 4-phenoxazinyl group, 10-phenoxazinyl group, 1-phenothiazinyl group, 2-phenothiazinyl group, 3-phenothiazinyl group, 4-phenothiazinyl group, 10-phenothiazinyl group, 1-phenadinyl group, 2-phenadinyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 2-phenanthrolinyl group, 3-phenanthrolinyl group, 4-phenanthrolinyl group, 5-phenanthrolinyl group, 6-phenanthrolinyl group, 7-phenanthrolinyl group, 8-phenanthrolinyl group, 9-phenanthrolinyl group, 10-phenanthrolinyl group, 1-thianthrenyl group, 2-thianthrenyl group, 1-indolidinyl group, 2-indolidinyl group, 3-indolidinyl group, 5-indolidinyl group, 6-indolidinyl group, 7-indolidinyl group, 8-indolidinyl group, 1-phenoxathiinyl group, 2-phenoxathiinyl group, 3-phenoxathiinyl group, 4-phenoxathiinyl group, thieno[2,3-b]furyl group, pyrrolo[1,2-b]pyridazinyl group, pyrazolo[1,5-a]pyridyl group, imidazo[11,2-a]pyridyl group, imidazo[1,5-a]pyridyl group, imidazo[1,2-b]pyridazinyl group, imidazo[1,2-a]pyrimidinyl group, 1,2,4-triazolo[4,3-a]pyridyl group, and 1,2,4-triazolo[4,3-a]pyridazinyl group.

The partially unsaturated or saturated heterocyclic group containing one or two or more ring-constituting heteroatoms may be either a monocyclic non-aromatic heterocyclic group or a condensed polycyclic non-aromatic heterocyclic group. The ring-constituting heteroatom is the same as that mentioned above.

Examples of the "monocyclic non-aromatic heterocyclic group" include, for example, 3- to 7-membered saturated or unsaturated monocyclic non-aromatic heterocyclic groups such as 1-aziridinyl group, 1-azetidinyl group, 1-pyrrolidinyl group, 2-pyrrolidinyl group, 3-pyrrolidinyl group, 2-tetrahydrofuryl group, 3-tetrahydrofuryl group, thiolanyl group, 1-imidazolidinyl group, 2-imidazolidinyl group, 4-imidazolidinyl group, 1-pyrazolidinyl group, 3-pyrazolidinyl group, 4-pyrazolidinyl group, 1-(2-pyrrolinyl) group, 1-(2-imidazolinyl) group, 2-(2-imidazolinyl) group, 1-(2-pyrazolinyl) group, 3-(2-pyrazolinyl) group, piperidino group, 2-piperidinyl group, 3-piperidinyl group, 4-piperidinyl group, 1-homopiperidinyl group, 2-tetrahydropyranyl group, morpholino group, (thiomorpholin)-4-yl group, 1-piperazinyl group, and 1-homopiperazinyl group.

Examples of the "condensed polycyclic non-aromatic heterocyclic group" include, for example, 8- to 10-membered saturated or unsaturated condensed polycyclic non-aromatic heterocyclic groups such as 2-quinuclidinyl group, 2-cromanyl group, 3-cromanyl group, 4-cromanyl group, 5-cromanyl group, 6-cromanyl group, 7-cromanyl group, 8-cromanyl group, 1-isocromanyl group, 3-isocromanyl group, 4-isocromanyl group, 5-isocromanyl group, 6-isocromanyl group, 7-isocromanyl group, 8-isocromanyl group, 2-thiocromanyl group, 3-thiocromanyl group, 4-thiocromanyl group, 5-thiocromanyl group, 6-thiocromanyl group, 7-thiocromanyl group, 8-thiocromanyl group, 1-isothiocromanyl group, 3-isothiocromanyl group, 4-isothiocromanyl group, 5-isothiocromanyl group, 6-isothiocromanyl group, 7-isothiocromanyl group, 8-isothiocromanyl group, 1-indolinyl group, 2-indolinyl group, 3-indolinyl group, 4-indolinyl group, 5-indolinyl group, 6-indolinyl group, 7-indolinyl group, 1-isoindolinyl group, 2-isoindolinyl group, 4-isoindolinyl group, 5-isoindolinyl group, 2-(4H-chromenyl) group, 3-(4H-chromenyl) group, 4-(4H-chromenyl) group, 5-(4H-chromenyl) group, 6-(4H-chromenyl) group, 7-(4H-chromenyl) group, 8-(4H-chromenyl) group, 1-isochromenyl group, 3-isochromenyl group, 4-isochromenyl group, 5-isochromenyl group, 6-isochromenyl group, 7-isochromenyl group, 8-isochromenyl group, 1-(1H-pyrrolidinyl) group, 2-(1H-pyrrolidinyl) group, 3-(1H-pyrrolidinyl) group, 5-(1H-pyrrolidinyl) group, 6-(1H-pyrrolidinyl) group, and 7-(1H-pyrrolidinyl) group.

Examples of the partially unsaturated or saturated heterocyclic ring containing one or two or more ring-constituting heteroatoms formed by $R^1$ and $R^6$ combined together, and the partially unsaturated or saturated heterocyclic ring containing one or two or more ring-constituting heteroatoms formed by $R^5$ and $R^6$ combined together include the heterocyclic moieties constituting the aforementioned heterocyclic groups.

When the expression "which may have a substituent" is used for a certain functional group in this specification, it means that the functional group is unsubstituted, or the functional group has one or two or more "substituents" at chemically substitutable positions, unless otherwise indicated. Type, number, and position of substituent existing on a functional group are not particularly limited, and when there are two or more substituents, they may be the same or different. Examples of the "substituent" existing on a functional group include, for example, a halogen atom, oxo group, thioxo group, nitro group, nitroso group, cyano group, isocyano group, cyanato group, tiocyanato group, isocyanato group, isotiocyanato group, hydroxy group, sulfanyl group, carboxy group, sulfanylcarbonyl group, oxalo group, mesoxalo group, thiocarboxy group, dithiocarboxy group, carbamoyl group, thiocarbamoyl group, sulfo group, sulfamoyl group, sulfino group, sulfinamoyl group, sulfeno group, sulfenamoyl group, phosphono group, hydroxyphosphonyl group, a hydrocarbon group, a heterocyclic group, a hydrocarbon-oxy group, a (heterocyclic ring)-oxy group, a hydrocarbon-sulfanyl group, a (heterocyclic ring)-sulfanyl group, an acyl group, amino group, hydrazino group, hydrazono group, diazenyl group, ureido group, thioureido group, guanidino group, carbamoimidoyl group (amidino group), azido group, imino group, hydroxyamino group, hydroxyimino group, aminoxy group, diazo group, semicarbazino group, semicarbazono group, allophanyl group, hydantoyl group, phosphano group, phosphoroso group, phospho group, boryl group, silyl group, stanyl group, selanyl group, oxido group, and the like, but it is not limited to these examples.

When there are two or more "substituents", the two or more substituents may combine to form a cyclic group together with the atom to which they bind. Such a cyclic group may contain 1 to 3 kinds of heteroatoms selected from oxygen atom, sulfur atom, nitrogen atom and the like as atoms constituting the ring system (ring atoms), and the ring may have one or more substituents. The ring may be either a monocyclic or condensed polycyclic ring, and may be either an aromatic or non-aromatic ring.

The "substituent" in the aforementioned definitions may be substituted with another substituent at a chemically substitutable position on the substituent. Type, number, and position of the substituent are not particularly limited, and when the substituent is substituted with two or more substituents, they may be the same or different. Examples of such a substituent include, for example, a halogenated alkyl-carbonyl group (specifically, such groups as trifluoroacetyl), a halogenated alkylsulfonyl group (specifically, such groups as trifluoromethanesulfonyl), an acyl-oxy group, an acyl-sulfanyl group, an N-hydrocarbon-amino group, an N,N-di(hydrocarbon)-amino group, an N-(heterocyclic ring)-amino group, an N-hydrocarbon-N-(heterocyclic ring)-amino group, an acyl-amino group, and a di(acyl)-amino group, and the like, but it is not limited to these examples.

In the general formula (I), $R^1$ represents a C1-C6 alkyl group which may have a substituent, a C1-C6 alkenyl group which may have a substituent, or an aryl group which may have a substituent. Examples of the C1-C6 alkyl group which may have a substituent as $R^1$ include, for example, an unsubstituted C1-C6 alkyl group, a C1-C6 alkyl group substituted with one or two or more halogen atoms, preferably fluorine atoms, a hydroxy-C1-C6 alkyl group substituted with hydroxy group, and the like. Preferred examples are methyl group, ethyl group, cyclopropyl group, methyl group or ethyl group substituted with fluorine atom, cyclopropyl group substituted with fluorine atom, and the like. Examples of the C1-C6 alkenyl group which may have a substituent as $R^1$ include an unsubstituted C1-C6 alkenyl group, for example, vinyl group, allyl group, and the like. Examples of the aryl group which may have a substituent as $R^1$ include, for example, phenyl group, phenyl group substituted with one or two or more fluorine atoms such as 2,4-difluorophenyl group, and the like.

As $R^1$, ethyl group or cyclopropyl group is particularly preferred.

$R^1$ and $R^6$ may combine to form a partially unsaturated or saturated heterocyclic ring containing one or two or more ring-constituting heteroatoms (the ring may have a substituent). It is preferred that $R^1$ and $R^6$ combine to form a

5- to 7-membered saturated heterocyclic ring containing one or two or more ring-constituting heteroatoms (the ring may have a substituent). Specific examples of the partially unsaturated or saturated heterocyclic ring containing one or two or more ring-constituting heteroatoms (the ring may have a substituent) formed by $R^1$ and $R^6$ that combine together include a 6-membered ring containing one oxygen atom and one nitrogen atom (as in the case of the compound represented by the structural formula (21) and the like among the exemplary compounds mentioned below), and a 5-membered ring containing one oxygen atom and one nitrogen atom (as in the case of the compound represented by the structural formula (4) and the like among the exemplary compounds mentioned below). Examples of the substituent on the ring include, for example, a C1-C6 alkyl group, preferably methyl group, and the like. The alkyl group on the ring, preferably methyl group, may be in either the S- or R-configuration, but there is a case where the S-configuration is preferred.

It is preferred that $R^1$ is any of methyl group, ethyl group, and cyclopropyl group (the methyl group, ethyl group, or cyclopropyl group may be substituted with one or two or more fluorine atoms), or $R^1$ binds with $R^6$ to form a 5- or 6-membered saturated heterocyclic ring containing two ring-constituting heteroatoms (the ring may have one C1-C6 alkyl group, preferably methyl group, as a substituent). It is preferred that one of the two ring-constituting heteroatoms is the nitrogen atom of the quinoline ring in the general formula (I), and the other one ring-constituting heteroatom is oxygen atom.

In the general formula (I), $R^2$ represents hydrogen atom, carboxyl group, or amino group, and it is preferred that $R^2$ is hydrogen atom.

In the general formula (I), $R^3$ represents hydroxy group, or a C1-C6 alkyl group which may have a substituent.

As the C1-C6 alkyl group as $R^3$, methyl group, ethyl group, n-propyl group, isopropyl group, and the like are preferred, and methyl group and ethyl group are more preferred. When $R^3$ is a C1-C6 alkyl group which does not have a substituent, methyl group and ethyl group are preferred, and ethyl group is particularly preferred. When $R^3$ is a C1-C6 alkyl group having a substituent, methyl group is preferred as the C1-C6 alkyl group, and hydroxy group, amino group, a halogen atom, and the like are preferred as the substituent. The hydroxy group or amino group on the C1-C6 alkyl group may have a substituent.

As $R^3$, hydroxy group, an unsubstituted C1-C6 alkyl group, a hydroxy(C1-C6 alkyl) group which may have a substituent, and an amino(C1-C6 alkyl) group which may have a substituent are preferred. Examples of the hydroxy(C1-C6 alkyl) group which may have a substituent include a hydroxy(C1-C6 alkyl) group in which hydroxy group is substituted with a C1-C6 alkylcarbonyl group (the alkyl moiety of the C1-C6 alkylcarbonyl group may be substituted with carboxyl group), a hydroxy(C1-C6 alkyl) group in which hydroxy group is substituted with a C1-C6 alkyl group, and the like, and examples of the amino(C1-C6 alkyl) group which may have a substituent include a carbamoyl-substituted C1-C6 alkyl group, and the like. As $R^3$, for example, a carboxy-substituted ethylcarbonyloxy(C1-C6 alkyl) group, ethylcarbonyloxy(C1-C6 alkyl) group, methylcarbonyloxy(C1-C6 alkyl) group (acetoxy(C1-C6 alkyl) group), and the like can also be used. As $R^3$, more specifically, such groups as hydroxy group, $-CH_3$, $-CH_2CH_3$,$-CH_2OH$, $-CH_2OCH_3$, $-CH_2NH_2$, $-C(O)NH_2$, $-CH_2OCOCH_3$, $-CH_2OCOCH_2CH_3$,$-CH_2OCOCH_2COOH_3$, $-CH_2OCOCH_2CH_2COOH$, $-CH_2OCH_3$, and $-CH_2ONH_2$ can be exemplified, but not limited to these examples.

In the general formula (I), $R^4$ represents hydrogen atom, a halogen atom, a C1-C6 alkyl group which may have a substituent, or a C1-C6 alkoxy group which may have a substituent. It is preferred that $R^4$ is hydrogen atom, a halogen atom, or a C1-C6 alkyl group which may have a substituent. It is more preferred that $R^4$ is hydrogen atom, fluorine atom, chlorine atom, or methyl group, and it is particularly preferred that $R^4$ is hydrogen atom or fluorine atom.

$R^4$ and $R^5$ may combine together to form a partially unsaturated or saturated heterocyclic ring containing one or two or more ring-constituting heteroatoms (the ring may have a substituent). Examples of the heterocyclic ring formed by $R^4$ and $R^5$ that combine together include methylenedioxy group, and the like.

$R^5$ represents a C1-C6 alkyl group which may have a substituent, a C1-C6 alkoxy group which may have a substituent, an aryl group which may have a substituent, a heteroaryl group which may have a substituent, or a partially saturated or saturated heterocyclic group which may have a substituent. It is preferably an aryl group which may have a substituent, or a partially saturated or saturated heterocyclic group which may have a substituent. As the C1-C6 alkyl group as $R^5$, for example, methyl group, ethyl group, and the like are preferred, and as the C1-C6 alkoxy group as $R^5$, for example, methoxy group, ethoxy group, and the like are preferred. As the aryl group as $R^5$, phenyl group is preferred, and the phenyl group preferably has one or two or more substituents. Examples include a C1-C6 alkyl group which may have hydroxy group, amino group, fluorine atom, amino group, or the like as the substituent, and for example, a phenyl group having hydroxy group or amino group at the para-position, a phenyl group having aminomethyl group at the para-position, and the like are preferred. p-Hydroxyphenyl group is particularly preferred. Examples of the heteroaryl group as $R^5$ include, for example, pyridinyl group, imidazolyl group, and the like, but it is not limited to these examples. As the partially saturated or saturated heterocyclic group, a 5- or 7-membered saturated heterocyclic group containing one or two ring-constituting nitrogen atoms is preferred, and for example, piperazinyl group, pyrrolidinyl group, piperidinyl group, and the like are preferred. As a bicyclic heterocyclic group, for example, isoindolinyl group, and the like can be mentioned. The heterocyclic group may have a substituent, and as the substituent, for example, a C1-C6 alkyl groups such as methyl group, amino group, hydroxy group, and the like can be used. Example of the heterocyclic group having a substituent as $R^5$ include, for example, 4-methylpiperazinyl group, 3-methyl-1-piperazinyl group, 3-amino-1-pyrrolidinyl group, 3-hydroxy-1-pyrrolidinyl group, homopiperazinyl group, 4-hydroxypiperidinyl group, 3-amino-1-piperazinyl group, 3-hydroxyazetidinyl group, and the like, but it is not limited to these examples.

It is preferred that, for example, $R^5$ is phenyl group (the phenyl group may have one or more substituents selected from the group consisting of hydroxy group, amino group, a C1-C6 alkyl group, and aminomethyl group), pyridyl group (the pyridyl group may have one or more substituents selected from the group consisting of hydroxy group, amino group, a C1-C6 alkyl group, and aminomethyl group), piperazinyl group (the piperazinyl group may have one or more substituents selected from the group consisting of hydroxy group, amino group, a C1-C6 alkyl group, and aminomethyl group), piperidinyl group (the piperidinyl group may have one or more substituents selected from the group consisting of hydroxy group, amino group, a C1-C6 alkyl group, and aminomethyl group), or isoindolinyl group (the isoindolinyl group may have one or more substituents selected from the group consisting of hydroxy group, amino group, a C1-C6 alkyl group, and aminomethyl group, and may be partially saturated).

$R^5$ and $R^6$ may combine together to form a partially unsaturated or saturated heterocyclic ring containing one or two or more ring-constituting heteroatoms (the ring may have a substituent), preferably a 5- to 7-membered partially unsaturated or saturated heterocyclic ring containing one or two or more ring-constituting heteroatoms (the ring may have a substituent). It is more preferred that $R^5$ binds with $R^6$ to form a saturated 5- or 6-membered heterocyclic ring containing two ring-constituting heteroatoms (the ring may have, as a substituent, a C1-C6 alkyl group which may have a substituent). Examples of the heterocyclic ring formed by $R^5$ and $R^6$ that combine together include methylenedioxy group, a 5-membered ring containing one ring-constituting nitrogen atom and one ring-constituting oxygen atom, and the like. These rings may have a substituent, and for example, a C1-C6 alkyl group such as methyl group, amino group, hydroxy group, a C1-C6 alkylcarbonyl groups such as acetyl group, and the like can be used. For example, it is more preferred that the 5- or 6-membered heterocyclic rings shown in the structural formulas (2) and (3) mentioned below are formed, and it is more preferred that the substituent on the heterocyclic ring is a C1-C6 alkylcarbonyl group.

$R^6$ represents hydrogen atom, a C1-C6 alkyl group which may have a substituent, a C1-C6 alkoxy group which may have a substituent, or a halogen atom. It is more preferred that $R^6$ is hydrogen atom, fluorine atom, chlorine atom, a C1-C6 alkyl group (the alkyl group may be substituted with fluorine atom), or a C1-C6 alkoxy group (the alkoxy group may be substituted with fluorine atom), and it is further preferred that $R^6$ is hydrogen atom, fluorine atom, chlorine atom, a C1-C6 alkyl group (the alkyl group may be substituted with fluorine atom), or a C1-C6 alkoxy group.

More specific examples of the compounds represented by the general formula (I) include the following compounds. However, the compounds of the present invention are not limited to these compounds.

[Formula 2]

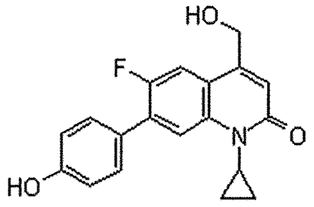

(1)

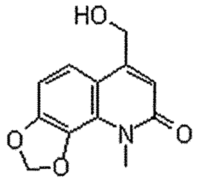

(2)

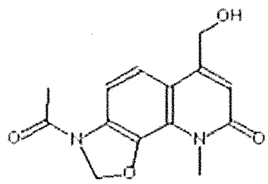

(3)

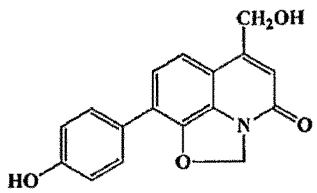

(4)

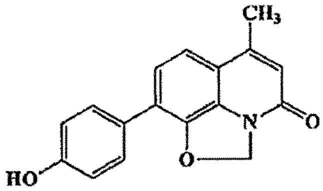

(5)

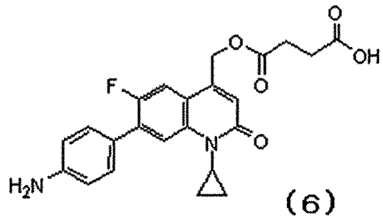

(6)

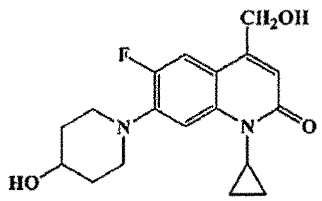

(7)

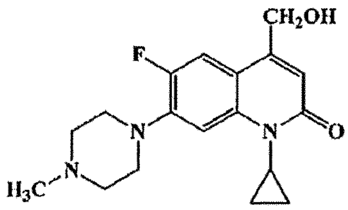

(8)

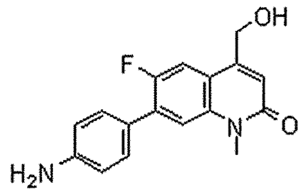

(9)

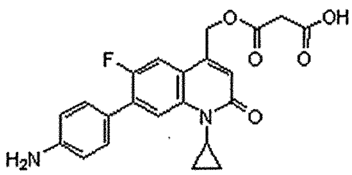

(10)

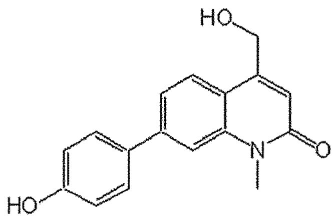

(11)

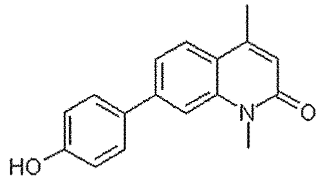

(12)

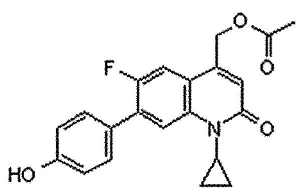

(13)

[Formula 3]

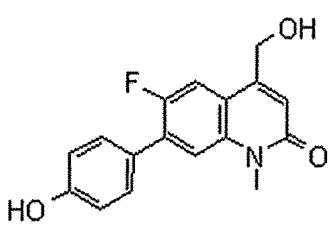

(14)

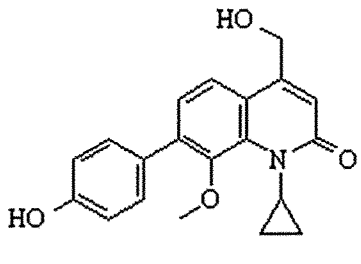

(15)

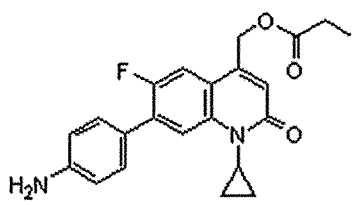

(16)

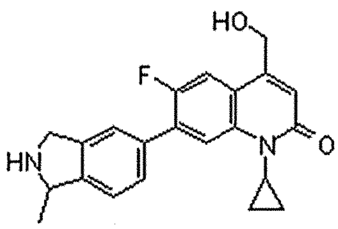

(17)

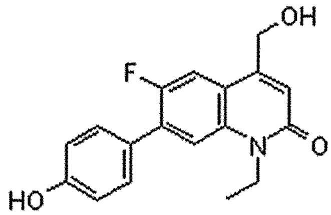

(18)

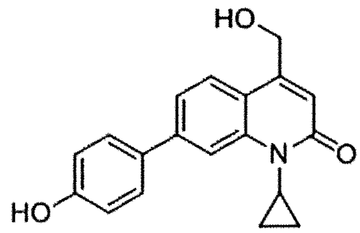

(19)

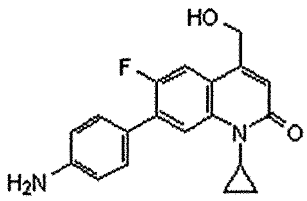

(20)

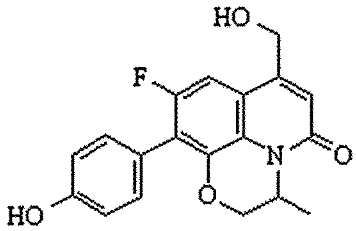

(21)

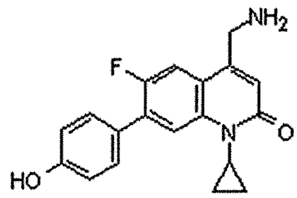

(22)

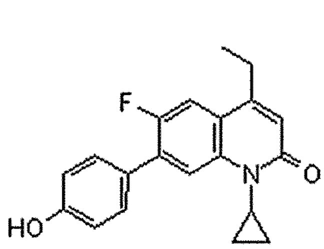

(23)

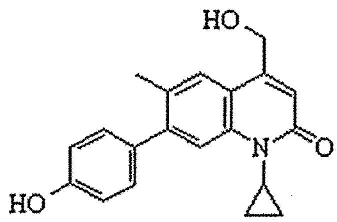

(24)

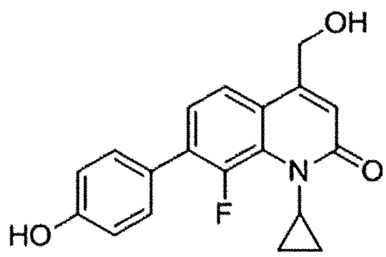

(25)

[Formula 4]

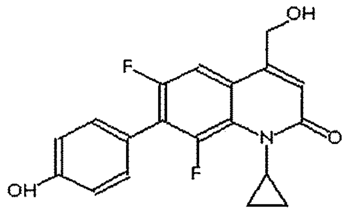

(26)

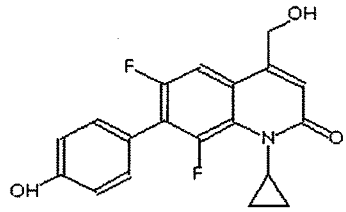

(27)

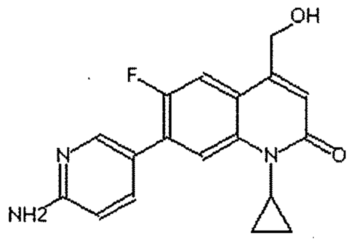

(28)

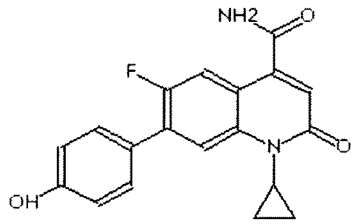

(29)

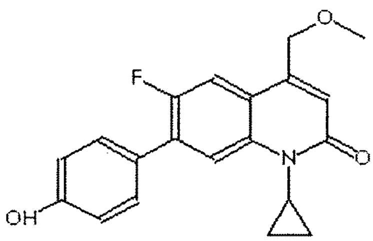

(30)

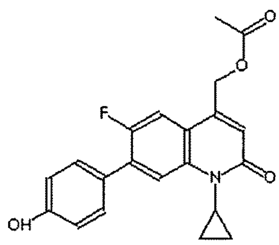

(31)

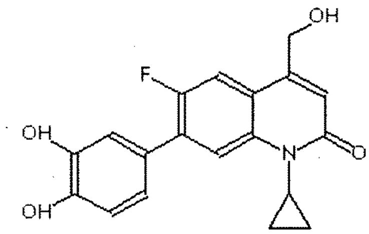

(32)

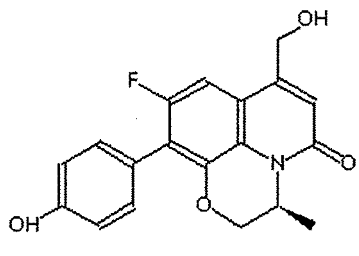

(33)

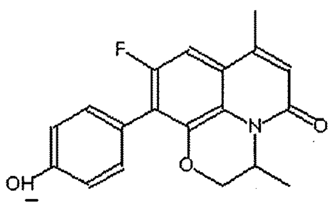

(34)

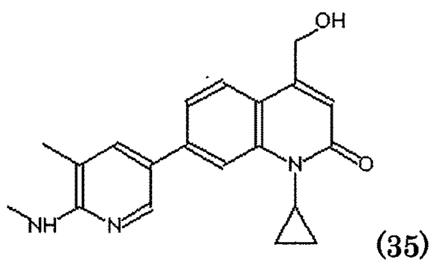

(35)

[Formula 5]

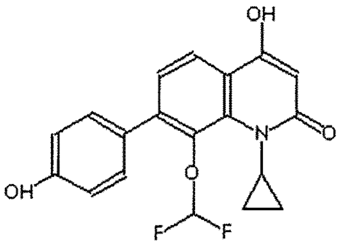
(36)

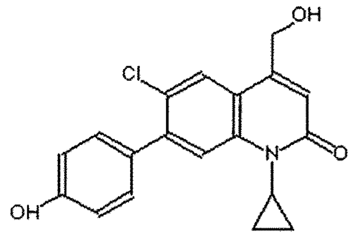
(37)

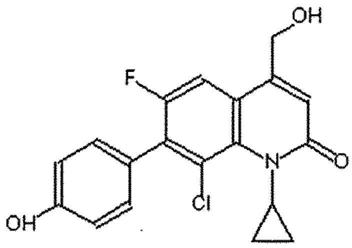
(38)

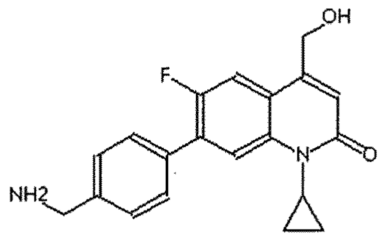
(39)

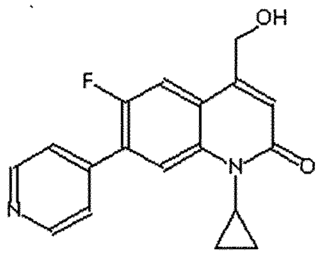
(40)

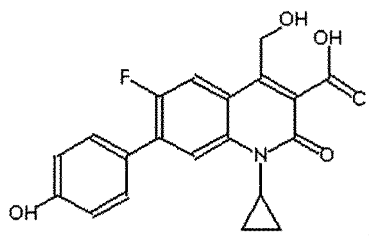
(41)

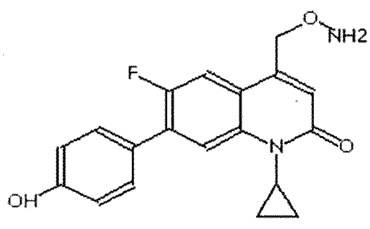
(42)

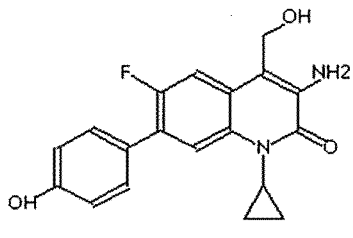
(43)

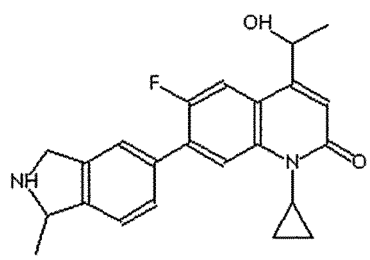
(44)

The compounds represented by the general formula (I) may be in the form of a salt. Type of the salt is not particularly limited, and it can be appropriately chosen depending on the purpose. Examples include, for example, salts with an alkali metal such as sodium and potassium; salts with an alkaline earth metal such as calcium and magnesium;

salts with an organic amine such as methylamine, ethylamine, and diethanolamine; salts with a mineral acid such as hydrochloride, sulfate, and nitrate; salts with an organic acid such as p-toluenesulfonate, maleate, and tartarate, and the like.

The compounds represented by the general formula (I) and salts thereof may exist in the form of a hydrate or solvate. Although type of the solvent that forms the solvate is not particularly limited, examples include, for example, ethanol, ethyl acetate, acetone, and the like. The compounds represented by the general formula (I) may exist as an optical isomer, diastereoisomer, or geometrical isomer depending on type of the substituent. An arbitrary isomer in pure form or a mixture of arbitrary isomers also falls within the scope of the present invention.

The compounds represented by the general formula (I) and salts thereof can be easily synthesized from starting compounds easily available for those skilled in the art by performing general chemical reactions commonly used by those skilled in the art. Specific preparation methods of the compounds of the present invention are shown in the section of Examples in this specification. By referring to those synthesis methods, those skilled in the art can easily prepare the compounds of the present invention falling within the scope of the general formula (I).

The compounds represented by the general formula (I) and salts thereof of the present invention have a potent inhibitory activity against a DNA gyrase of bacteria, in particular, a mutant DNA gyrase of a bacterium that has acquired resistance to quinolones.

The DNA gyrase is an essential factor for the DNA replication having a function of duly advancing DNA polymerase when the DNA polymerase advances along a template strand during DNA replication by repeatedly cleaving and religating DNA strands to ease the superhelix structure which is inevitably produced when the DNA polymerase advances along a template strand and makes extension of the replication difficult.

The compounds represented by the general formula (I) and salts thereof can appropriately inhibit the activity of a DNA gyrase A subunit of a mutant *Staphylococcus aureus* that has acquired resistance to quinolones through substitution of a leucine residue for the 84th serine residue from the amino terminus in the wild-type DNA gyrase A subunit of *Staphylococcus aureus.* A medicament containing a compound represented by the general formula (I) or a salt thereof as an active ingredient can exhibit a potent antibacterial activity specifically against bacteria that have acquired resistance to quinolones. Accordingly, the medicament can be used for prophylactic and/or therapeutic treatment of an infectious disease caused by a bacterium that has acquired resistance to quinolones.

Content of the compound represented by the general formula (I) or a salt thereof in the medicament is not particularly limited, and can be appropriately chosen according to the purpose. As the active ingredient of the medicament, a single kind of compound represented by the general formula (I) or a salt thereof may be used, or two or more kinds of them may be used in combination, if needed. As the compound represented by the general formula (I) or a salt thereof, for example, the compounds represented by any of the aforementioned structural formulas (1) to (44) are preferred, and the compounds represented by the structural formulas (20), (17), and (25), and the like are more preferred, because of the superior DNA gyrase inhibitory activity thereof.

The aforementioned medicament is generally provided in the form of a pharmaceutical composition utilizing one or two or more kinds of pharmaceutical additives commonly used in this field. For example, it can be provided in the form of a pharmaceutical composition for oral administration such as capsule, tablet, syrup, powder, granule, subtilized granule, or solution, injection or fusion drip for intravenous administration, injection for intramuscular administration or subcutaneous administration, inhalant, eye drop, ear drop, percutaneous absorption preparation, or the like, and the medicament can be formed by the methods well known to those skilled in the art with appropriately choosing pharmaceutical additives depending on the form of the pharmaceutical composition.

Method for administration, dose, time for administration, and object of administration of the aforementioned medicament are not particularly limited, and they can be appropriately chosen depending on the purpose. The dose can be appropriately chosen in consideration of various factors such as the age, body weight, body constitution of patients, symptoms, and type of causative bacterium, as well as whether medicaments containing other ingredients as active ingredients are administered or not.

Object of the administration is not particularly limited, and can be chosen depending on the purpose. Examples of species of the object of the administration include, for example, human, apes, swines, bovines, ovines, caprines, canines, felines, mouse, rat, birds, and the like, and among these, the medicament is preferably used for human.

The medicament of the present invention can exhibit an especially potent antibacterial activity against bacteria that have acquired resistance to quinolones. The medicament of the present invention can exhibit a potent antibacterial activity against any of Gram-positive bacteria that have acquired resistance to quinolones through introduction of a mutation into DNA gyrase, and it exhibits especially superior antibacterial activity against, for example, *Staphylococcus aureus* that have acquired resistance to quinolones. Although type of a causative bacterium of an infectious disease as the target of the prophylactic and/or therapeutic treatment by using the medicament of the present invention is not particularly limited, in view of the superior antibacterial activity of the medicament of the present invention, *Staphylococcus aureus* is preferred, and a quinolone-resistant *Staphylococcus aureus* is more preferred.

It is known that, in a quinolone-resistant *Staphylococcus aureus,* amino acid mutations are introduced into the

DNA gyrase (topoisomerase II) and topoisomerase IV. The subunits of the DNA gyrase are encoded by the *gyrA* and *gyrB* genes, and subunits of the topoisomerase IV are encoded by *parC* and *parE*. Quinolone-resistant *Staphylococcus aureus* may further be methicillin-resistant *Staphylococcus aureus* (MRSA), vancomycin-intermediate resistant *Staphylococcus aureus* (VISA), or vancomycin-resistant *Staphylococcus aureus* (VRSA), and the medicament of the present invention can exhibit a potent antibacterial activity also against these bacteria by acting on such a mutant DNA gyrase as mentioned above as a target. The medicament of the present invention has antibacterial activity especially suitable for *Staphylococcus aureus* having a mutant DNA gyrase A subunit in which a leucine residue substitute for the 84th serine residue counted from the amino terminus of the DNA gyrase A subunit of wild-type *Staphylococcus aureus.*

The method for measuring the antibacterial activity is not particularly limited, and can be appropriately chosen by those skilled in the art. Examples include the method of determining the minimum inhibitory concentration (henceforth also referred to as "MIC"), and the like. As the method for determining MIC, known methods can be appropriately used, but the broth microdilution method is preferred.

MIC of the medicament of the present invention for quinolone-resistant bacteria is preferably, for example, 4 mg/L or lower, more preferably 1 mg/L or lower, still more preferably 0.25 mg/L or lower, particularly preferably 0.0625 mg/L or lower.

The medicament of the present invention has a potent antibacterial activity against bacteria that have acquired resistance to quinolones. However, if a quinolone-resistant bacterium further acquires resistance to the medicament, the mutation introduced into the DNA gyrase is reversed, and the DNA gyrase becomes a non-mutated DNA gyrase of a quinolone-susceptible bacterium. Therefore, by using a quinolone and the medicament of the present invention in combination, emergence of bacteria resistant to quinolones can be effectively prevented.

Therefore, as one embodiment of the medicament of the present invention, there is provided a medicament comprising at least a combination of a compound represented by the aforementioned general formula (I) or a salt thereof, and a quinolone. The quinolone that can be used for the combination is not particularly limited, and an old quinolone or new quinolone may be used. A single kind of quinolone may be used, and two or more kinds of quinolones may be used in combination. Amount of the quinolone to be combined with the compound represented by the aforementioned general formula (I) or a salt thereof is not particularly limited, and it can be appropriately chosen according to the type of the quinolone, type of infectious disease, and the like.

Since the old quinolones exhibit antibacterial activity against Gram-negative bacteria, they are used for urogenital infectious diseases, enteric infections, and the like. However, since they only exhibit weak antibacterial activity against Gram-positive bacteria, they cannot generally be used for infectious diseases of the respiratory tract caused by pneumococci and the like. Specific examples of the old quinolones include nalidixic acid, piromidic acid, pipemidic acid, and the like.

The new quinolones generally have fluorine or hydrogen at the 6-position of the quinolone ring, and a cyclic basic group at the 7-position of the same. Since the new quinolones exhibit high antibacterial activity against not only Gram-negative bacteria, but also Gram-positive bacteria, they are also widely used for infectious diseases induced by pneumococci or streptococci, besides urogenital infectious diseases, enteric infections, and the like. Specific examples of the new quinolones include norfloxacin, levofloxacin, ofloxacin, enoxacin, ciprofloxacin chloride, tosufloxacin tosylate, lomefloxacin hydrochloride, fleroxacin, sparfloxacin, gatifloxacin, puzufloxacin mesylate, moxifloxacin, garenoxacin, and the like, but not limited to these examples.

In the present specification, the "combination" means use of the compound represented by aforementioned general formula (I) or a salt thereof together with at least one kind of such a quinolone as mentioned above (henceforth also referred to as "concomitant use"). The timing of the combination use is not particularly limited, and can be appropriately determined depending on the purpose. The compound or a salt and at least one kind of quinolone may be simultaneously administered, or they may be successively administered with an appropriately chosen time interval so long as the effect of the present invention is not degraded. When the compound represented by the aforementioned general formula (I) or a salt thereof and at least one kind of quinolone are simultaneously administered, they may be administered as separate agents (separate dosage forms), or as a single agent containing the both in one preparation (so-called combination drug).

When a medicament containing the compound represented by the aforementioned general formula (I) or a salt thereof and at least one kind of quinolone are administered with an appropriate time interval, order of the administration of the medicament of the present invention and the quinolone is not particularly limited, and can be appropriately determined depending on the purpose. The infectious disease as the target of the prophylactic and/or therapeutic treatment is not particularly limited, and it can apply to infectious diseases caused by a quinolone-resistant bacterium, infectious diseases caused by a quinolone-susceptible bacterium, or infectious diseases caused by the both. Examples include, for example, infectious diseases caused by a quinolone-susceptible *Staphylococcus aureus*, infectious diseases caused by a quinolone-resistant *Staphylococcus aureus,* or complex infectious diseases caused by the both.

Upon use of the medicament containing such a combination as mentioned above, the compound represented by the aforementioned general formula (I) or a salt thereof as the active ingredient contained in the medicament of the

present invention acts on quinolone-resistant *Staphylococcus aureus*, and the quinolone mainly acts on quinolone-susceptible *Staphylococcus aureus*, and therefore the medicament is advantageous in that antibacterial activity can be obtained irrespective of the presence or absence of quinolone resistance in causative bacteria of infectious diseases, and can be widely used for prophylactic and/or therapeutic treatment of infectious diseases.

As another one embodiment of the medicament of the present invention, there is also provided a medicament containing at least a combination of a compound represented by the aforementioned general formula (I) or a salt thereof, and a drug efflux pump inhibitor. As for the drug efflux pump, and inhibitor for it, for example, Japanese Patent Unexamined Publication (KOHYO) No. 2008-502720 and references cited therein, the general remarks of Xian-Zhi Li and Hiroshi Nikaido (Drugs, 69, pp.1555-1623, 2009, especially Table 4), as well as Antibiotics, 2, pp.28-45, 2013; Journal of Antimicrobial Chemotherapy, 59, pp.1223-1229, 2007; Bioorganic & Medicinal Chemistry, 19, pp.7679-7689, 2011; Antimicrobial Agents and Chemotherapy, 48, pp.4171-4176, 2004; Journal of Antimicrobial Chemotherapy, 59, pp.1247-1260, 2007; Antimicrobial Agents and Chemotherapy, 52, pp.3202-3209, 2008, and the like can be referred to. As for the relation between quinolones and drug efflux pump inhibitor, for example, Journal of the Japanese Society of Chemotherapy, 52 (7), pp.355-360, 2004, and the like can be referred to.

Although the drug efflux pump inhibitor that can be used in the combination according to the present invention is not particularly limited, there is, for example, a case where use of Phe-Arg-β-naphthylamine (PABN: PLoS ONE, 8, e60666, 2013) and the like is preferred. Amount of the drug efflux pump inhibitor to be combined with the compound represented by the aforementioned general formula (I) or a salt thereof is not particularly limited, and can be appropriately chosen according to the type thereof, type of the infectious disease, and the like. Furthermore, a drug efflux pump inhibitor may be further combined with the medicament containing at least a combination of a compound represented by the aforementioned general formula (I) or a salt thereof, and a quinolone, and the medicament may be provided as a medicament containing a combination of a compound represented by aforementioned general formula (I) or a salt thereof, a quinolone, and a drug efflux pump inhibitor. The medicament containing the aforementioned combination may provided as a combination drug containing the three kinds of substances, i.e., a compound represented by aforementioned general formula (I) or a salt thereof, a quinolone, and a drug efflux pump inhibitor, or a combination drug containing arbitrary two kinds of the substances and a unit dosage form containing the remaining one kind of substance may be administered in combination. Alternatively, a medicament containing a compound represented by the aforementioned general formula (I) or a salt thereof, a quinolone, and a drug efflux pump inhibitor can also be administered in combination as separate unit dosage forms.

## Examples

The present invention will be more specifically explained with reference to examples. However, the scope of the present invention is not limited by these examples.

### Example 1: Synthesis of the compound represented by the structural formula (1) (compound J-131CP)

A mixture of 7-bromo-6-fluoro-4-methyl-1,2-dihydroquinolin-2-one (800 mg, 3.2 mmol), cyclopropylboronic acid (550 mg, 6.4 mmol), 2,2'-bipyridine (BiPy, 500 mg, 3.2 mmol), sodium carbonate (678 mg, 6.4 mmol), anhydrous copper(II) acetate ($Cu(OAc)_2$, 581 mg, 3.2 mmol), and 1,2-dichloroethane (DCE, 24 mL) was stirred at 70°C for 4 hours.

After completion of the stirring, the mixture was cooled to room temperature, saturated ammonium chloride (50 mL) was added to the mixture, and then the resulting mixture was extracted twice with dichloromethane (50 mL each). The resulting extract was washed twice with water (50 mL), and then washed with brine (50 mL). Anhydrous sodium sulfate was added to the washed extract to dry the extract, then the extract was filtered, and the filtrate was concentrated under reduced pressure. The concentrated filtrate was subjected to silica gel chromatography (eluted with ethyl acetate/petroleum ether = 1/10 to 1/2) to give 7-bromo-1-cyclopropyl-6-fluoro-4-methyl-1,2-dihydroquinolin-2-one (330 mg, yield 34%).

A mixture containing the resulting 7-bromo-1-cyclopropyl-6-fluoro-4-methyl-1,2-dihydroquinolin-2-one (320 mg, 1.08 mmol), and selenium dioxide (360 mg, 3.24 mmol) suspended in xylene (4 mL) was stirred at 150°C for 6 hours. Selenium dioxide (360 mg, 3.24 mmol) was further added to the stirred mixture, and the resulting mixture was stirred at 140°C for 16 hours. Then, the mixture was cooled to room temperature, water (30 mL) was added to the mixture, and the resulting mixture was extracted twice with ethyl acetate (30 mL) to obtain an extract. The resulting extract was washed with brine (30 mL). Anhydrous sodium sulfate was added to the washed extract, then the resulting mixture was filtered, and the filtrate was concentrated under reduced pressure. The residue remained after the concentration was ground in petroleum ether to give 7-bromo-1-cyclopropyl-6-fluoro-2-oxo-1,2-dihydroquinoline-4-carboaldehyde (280 mg, yield 84%).

The resulting 7-bromo-1-cyclopropyl-6-fluoro-2-oxo-1,2-dihydroquinoline-4-carboaldehyde (280 mg, 0.90 mmol) was dissolved in methanol (5 mL), and sodium borohydride (69 mg, 1.81 mmol) was added portionwise to the

solution in an ice-cooled state to obtain a mixture. The mixture was stirred at 25°C for 30 minutes. Ammonium chloride (30 mL) was added to the stirred mixture, and then the resulting mixture was extracted twice with ethyl acetate (30 mL) to obtain an extract. The extract was washed with brine (30 mL), anhydrous sodium sulfate was added to the extract for drying, then the extract was filtered, and the filtrate concentrated under reduced pressure to give 7-bromo-1-cyclopropyl-6-fluoro-4-(hydroxymethyl)-1,2-dihydroquinolin-2-one (270 mg, yield 96%).

A mixture containing the resulting 7-bromo-1-cyclopropyl-6-fluoro-4-(hydroxymethyl)-l,2-dihydroquinolin-2-one (260 mg, 0.833 mmol), 4-hydroxyphenylboronic acid (150 mg, 1.08mmoL), 1,1'-bis(diphenylphosphino)ferrocene-palladium(II) dichloride-dichloromethane complex ($PdCl_2$(dppf), 62 mg, 0.083 mmol), and potassium carbonate (172 mg, 1.25 mmol) in dried dioxane (10 mL) was maintained at 80°C for 6 hours under a nitrogen atmosphere.

Then, the mixture was cooled to room temperature, and diluted with ethyl acetate (50 mL), and a 10 mass % solution of ammonium chloride (50 mL). The organic layer was separated, and the aqueous layer was extracted with ethyl acetate (50 mL) to obtain an extract. The resulting extract and the separated organic layer were combined, washed with water (50 mL), and then washed with brine (50 mL), dried by using magnesium sulfate, and then concentrated in vacuo. After the concentration in vacuo, the resulting black residue was passed through silica gel, and then further concentrated to give the compound J-131CP, 1-cyclopropyl-6-fluoro-4-(hydroxymethyl)-7-(4-hydroxyphenyl)-1,2-dihydroquinolin-2-one, as gray solid (220 mg, yield 81%).
$^1$H-NMR (300MHz, DMSO-$d_6$):

δ (ppm): 0.73-0.75 (m, 2H), 1.25-1.27 (m, 2H), 2.95-2.96 (m, 1H), 4.69 (m, 2H), 5.48 (s, 1H), 6.53-6.56 (m, 1H), 6.90 (d, J=7.5Hz, 2H), 7.47 (d, J=7.2Hz, 2H), 7.55 (d, J=11.7Hz, 1H), 7.85 (d, J=7.2Hz, 1H), 9.74 (brs, 1H)

The resulting compound J-131CP was analyzed by LCMS. For the LCMS analysis, a quadrupole mass spectrometer, LC/MSD 1200 System (Agilent, column ODS2000 (50 mm x 4.6 mm, 5 μm), operated in ES (+) or (-) ionization mode, T = 30°C, flow rate = 1.5 mL/min, detection wavelength 214 nm) was used.

Mobile phrase: Solution containing 80 mass % water (containing 0.05 mass % TFA) and 20 mass % acetonitrile -> Solution containing 5 mass % water (containing 0.05 mass % TFA) and 95 mass % acetonitrile
Run time: 6 minutes
Equilibration: 0.5 minutes
Purity: >90%
Rt = 2.572 minutes
MS Calcd.: 325
MS Found: 326 ($[M+H]^+$)

Example 2: Synthesis of the compound represented by the structural formula (13) (compound J-131ACp)

To a solution of the compound J-131CP, 1-cyclopropyl-6-fluoro-4-(hydroxymethyl)-7-(4-hydroxyphenyl)-1,2-dihydroquinolin-2-one (100 mg, 0.154 mmol) in acetic acid (10 mL), KF (26.8 mg, 0.412 mmol) was added. The resulting mixture was stirred at 95°C overnight, then the solvent was removed from the mixture, and the residue was purified by preparative HPLC to give the compound J-131ACp, [1-cyclopropyl-6-fluoro-7-(4-hydroxyphenyl)-2-oxo-4-hydroquinolyl]methyl·acetate, as white solid (28 mg, yield 24%).
$^1$H-NMR (300MHz, DMSO-$d_6$):

δ (ppm): 0.75-0.77 (m, 2H), 1.26-1.28 (m, 2H), 2.13 (s, 3H), 2.95-3.01 (m, 1H), 5.30 (s, 1H), 6.52 (s, 1H), 6.91 (d, J=6.6Hz, 2H), 7.48 (d, J=6.6Hz, 3H), 7.62 (d, J=11.7Hz, 1H), 7.89 (d, J=6.9Hz, 1H), 9.80 (s, 1H)
MS Calcd.: 367; MS Found: 368 ($[M+1]^+$)

Example 3: Synthesis of the compound represented by the structural formula (2) (compound J-103)

[Formula 6]

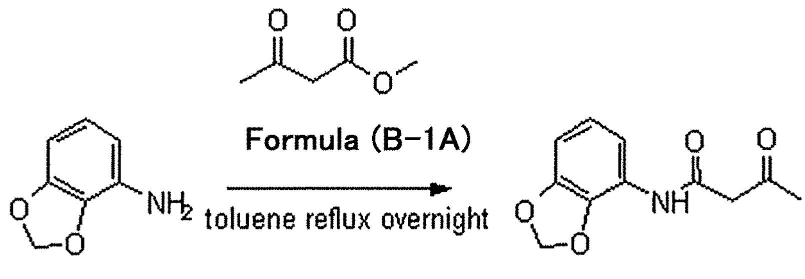


Formula (B-1) Formula (B-2)

A mixture of the compound represented by the structural formula (B-1) (5.0 g, 36.5 mmol) and the compound represented by the structural formula (B-1A) (4.66 g, 40.1 mmol) in toluene (50 mL) was heated to reflux, and stirred overnight. Then, the mixture was cooled to room temperature, and toluene was removed. The residue was purified by silica gel chromatography (petroleum ether:ethyl acetate (henceforth also referred to as "EA") = 100:0 to 2:1) to give the compound represented by the structural formula (B-2), N-(2H-benzo[d]1,3-dioxolen-4-yl)-3-oxobutanamide (1.08 g, yield 13%), as red solid.
$^1$H-NMR Spectrum (300MHz, $CDCl_3$):

δ (ppm): 9.08 (brs, 1H), 7.57-7.60 (m, 1H), 6.78-6.84 (m, 1H), 6.63-6.66 (m, 1H), 6.01 (s, 2H), 3.61 (s, 2H), 2.33 (s, 3H)

A solution of N-(2H-benzo[d] 1,3-dioxolen-4-yl)-3-oxobutanamide (1.08 g, 4.89 mmol) in 70% $H_2SO_4$ (24 mL) was stirred at 90°C for 1 hour. Then, the mixture was poured on ice (60 g), the resulting mixture was filtered, and the solid was washed with water, and ground in acetonitrile (10 mL) to give 6-methyl-9-hydro-2H-1,3-dioxoleno[4,5-h]quinolin-8-one as brown solid (780 mg, yield 79%).
$^1$H-NMR Spectrum (300MHz, DMSO-$d_6$):

δ (ppm): 11.37 (brs, 1H), 7.25 (d, J=8.4Hz, 1H), 6.87 (d, J=8.4Hz, 1H), 6.23 (s, 1H), 6.16 (s, 2H), 2.36 (s, 3H)

To a solution of 6-methyl-9-hydro-2H-1,3-dioxoleno[4,5-h]quinolin-8-one (780 mg, 3.8 mmol) in N,N-dimethylformamide (DMF) (10 mL), 60% NaH (231 mg, 5.8 mmol) was added at room temperature, the resulting mixture was stirred for 0.5 hour, iodomethane (MeI, 1.091 g, 7.7 mmol) was added to the mixture, and the resulting mixture was stirred at room temperature for 0.5 hour. Then, the mixture was poured into water (60 mL), and the resulting mixture was extracted with ethyl acetate (2 x 50 mL). The combined organic layer was washed with brine (50 mL), dried over anhydrous sodium sulfate, filtered, and concentrated. The residue was purified by silica gel column chromatography (eluted with ethyl acetate/petroleum ether (1:4)) to give 6,9-dimethyl-9-hydro-2H-1,3-dioxoleno[4,5-h]quinolin-8-one as yellow solid (690 mg, yield 83%).
$^1$H-NMR Spectrum (300MHz, $CDCl_3$):

δ (ppm): 7.23 (d, J=8.4Hz, 1H), 6.81 (d, J=8.4Hz, 1H), 6.43 (s, 1H), 6.04 (s, 2H), 3.87 (s, 3H), 2.39 (s, 3H)

A suspension of 6,9-dimethyl-9-hydro-2H-1,3-dioxoleno[4,5-h]quinolin-8-one (690 mg, 3.18 mmol) and $SeO_2$ (2.12 g, 19.1 mmol) in xylene (15 mL) was heated to 150°C, and stirred at the same temperature for 20 hours. The mixture was cooled to room temperature, then filtered, and concentrated, water (20 mL) was added to the residue, and the resulting mixture was extracted with ethyl acetate (2 x 50 mL). The combined organic layer was washed with brine (50 mL), dried over anhydrous sodium sulfate, filtered, and concentrated. The residue was purified by silica gel column chromatography (eluted with ethyl acetate/petroleum ether (1:5)) to give 9-methyl-8-oxo-9-hydro-2H-1,3-dioxoleno[4,5-h]quinoline-6-carboaldehyde as yellow solid (723 mg, yield 98%).
$^1$H-NMR Spectrum (300MHz, $CDCl_3$):

δ (ppm): 10.05 (s, 1H), 8.44 (d, J=8.4Hz, 1H), 6.98 (s, 1H), 6.89 (d, J=9.0Hz, 1H), 6.08 (s, 2H), 3.94 (s, 3H)

To an ice-cooled solution of 9-methyl-8-oxo-9-hydro-2H-1,3-dioxoleno[4,5-h] quinoline-6-carboaldehyde (723 mg, 3.1 mmol) in methanol (20 mL), sodium borohydride (238 mg, 6.26 mmol) was added portionwise. After the addition, the reaction mixture was stirred at room temperature for 30 minutes. Ammonium chloride (20 mL) was added to the reaction mixture, and the resulting mixture was extracted with ethyl acetate (2 x 50 mL). The organic layer was washed with brine (50 mL), dried over anhydrous sodium sulfate, filtered, and concentrated to give the compound J-103, 6-(hydroxymethyl)-9-methyl-9-hydro-2H-1,3-dioxoleno[4,5-h]quinolin-8-one, as yellow solid (311 mg, yield 43%).
$^1$H-NMR Spectrum (300MHz, DMSO-$d_6$):

δ (ppm): 7.27 (d, J=8.4Hz, 1H), 6.94 (d, J=8.4Hz, 1H), 6.51 (s, 1H), 6.11 (s, 2H), 5.47 (t, J=6.6Hz, 1H), 4.66 (d, J=6.6Hz, 2H), 3.74 (s, 3H)
MS Calcd.: 233
MS Found: 234 ([M+H]$^+$)

Example 4: Synthesis of the compound represented by the structural formula (3) (compound J-125a)

[Formula 7]

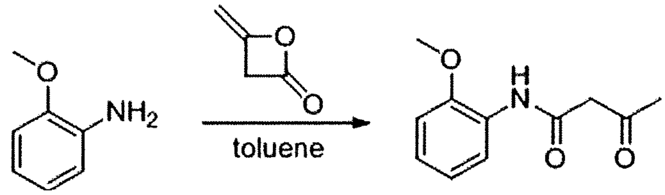


Formula (C-1)    Formula (C-2)

A mixture of the compound represented by the structural formula (C-1) (25 g, 203 mmol) and diketene (20.5 g, 24 mmol) in toluene (150 mL) was stirred overnight at 120°C. The reaction mixture was concentrated, and the residue was purified by silica gel chromatography (petroleum ether/ethyl acetate) = 10/1 to 2/1) to give N-(2-methoxyphenyl)-3-oxo-butylamide as yellow solid (37.5 g, yield 89%).
$^1$H-NMR Spectrum (300MHz, $CDCl_3$):

δ (ppm): 2.33 (s, 3H), 3.59 (s, 2H), 3.91 (s, 3H), 6.87-6.97 (m, 2H), 7.03-7.08 (m, 1H), 8.32 (dd, J=1.5,7.8Hz, 1H), 9.22 (brs, 1H)

To PPA (60 mL), N-(2-methoxyphenyl)-3-oxo-butylamide (31.0 g, 150 mmol) was added with stirring at 110°C. The reaction mixture was stirred overnight at 115°C. The reaction mixture was slowly poured into ice water with heating. The mixture was stirred for 15 minutes, and then filtered. The filtrate was neutralized with $NaHCO_3$ (solid) to pH 7, and the precipitates were collected by filtration, and dried in vacuo to give 8-methoxy-4-methyl-1H-quinolin-2-one as yellow solid (21.8 g, yield 76%).
$^1$H-NMR Spectrum (400MHz, DMSO-$d_6$):

δ (ppm): 2.39 (s, 3H), 3.87 (s, 3H), 6.40 (s, 1H), 7.12-7.14 (m, 2H), 7.25-7.28 (m, 1H), 10.57 (brs, 1H)

To a solution of 8-methoxy-4-methyl-1H-quinolin-2-one (21.8 g, 15.3 mmol) in DMF (250 mL), NaH (60% in oil, 13.8 g, 346 mmol) was added portionwise at room temperature, and the resulting mixture was stirred at the same temperature for 1 hour. Then, the mixture was transferred to an autoclave, and $CH_3I$ (32.8 g, 230.7 mmol) was added to the mixture. The reaction mixture was stirred overnight at 60°C in the autoclave, cooled to room temperature, and poured into ice water (500 mL). The mixture was extracted with ethyl acetate (EA), the combined organic layer was washed with water and brine, dried over $Na_2SO_4$, and filtered. The solvent was removed, and the residue was purified by silica gel chromatography (petroleum ether/EA = 10/1 to 2/1) to give 8-methoxy-1,4-dimethyl-1H-quinolin-2-one as yellow solid (15 g, yield 63.8%).
$^1$H-NMR Spectrum (300MHz, DMSO-$d_6$):

δ (ppm): 2.38 (s, 3H), 3.75 (s, 3H), 3.87 (s, 3H), 6.51 (s, 1H), 7.19-7.29 (m, 2H), 7.33-7.36 (m, 1H)

A suspension of 8-methoxy- 1,4-dimethyl- 1H-quinolin-2-one (15.0 g, 73.5 mmol) and $SeO_2$ (40.6 g, 368 mmol) in dioxane (150 mL) was stirred at 125°C for 3 days. The reaction mixture was cooled, and filtered through Celite. The solvent was removed from the filtrate in vacuo to give a crude product of 8-methoxy-1-methyl-2-oxo-1,2-dihydroquinoline-4-carboaldehyde (16.1 g, quantitative), which was used for the following reaction without purification.
To a mixture of 8-methoxy-1-methyl-2-oxo-1,2-dihydroquinoline-4-carboaldehyde (16.0 g, 73.7 mmol) in meth-

anol (150 mL), $NaBH_4$ was added portionwise at room temperature. The reaction mixture was stirred for 30 minutes, quenched with water (100 mL), and concentrated in vacuo to 120 mL. The mixture was extracted with dichloromethane, and the combined organic layer was washed with water and brine, dried over $Na_2SO_4$, and filtered. The solvent was removed in vacuo to give a crude product of 4-hydroxymethyl-8-methoxy-1-methyl-1H-quinolin-2-one as yellow solid (16.3 g, quantitative), which was used for the following reaction without purification.

A mixture of 4-hydroxymethyl-8-methoxy-1-methyl-1H-quinolin-2-one in HBr (40%, 150 mL) was stirred overnight at 130°C. The reaction mixture was cooled, and filtered. The cake was washed with water and diethyl ether, and then dried in vacuo to give 4-bromomethyl-8-hydroxy-1-methyl-1H-quinolin-2-one (16 g, yield 81%), which was used for the following reaction without purification.

A mixture of 4-bromomethyl-8-hydroxy-1-methyl-1H-quinolin-2-one (14 g, 52 mmol), and potassium acetate (KOAc, 15.0 g, 156 mmol) in DMF (250 mL) was stirred at 100°C for 3 hours. The reaction mixture was diluted with water, and extracted with ethyl acetate. The combined organic layer was washed with water and brine, and dried. The solvent was removed in vacuo, and the residue was treated with ethyl acetate/diethyl ether to give 8-hydroxy-1-methyl-2-oxo-4-hydroquinolyl)methyl·acetate as off white solid (5.7 g, yield 44%).
$^1$H-NMR Spectrum (300MHz, DMSO-$d_6$):

δ (ppm): 2.15 (s, 3H), 3.85 (s, 3H), 5.29 (s, 2H), 6.56 (s, 1H), 7.09-7.15 (m, 3H), 10.15 (brs, 1H)

To a suspension of 8-hydroxy-1-methyl-2-oxo-4-hydroquinolyl)methyl·acetate (4.73 g, 19.2 mmol) in acetic anhydride (60 mL), a solution of $HNO_3$ (65% to 68%, 1.17 mL, 0.9 eq) and acetic acid (3.52 mL) was added at -3 to 3°C. The resulting mixture was further stirred at the same temperature for 3 hours. The reaction mixture was quenched with ice water, and the resulting mixture was extracted with ethyl acetate. The combined organic layer was washed with water and brine, dried over $Na_2SO_4$, and filtered, and the solvent was removed in vacuo. The residue was purified by silica gel column chromatography (100% dichloromethane to dichloromethane/methanol = 200/1) to give solid. The resulting solid was treated with diethyl ether/ethanol to give further purified (8-hydroxy-1-methyl-7-nitro-2-oxo-4-hydroquinolyl)methyl·acetate (1.9 g, yield 25%).
$^1$H-NMR Spectrum (300MHz, DMSO-$d_6$):

δ (ppm): 2.15 (s, 3H), 3.86 (s, 3H), 5.33 (s, 2H), 6.80 (s, 1H), 7.35 (d, J=9.0Hz, 1H), 7.81 (d, J=9.0Hz, 1H)

To a suspension of (8-hydroxy-1-methyl-7-nitro-2-oxo-4-hydroquinolyl)methyl· acetate (1.9 g, 6.5 mmol) in ethanol/water (150 mL/75 mL), $Na_2S_2O_4$ (2.26 g, 13.0 mmol) was added at room temperature, and the resulting mixture was stirred at 75°C for 30 minutes. The reaction mixture was cooled, diluted with water, and extracted with ethyl acetate/methanol (20/1). The combined organic layer was washed with water and brine, dried over $Na_2SO_4$, and filtered. The solvent was removed in vacuo, and the residue was treated with ethanol/diethyl ether to give (7-amino-8-hydroxy-1-methyl-2-oxo-4-hydroquinolyl)methyl·acetate as yellow solid (1.15 g, yield 65%).
$^1$H-NMR Spectrum (300MHz, DMSO-$d_6$):

δ (ppm): 2.14 (s, 3H), 3.79 (s, 3H), 5.22 (s, 2H), 6.19 (s, 1H), 6.67 (d, J=9.0Hz, 1H), 7.06 (d, J=9.0Hz, 1H)

A mixture of (7-amino-8-hydroxy-1-methyl-2-oxo-4-hydroquinolyl)methyl· acetate (1.15 g, 4.39 mmol) and acetic anhydride (1.8 g, 17.6 mmol) in a solution of water/DMF (35 mL/130 mL) was stirred overnight at room temperature. The reaction mixture was poured into water. The precipitates were collected by filtration, and dried in vacuo to give [7-(acetylamino)-8-acetyloxy-1-methyl-2-oxo-4-hydroquinolyl]methyl· acetate as white solid (1.2 g, yield 79%).

A mixture of [7-(acetylamino)-8-acetyloxy-1-methyl-2-oxo-4-hydroquinolyl]methyl·acetate (1.2 g, 3.46 mmol) in a solution of methanol/water (50 mL/5 mL) was stirred under reflux for 2.5 hours. Then, the reaction mixture was concentrated, and the solid was collected by filtration, and dried in vacuo to give [7-(acetylamino)-8-hydroxy-1-methyl-2-oxo-4-hydroquinolyl] methyl·acetate as white solid (0.89 g, yield 84.7%).

To a solution of [7-(acetylamino)-8-liydroxy-1-metliyl-2-oxo-4-hydroquinolyl]methyl· acetate (500 mg, 1.64 mmol) in DMF (20 mL), $CH_2I_2$ (440 mg, 16.5 mmol) and $C_{S2}CO_3$ (1.15 g, 4.94 mmol) were added at room temperature. The reaction mixture was stirred at 40°C for 3 hours, and quenched with water. The mixture was extracted with ethyl acetate, and the combined organic layer was washed with water and brine, dried over $Na_2SO_4$, and filtered. The solvent was removed in vacuo, and the residue was treated with ethyl acetate/diethyl ether to give (3-acetyl-9-methyl-8-oxo-9-hydro-2H-1,3-oxazolino[4,5-h]quinolin-6-yl)methyl· acetate as brown solid (380 mg, yield 73%).
$^1$H-NMR Spectrum (300MHz, DMSO-$d_6$):

δ (ppm): 2.15s, 3H), 3.86 (s, 3H), 5.33 (s, 2H), 6.80 (s, 1H), 7.35 (d, J=9.0Hz, 1H), 7.81 (d, J=9.0Hz, 1H)

To a suspension of (3-acetyl-9-methyl-8-oxo-9-liydro-2H-1,3-oxazolino[4,5-h]quinolin-6-yl)methyl acetate (380 mg, 120 mmol) in tetrahydrofuran/methanol (38 mL/38 mL), acetyl chloride (190 μL) was added at 0 to 5°C. The reaction mixture was stirred at room temperature for 2 days, and then diluted with water. The mixture was concentrated at 40°C, and extracted with ethyl acetate. The combined organic layer was washed with brine, dried over $Na_2SO_4$, and filtered. The solvent was removed in vacuo, and the residue was purified by preparative HPLC to give the compound J-125a, 3-acetyl-6-(hydroxymethyl)-9-methyl-8-oxo-9-hydro-2H-1,3-oxazolino[4,5-h]quinoline (52 mg, yield 15%), which was dried by lyophilization (tea green powder).
$^1$H-NMR Spectrum (300MHz, $CDCl_3$):

δ (ppm): 2.14 (s, 3H), 3.77 (s, 3H), 4.67 (d, J=4.5Hz, 2H), 5.48 (t, J=5.7, 1H), 6.09 (s, 1H), 6.55 (s, 1H), 7.28 (d, J=8.7Hz, 1H), 7.74 (d, J=8.1Hz, 1H)
MS Calcd.: 274
MS Found: 275 ($[M+1]^+$)

Example 5: Synthesis of the compound represented by the structural formula (5) (compound J-2.1.1)

To 8-hydroxy-4-methylquinolin-2(1H)-one (1.75 g, 10 mmol), chloroform (40 mL) and methanol (10 mL) were added, and the resulting mixture was cooled to 0°C, and stirred. To this solution, bromine (0.56 mL, 11 mmol) was added dropwise. After completion of the addition, the resulting mixture was stirred at 0 to 5°C for 2 hours under ice cooling to complete the reaction. This reaction mixture was poured into water (150 mL) to deposit crystals. The crystals were collected by filtration, washed with water, and dried to give 7-bromo-8-hydroxy-4-methylquinolin-2(1H)-one (1.65 g), which was used for the following step without purification.

To 7-bromo-8-hydroxy-4-methylquinolin-2(1H)-one (2.50 g, 10 mmol), and potassium carbonate (6.9 g, 50 mmol), dimethylacetamide (50 mL) was added, and the resulting mixture was heated to 55 to 60°C, and stirred. To this dispersion, dibromomethane (5.2 g, 30 mmol) was added. After completion of the addition, the resulting mixture was stirred for 3 hours to complete the reaction. After completion of the reaction, the reaction mixture was poured into water (200 mL), and extracted with chloroform (100 mL). The chloroform solution was washed with saturated brine, and then dried over anhydrous sodium sulfate. Chloroform was evaporated under reduced pressure, and the residue was separated and purified by silica gel chromatography (eluent: chloroform/ethyl acetate = 20/1 (volume ratio)) to give amorphous 9-bromo-6-methyloxazolo[5,4,3-ij]quinolin-4(2H)-one (1.32 g), which was used for the following step without purification.

To 9-bromo-6-methyloxazolo[5,4,3-ij]quinolin-4(2H)-one (53.2 mg, 0.2 mmol), 1-(4-hydroxyphenyl)-3,3,4,4-tetramethylborolane-2,5-dione (52.8 mg, 0.24 mmol), potassium phosphate (212.3 mg, 1.0 mmol), $Pd(dba)_3(0)$ (9 mg, 0.01 mmol), and triphenylphosphine (10.5 mg, 0.04 mmol), toluene (10 mL) was added, the resulting mixture was heated to 65 to 70°C, and stirred for 5 hours under an argon gas flow to complete the reaction. After completion of the reaction, the reaction mixture was subjected to silica gel chromatography. By successively changing the eluent from chloroform alone to chloroform/ethyl acetate =10/1 (volume ratio), chloroform/ethyl acetate = 5/1 (volume ratio), and chloroform/ethyl acetate = 2/1 (volume ratio), separation and purification were performed. The eluted fraction containing the objective substance was concentrated under reduced pressure, and ethyl acetate (10 mL) was added to the residue to deposit crystals. The crystals were collected by filtration, and dried to give the compound J-2.1.1, 9-(hydroxyphenyl)-6-methyloxazolo[5,4,3-ij]quinolin-4(2H)-one, as white powder (15 mg, yield 26.8%).
$^1$H-NMR Spectrum (400MHz, DMSO-$d_6$):

δ (ppm): 9.78 (1H, s), 7.71 (2H, d), 7.40 (1H, d), 7.28 (1H, d), 6.89 (2H, d), 6.42 (1H, s), 6.39 (2H, s), 2.44 (3H, s)

Example 6: Synthesis of the compound represented by the structural formula (4) (compound J-2.1.2)

To 9-bromo-6-methyloxazolo[5,4,3-ij]quinolin-4(2H)-one (2.0 g, 7.5 mmol), and selenium oxide (1.25 g, 11.3 mmol), 1,2-dichlorobenzene (15 mL) was added, and the resulting mixture was heated to 145 to 150°C, and stirred for 4 hours to complete the reaction. After completion of the reaction, chloroform (20 mL) was added to the reaction mixture, and the resulting mixture was subjected to silica gel chromatography. Separation and purification were performed by the column chromatography using chloroform as the eluent. The eluted fraction containing the objective substance was concentrated under reduced pressure to give 9-bromo-4-oxo-2,4-dihydrooxazolo[5,4,3-ij]quinoline-6-carboaldehyde as crystals, and this compound was used for the following step without purification.

To 9-bromo-4-oxo-2,4-dihydrooxazolo[5,4,3-ij]quinoline-6-carboaldehyde (0.84 g, 3.0 mmol), methanol (20 mL) was added to give a dispersion. To this dispersion, $NaBH_4$ (340 mg) was added at room temperature. After completion of the addition, the resulting mixture was stirred at room temperature for 2 hours to complete the reaction, and then water (50 mL) was added to the mixture to deposit crystals. The crystals were collected by filtration, washed with water, and dried to give 9-bromo-6-(hydroxymethyl)oxazolo[5,4,3-ij]quinolin-4(2H)-one, and this compound was used for the

following step without purification.

To Pd(dba)$_3$ (8 mg, 0.0087 mmol), and triphenylphosphine (5 mg, 0.19 mmol), dimethyl ether (10 mL) was added, and the resulting mixture was heated to 60°C, and stirred under an argon gas flow for 30 minutes. To this solution, potassium phosphate (105 mg), 1-(4-hydroxyphenyl)-3,3,4,4-tetramethylborolane-2,5-dione (54 mg, 2.45 mmol), and 9-bromo-6-(hydroxymethyl)oxazolo[5,4,3-ij]quinolin-4(2H)-one (46 mg, 0.163 mmol) were successively added. After completion of the addition, the resulting mixture was heated to 95 to 100°C, and stirred for 10 hours to complete the reaction. After completion of the reaction, the reaction mixture was cooled to room temperature, and then poured into water (50 mL), and concentrated hydrochloric acid was added to the resulting mixture to adjust the mixture to pH 5 to 6, and thereby deposit crystals. The crystals were separated and purified by silica gel chromatography (eluent: chloroform, and then chloroform/ethyl acetate = 2/1 to 1/1 (volume ratio)). The eluted fraction containing the objective substance was concentrated under reduced pressure, and methanol was added to the residue to deposit crystals. The crystals were collected by filtration, and dried to give the compound J-2.1.2, 6-(hydroxymethyl)-9-(4-hydroxyphenyl)oxazolo[5,4,3-ij]quinolin-4(2H)-one, as beige powder (16.7 mg, yield 34.7%).
$^1$H-NMR Spectrum (400MHz, DMSO-d$_6$):

δ (ppm): 9.78 (1H, s), 7.70 (2H, d), 7.34 (1H, d), 7.26 (1H, d), 6.55 (1H, s), 6.39 (2H, s), 5.63 (1H, t), 4.77 (2H, d)

Example 7: Synthesis of the compound represented by the structural formula (8) (compound J-146)

To 3,4-difluoroaniline (6.46 g, 0.05 mol), acetonitrile (20 mL) was added, and the resulting mixture was stirred at 5 to 10°C. To this solution, diketene (5.0 g, 0.05 mol) was added dropwise. After completion of the addition, the resulting mixture was stirred at 5°C for 30 minutes, and then at room temperature for 1 hour to complete the reaction. After completion of the reaction, the reaction mixture was poured into water (300 mL) to deposit crystals. The crystals were collected by filtration, washed with water, and dried to give N-(3,4-difluoroplienyl)-3-oxobutanamide (7.90 g, yield 74.1%), which was used for the following step without purification.

To N-(3,4-difluorophenyl)-3-oxobutanamide (5.5 g, 0.0258 mol), acetic acid (30 mL) was added, and the resulting mixture was stirred at room temperature. To this solution, bromine (4.54 g, 0.0284 mol) was added dropwise. After completion of the addition, the resulting mixture was stirred at room temperature for 4 hours, and left standing overnight. To this reaction mixture, acetone (5 mL) was added, and the resulting mixture was stirred at room temperature for 3 hours. Then, ethyl acetate (200 mL) and water (300 mL) were added to the mixture for extraction. This ethyl acetate solution was washed with saturated brine, then washed with saturated aqueous sodium hydrogencarbonate, and then dried over anhydrous sodium sulfate. Ethyl acetate was evaporated under reduced pressure to give 4-bromo-N-(3,4-difluorophenyl)-4-methyl-3-oxopentanamide as oil (7.50 g), which was used for the following step without purification.
$^1$H-NMR Spectrum (400MHz, $CDCl_3$):

δ (ppm): 8.85 (0.72H, brs), 7.64-7.58 (1H, m), 7.30 (0.28H, brs), 7.13-7.09 (2H, m), 5.29 (0.28H, s), 4.11 (1.72H, s), 3.86 (0.28H, s), 3.83 (1.72H, s)

To 4-bromo-N-(3,4-difluorophenyl)-4-methyl-3-oxopentanamide (7.50 g, 0.0257 mol), concentrated sulfuric acid (35 mL) was added, and the resulting mixture was heated to 75 to 80°C, and stirred for 4 hours, and then left standing overnight. To this reaction mixture, concentrated sulfuric acid (10 mL) was further added, and the resulting mixture was heated to 75 to 80°C, and stirred for 3 hours to complete the reaction. After completion of the reaction, the reaction mixture was cooled to room temperature, and poured into ice water (300 mL) with stirring to deposit crystals. The crystals were collected by filtration, washed with water, and dried. Then, acetonitrile (75 mL) was added to the crystals, and they were dispersed by stirring at room temperature. The crystals were collected by filtration, and dried to give 4-(bromomethyl)-6,7-difluoroquinolin-2(1H)-one (5.0 g, yield 66.2%), which was used for the following step without purification.

To sodium acetate (4.5 g), DMSO (20 mL) was added, and the resulting mixture was stirred at room temperature. To this dispersion, 4-(bromomethyl)-6,7-difluoroquinolin-2(1H)-one (5.0 g, 18.2 mmol) was added. This solution was stirred at room temperature for 2 hours to complete the reaction. After completion of the reaction, the reaction mixture was poured into water (150 mL) to deposit crystals. The crystals were collected by filtration, and washed with water to give (6,7-difluoro-2-oxo-1,2-dihydroquinolin-4-yl)methyl·acetate in a wet state.

To the wet (6,7-difluoro-2-oxo-1,2-dihydroquinolin-4-yl)methyl·acetate, methanol (35 mL) was added, and the resulting mixture was stirred at room temperature. To this dispersion, an aqueous solution containing sodium hydroxide (1.5 g) dissolved in water (15 mL) was added. After completion of the addition, the mixture was stirred at room temperature for 2 hours to complete the reaction, and then diluted with water (100 mL). To this aqueous solution, concentrated hydrochloric acid was added dropwise to adjust the solution to pH 4 to 5 to deposit crystals. The crystals were collected by filtration, washed with water, then dispersed in acetonitrile (50 mL) for washing, collected by filtration, and dried to give 6,7-difluoro-4-(hydroxymethyl)quinolin-2(1H)-one (3.88 g, yield 72.0% for 2 steps). The resulting compound was

used for the following step without purification.

To 6,7-difluoro-4-(hydroxymethyl)quinolin-2(1H)-one (1.06 g, 5 mmol), and 1-methylpiperazine (2.0 g, 20 mmol), DMSO (10 mL) was added, and the resulting mixture was heated to 80 to 85°C, and stirred for 20 hours. After completion of the reaction, water (25 mL) was added dropwise to the reaction mixture to deposit crystals, and the crystals were collected by filtration, and washed with water. The crystals were dispersed in a mixture of acetonitrile (25 mL) and water (50 mL) for washing, collected by filtration, and dried to give 6-fluoro-4-(hydroxymethyl)-7-(4-methylpiperazin-1-yl)quinolin-2(1H)-one (1.10 g, yield 75.3%).

$^1$H-NMR Spectrum (400MHz, DMSO-$d_6$):

δ (ppm): 11.45 (1H, s), 7.41 (1H, d), 6.88 (1H, d), 6.40 (1H, s), 5.46 (1H, t), 4.64 (2H, d), 3.07-3.06 (4H, brm), *2.51-2.5 (4H, br), 2.23 (3H, s)

*Around 2.51 to 2.5, the peak overlapped with that of proton of the dimethyl group of DMSO, and therefore the integral value was not determined.

To cyclopropylboronic acid hydrate (0.73 g, 7 mmol), benzene (20 mL) was added, and the resulting mixture was heated with bubbling nitrogen gas to evaporate benzene. Dry toluene (30 mL) was added to the residue, and the resulting mixture was stirred. To this dispersion, 6-fluoro-4-(hydroxylmethyl)-7-(4-methylpiperazin-1-yl)quinolin-2(1H)-one (1.02 g, 3.5 mmol), dimethylaminopyridine (1.28 g, 10.5 mmol), copper acetate (0.64 g, 3.5 mmol), and sodium bis(trimethylsilyl)amide (NaHMDS, 1.9 M solution in THF, 2.1 mL, 3.9 mmol) were successively added. After completion of the addition, the resulting mixture was heated to 90 to 100°C, and stirred for 2 hours with bubbling air. After completion of the reaction, the reaction mixture was cooled to room temperature, chloroform (100 mL) and methanol (5 mL) were added to the reaction mixture, and the resulting mixture was stirred. The insoluble matter was removed by using Celite, and then the filtrate was concentrated under reduced pressure. The residue was separated and purified by silica gel column chromatography (eluent: chloroform, and then chloroform/methanol = 20/1 to 10/1 (volume ratio)). The eluted fraction containing the objective substance was concentrated under reduced pressure, ethyl acetate was added to the residue to deposit crystals. The crystals were collected by filtration, and dried to give the compound J-146, 1-cyclopropyl-6-fluoro-4-(hydroxymethyl)-7-(4-methylpiperazin-1-yl)quinolin-2(1H)-one, as white powder (130 mg, yield 11.0%). The HPLC purify (254 nm) of the product was 95.98%.

$^1$H-NMR Spectrum (400MHz, $CDCl_3$):

δ (ppm): 7.33-7.28 (2H, m), 6.65 (1H, s), 4.79 (2H, s), 3.24 (4H, t), 2.91-2.83 (1H, m), 2.65 (4H, t), 2.38 (3H, s), 1.37-1.28 (2H, m), 0.87-0.83 (2H, m)

Example 8: Synthesis of the compound represented by the structural formula (7) (compound J-147)

To 6,7-difluoro-4-(hydroxymethyl)quinolin-2(1H)-one (2.11 g, 0.01 mol), 4-hydroxypiperidine (2.0 g, 0.02 mol), and diisopropylethylamine (3.1 g), DMSO (20 mL) was added, and the resulting mixture was heated to 85 to 90°C, and stirred for 12 hours. After completion of the reaction, the reaction mixture was poured into water (200 mL) with stirring to deposit crystals. The crystals were collected by filtration, washed with water, and dried. The crystals were separated and purified by silica gel chromatography (eluent: chloroform, and then chloroform/methanol = 20/1 to 4/1 (volume ratio)). The eluted fraction containing the objective substance was concentrated under reduced pressure, and ethyl acetate was added to the residue to deposit crystals. The crystals were collected by filtration, and dried to give 6-fluoro-4-(hydroxymethyl)-7-(4-hydroxypiperidin-1-yl)quinolin-2(1H)-one (1.5 g, yield 51.3%). Although the objective substance obtained in this synthesis contained about 10% of the starting material compound, it was used for the following step without purification.

To cyclopropylboronic acid hydrate (0.84 g, 8.1 mmol), benzene (20 mL) was added, and the resulting mixture was heated under a nitrogen flow to evaporate benzene. Toluene (20 mL) was added to the residue, and the resulting mixture was stirred at room temperature. To this dispersion, 6-fluoro-4-(hydroxymethyl)-7-(4-hydroxypiperidin-1-yl)quinolin-2(1H)-one (1.18 g, 4.04 mmol), dimethylaminopyridine (1.48 g, 12.1 mmol), copper acetate (0.72 g, 4.04 mmol), and NaHMDS (1.9 M solution in THF, 2.6 mL, 4.85 mmol) were successively added. After completion of the addition, the reaction mixture was heated to 95 to 100°C, and stirred for 2 hours with bubbling air. After completion of the reaction, the reaction mixture was cooled to room temperature, and poured into water (100 mL) with stirring. The deposited crystals were collected by filtration, washed with water, and dried. The crystals were separated and purified by silica gel column chromatography (eluent: chloroform, and then chloroform/methanol = 10/1). The eluted fraction containing the objective substance was concentrated under reduced pressure, and ethyl acetate was added to the residue to deposit crystals. The crystals were collected by filtration, and dried to give the compound J-147, 1-cyclopropyl-6-fluoro-4-(hydroxymethyl)-7-(4-hydroxypiperidin-1-yl)quinolin-2(1H)-one, as pale yellow powder (430 mg, yield 32.1%). The purify of the product determined by HPLC (254 nm) was 98.1%.

$^1$H-NMR Spectrum (400MHz, DMSO-$d_6$):

δ (ppm): 7.42 (1H, d), 7.34 (1H, d), 6.42 (1H, s), 5.43 (1H, t), 4.78 (1H, d), 4.62 (2H, d), 3.71-3.66 (1H, m), 3.44-3.40 (2H, m), 2.96-2.88 (3H, m), 1.92-1.88 (2H, m), 1.62-1.53 (2H, m), 1.36-1.24 (2H, m), 0.78-0.65 (2H, m)

Example 9: Synthesis of the compound represented by the structural formula (9) (compound J-132)

[Formula 8]

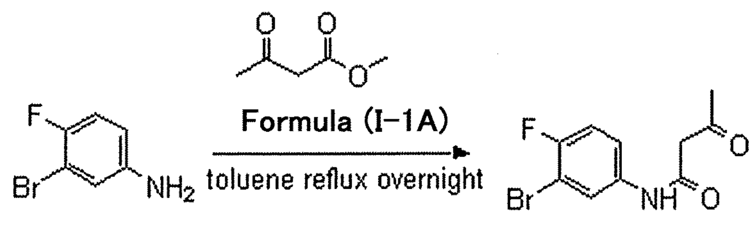


Formula (I-1) Formula (I-2)

A mixture of the compound represented by the structural formula (I-1) (7.60 g, 40 mmol), and the compound represented by the structural formula (I-1A) (5.56 g, 47.9 mmol) in toluene (70 mL) was heated to reflux, and stirred overnight. The reaction mixture was cooled to room temperature, and then concentrated under pressurization. The residue was purified by silica gel column chromatography (petroleum ether/ethyl acetate = 100:0 to 2:1) to give N-(3-bromo-4-fluorophenyl)-3-oxobutanamide (7.11 g, yield 65%) as yellow solid.
$^1$H-NMR Spectrum (300MHz, $CDCl_3$):

δ (ppm): 2.33 (s, 3H), 3.59 (s, 2H), 7.04-7.10 (m, 1H), 7.39-7.45 (m, 1H), 7.85-7.88 (m, 1H), 9.27 (brs, 1H)

A solution of N-(3-bromo-4-fluorophenyl)-3-oxobutanamide (5.46 g, 19.9 mmol) in $CF_3SO_3H$ (40 mL) was stirred at room temperature for 3 days. Then, the mixture was poured on ice (200 g), and filtered. The solid was washed with water, and ground in acetonitrile (40 mL) to give 7-bromo-6-fluoro-4-methylhydroquinolin-2-one as white solid (2.98 g, yield 58%).
$^1$H-NMR Spectrum (300MHz, **DMSO-$d_6$):**

δ (ppm): 2.39 (s, 3H), 6.48 (s, 1H), 7.56-7.58 (m, 1H), 7.69-7.72 (m, 1H), 11.68 (brs, 1H)

To a solution of 7-bromo-6-fluoro-4-methylhydroquinolin-2-one (290 mg, 1.13 mmol) and $K_2CO_3$ (234 mg, 1.70 mmol) in DMSO (4 mL), $CH_3I$ (209 mg, 1.47 mmol) was added, and the resulting mixture was stirred at 60°C for 15 hours. The reaction mixture was cooled to room temperature, then diluted with water (40 mL), and extracted with ethyl acetate (80 mL). The organic layer was washed with water (40 mL), and brine (40 mL), dried over $Na_2SO_4$, filtered, and concentrated. The residue was ground in petroleum ether/ethyl acetate = 20:1 to give 7-bromo-6-fluoro-1,4-dimethylhydroquinolin-2-one as yellow solid (198 mg, yield 65%).
$^1$H-NMR Spectrum (300MHz, $CDCl_3$):

δ (ppm): 2.41 (s, 3H), 3.67 (s, 3H), 6.64 (s, 1H), 7.41 (d, J=9.3Hz, 1H), 7.56 (d, J=5.7Hz, 1H)

A mixture of 7-bromo-6-fluoro-1,4-dimethylhydroquinolin-2-one (198 mg, 0.733 mmol) and $SeO_2$ (488 mg, 4.40 mmol) in xylene (4 mL) was heated to reflux, and stirred for 2 days. The mixture was cooled to room temperature, and then the resulting mixture was filtered, and concentrated under reduced pressure. The residue was purified by TLC (dichloromethane/methanol = 30:1) to give 7-bromo-6-fluoro-1-methyl-2-oxohydroquinoline-4-carboaldehyde as yellow solid (135 mg, yield 65%).
$^1$H-NMR Spectrum (300MHz, $CDCl_3$):

δ (ppm): 3.75 (s, 3H), 7.23 (s, 1H), 7.63 (d, J=6.0Hz, 1H), 8.71 (d, J=9.6Hz, 1H), 10.07 (s, 1H)

To a solution of 7-bromo-6-fluoro-1-methyl-2-oxohydroquinoline-4-carboaldehyde (135 mg, 0.475 mmol) in methanol (5 mL), $NaBH_4$ (36 mg, 0.95 mmol) was added at room temperature, and then the resulting mixture was stirred at room temperature for 0.5 hour. The resulting mixture was diluted with ethyl acetate (50 mL), and water (25 mL), and extracted with ethyl acetate (50 mL). The organic layer was washed with water (40 mL) and brine (40 mL), dried over $Na_2SO_4$, filtered, and concentrated to give 7-bromo-6-fluoro-4-(hydroxymethyl)-1-methylhydroquinolin-2-one as white solid (131 mg, yield 94%).
$^1$H-NMR Spectrum (300MHz, $CDCl_3$):

δ (ppm): 2.40 (brs, 1H), 3.66 (s, 3H), 4.86 (d, J=4.8Hz, 2H), 6.89 (s, 1H), 7.47 (d, J=9.0Hz, 1H), 7.56 (d, J=6.0Hz, 1H)

[Formula 9]

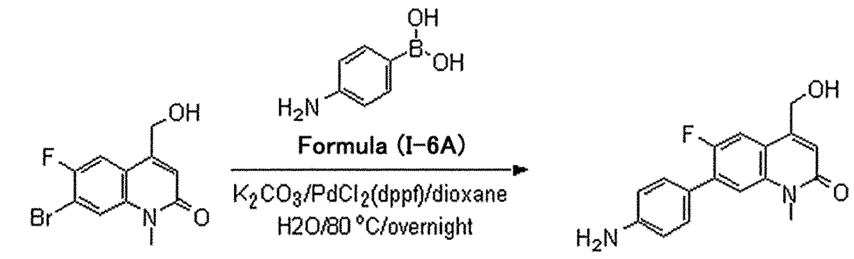


Formula (I-6) Formula (9)

A mixture of 7-bromo-6-fluoro-4-(hydroxymethyl)-1-methylhydroquinolin-2-one (200 mg, 0.699 mmol), the compound represented by the structural formula (I-6A) (115 mg, 0.839 mmol), $K_2CO_3$(145 mg, 1.05 mmol), and 1,1'-bis(diphenylphosphino)ferrocene-palladium(II) dichloride-dichloromethane complex (Pd(dppf)$Cl_2$, 57 mg, 0.07 mmol) in dioxane/water = 10/1 (11 mL) was heated to 80°C, and the resulting mixture was stirred overnight under a nitrogen gas atmosphere. The mixture was cooled to room temperature, and then concentrated under reduced pressure, and water was added to the residue. The suspended solid was collected by filtration, washed with water, and ground in ethyl acetate and ethyl acetate/methanol = 2:1 to give the compound J-132, 7-(4-aminophenyl)-6-fluoro-4-(hydroxymethyl)-1-methylhydroquinolin-2-one, as brown solid (128 mg, yield 61%).
$^1$H-NMR Spectrum (300MHz, DMSO-$d_6$):

δ (ppm): 3.66 (s, 3H), 4.73 (d, J=5.4Hz, 2H), 5.45 (s, 2H), 5.54 (t, J=5.7Hz, 1H), 6.66-6.69 (m, 3H), 7.39-7.46 (m, 3H), 7.57 (d, J=12.3Hz, 1H)
MS Calcd.: 298
MS Found: 299 ($[M+H]^+$)

Example 10: Synthesis of the compound represented by the structural formula (14) (compound J-131)

[Formula 10]

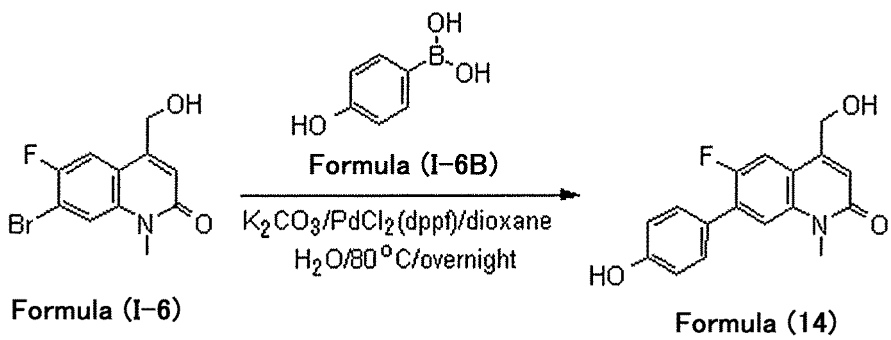


Formula (I-6) Formula (14)

A mixture of 7-bromo-6-fluoro-4-(hydroxymethyl)-1-methylhydroquinolin-2-one (131 mg, 0.458 mmol) represented by the structural formula (I-6), the compound represented by the structural formula (I-6B) (76 mg, 0.55 mmol),

$K_2CO_3$ (95 mg, 0.69 mmol), and Pd(dppf)$Cl_2$ (37 mg, 0.05 mmol) in dioxane/water = 10/1 (5.5 mL) was heated to 80°C, and stirred overnight under a nitrogen gas atmosphere. The mixture was cooled to room temperature, and then concentrated under reduced pressure, water (50 mL) was added to the residue, and the resulting mixture was extracted with ethyl acetate (100 mL). The organic layer was washed with brine (50 mL), dried over $Na_2SO_4$, filtered, and concentrated. The residue was purified by thin layer chromatography (dichloromethane/methanol = 15:1) to give the compound J-131, 6-fluoro-4-(hydroxymethyl)-7-(4-hydroxyphenyl)-1-methylhydroquinolin-2-one, as brown solid (82 mg, yield 60%).
$^1$H-NMR Spectrum (300MHz, DMSO-$d_6$):

δ (ppm): 3.67 (s, 3H), 4.74 (d, J=5.4Hz, 2H), 5.53 (t, J=5.4Hz, 1H), 6.68 (s, 1H), 6.91 (d, J=8.7Hz, 2H), 7.48-7.54 (m, 3H), 7.61 (d, J=12.3Hz, 1H), 9.75 (s, 1H)
MS Calcd.: 299
MS Found: 300 ($[M+1]^+$)

Example 11: Synthesis of the compound represented by the structural formula (11) (compound J-121)

To a mixture of 3-bromoaniline (5.00 g, 29.1 mmol), $K_2CO_3$ (12.1 g, 87.2 mmol), and DMF (10 mL), iodomethane (4.95 g, 34.9 mmol) was added, and the resulting mixture was stirred at 60°C for 2 hours with heating. Water (30 mL) was added to the reaction mixture, and the resulting mixture was extracted with ethyl acetate. The organic layer was washed with saturated brine, and dried over sodium sulfate, and the solvent was evaporated under reduced pressure. The resulting crude product was purified by silica gel column chromatography (ethyl acetate:hexane = 1:3) (Rf value, 0.44) to give N-methyl-3-bromoaniline (2.45 g, 13.1 mmol, yield 45%).
$^1$H-NMR Spectrum (300MHz, $CDCl_3$):

δ (ppm): 2.82 (3H, s), 3.78 (1H, br), 6.51 (1H, d, J=8.1Hz), 6.73 (1H, s), 6.81 (1H, d, J=8.1Hz), 7.00 (1H, t, J=8.1Hz)

[Formula 11]

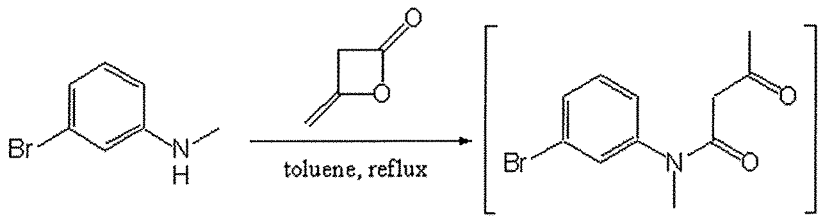


Formula (K-2)

Formula (K-3)

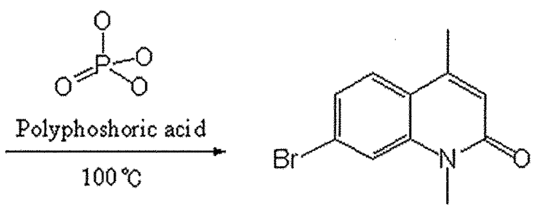


Formula (K-4)

A mixture of N-methyl-3-bromoaniline (K-2) (2.45 g, 13.1 mmol), diketene (1.22 g, 14.5 mmol), and toluene (10 mL) was heated to 100°C, and stirred for 6 hours. The solvent was evaporated under reduced pressure, the resulting compound represented by the structural formula (K-3) was added to polyphosphoric acid (10 mL) heated to 100°C, and the resulting mixture was stirred for 5 hours with heating. The reaction mixture was cooled to room temperature, water (100 mL) was added to the mixture, and the deposited solid was collected by filtration, and washed with hexane to give the compound represented by the structural formula (K-4) (631 mg, 2.50 mmol, yield 19%).
$^1$H-NMR Spectrum (300MHz, $CDCl_3$):

δ (ppm): 2.44 (3H, s), 3.67 (3H, s), 6.60 (1H, s), 7.37 (1H, dd, J=8.1Hz, 1.8Hz), 7.53-7.56 (2H, m)

[Formula 12]

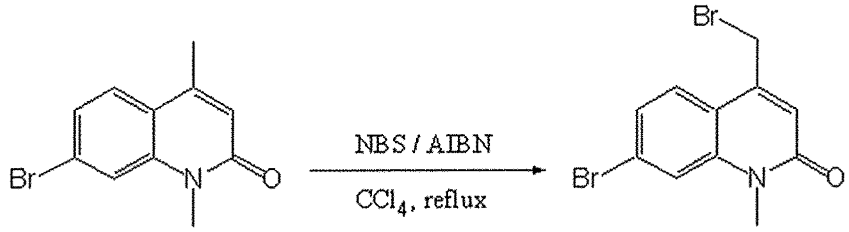


Formula (K-4) Formula (K-5)

A mixture of the compound represented by the structural formula (K-4) (380 mg, 1.51 mmol), N-bromosuccinimide (295 mg, 1.66 mmol), carbon tetrachloride (35 mL), and a catalytic amount of azoisobutyronitrile was heated to reflux. A catalytic amount of azoisobutyronitrile was added every 1 hour, and N-bromosuccinimide (402 mg, 0.75 mmol) was added every 3 hours. The reaction mixture was heated to reflux for 18 hours in total, and then the solvent was evaporated under reduced pressure. The crude product was purified by silica gel column chromatography (chloroform) to give the compound represented by the structural formula (K-5) (220 mg, 0.665 mmol, yield 44%).
$^1$H-NMR Spectrum (300MHz, $CDCl_3$):

δ (ppm): 3.69 (3H, s), 4.54 (2H, s), 6.81 (1H, s), 7.43 (1H, dd, J=8.4Hz, 1.8Hz), 7.58 (1H, d, J=1.8Hz), 7.70 (1H, d, J=8.4Hz)

[Formula 13]

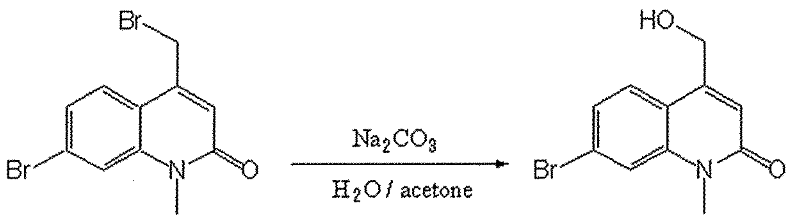


Formula (K-5) Formula (K-6)

To a suspension of the compound represented by the structural formula (K-5) (220 mg, 0.665 mmol) and acetone (10 mL), 2 M aqueous sodium carbonate (10 mL) was added, and the resulting mixture was stirred at 80°C for 10 hours with heating. 2 M Aqueous HCl was added to the reaction mixture to adjust the mixture to pH 4, and the mixture was extracted with ethyl acetate. The organic layer was washed with saturated brine, and dried over sodium sulfate, and the solvent was evaporated under reduced pressure. To the resulting crude product, chloroform (1 mL) and hexane (10 mL) were added to crystallize the product to give the compound represented by the structural formula (K-6) (170 mg, 0.634 mmol, yield 95%).
$^1$H-NMR Spectrum (300MHz, $CDCl_3$):

δ (ppm): 3.66 (3H, s), 4.91 (2H, s), 6.87 (1H, s), 7.37 (1H, dd, J=8.4Hz, 1.8Hz), 7.54-7.58 (2H, m)

OH could not be identified.

[Formula 14]

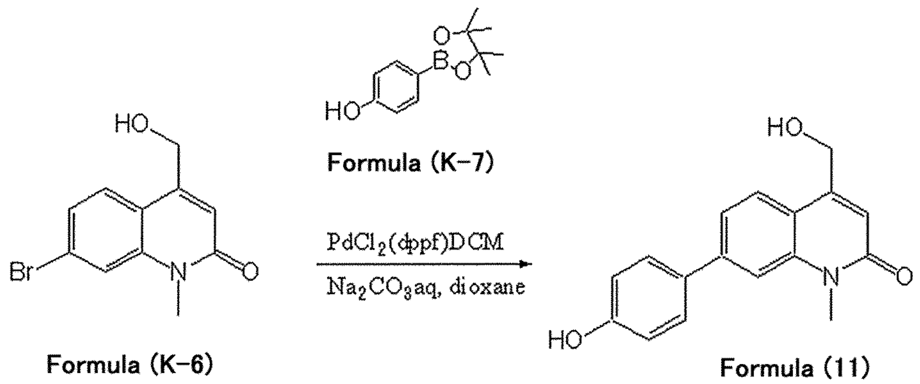


Formula (K-7)

Formula (K-6)

Formula (11)

To a mixture of the compound represented by the structural formula (K-6) (50 mg, 0.186 mmol), the compound represented by the structural formula (K-7) (45 mg, 0.205 mmol), dioxane (10 mL), and 2 M aqueous sodium carbonate (1 mL), a catalytic amount of 1,1'-bis(diphenylphosphino)ferrocene-palladium(II) dichloride-dichloromethane complex was added under an argon gas atmosphere, and the resulting mixture was heated to 100°C, and stirred for 4.5 hours. 2 M Aqueous sodium hydroxide was added to the reaction mixture to adjust it to pH 10. The mixture was filtered through Celite, and 2 M aqueous HCl was added to the filtrate to adjust it to pH 3. Ethyl acetate was added to the mixture for extraction. The organic layer was washed with saturated brine, and dried over sodium sulfate, and the solvent was evaporated under reduced pressure. The resulting crude product was purified by silica gel column chromatography (chloroform:methanol = 5:1) (Rf value, 0.47) to give the compound J-121 represented by the structural formula (11) (25 mg, yield 48%).
$^1$H-NMR Spectrum (300MHz, DMSO-$d_6$):

δ (ppm): 3.70 (3H, s), 4.78 (2H, d, J=5.7Hz), 5.50 (1H, t, J=5.7Hz), 6.64 (1H, s), 6.90 (2H, d, J=8.7Hz), 7.49 (1H, dd, J=8.4Hz, 1.8Hz), 7.62-7.68 (3H, m), 7.75 (1H, d, J=8.4Hz), 9.68 (1H, s)

Example 12: Synthesis of the compound represented by the structural formula (12) (compound J-121Me)

[Formula 15]

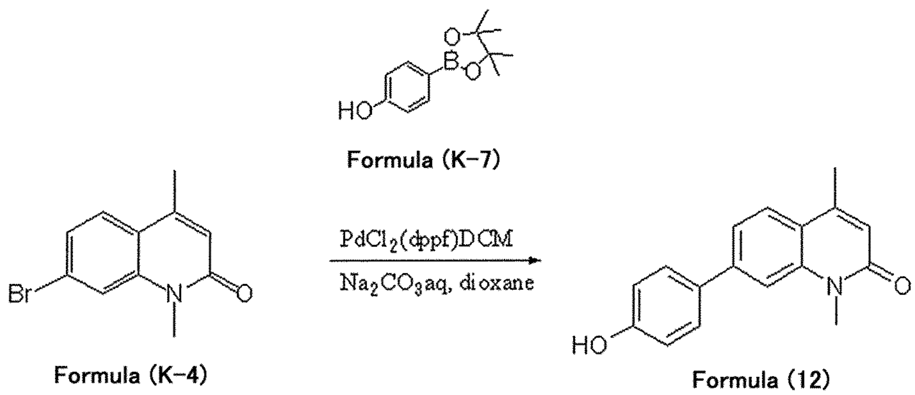


Formula (K-7)

Formula (K-4)

Formula (12)

To a mixture of the compound represented by the structural formula (K-4) (250 mg, 0.992 mmol), the compound represented by the structural formula (K-7) (240 mg, 1.09 mmol), dioxane (5 mL), and 2 M aqueous sodium carbonate (1.5 mL), a catalytic amount of 1,1'-bis(diphenylphosphino)ferrocene-palladium(II) dichloride-dichloromethane complex was added under an argon gas atmosphere, and the resulting mixture was heated to 100°C, and stirred for 2.5 hours. The mixture was filtered through Celite, and 2 N aqueous HCl was added to the filtrate to adjust it to pH 5. To the mixture, ethyl acetate was added for extraction. The organic layer was washed with saturated brine, and dried over sodium sulfate, and the solvent was evaporated under reduced pressure. The residue was washed by suspending it in a small

volume of methanol, and then collected by filtration to give the compound represented by the structural formula (12), J-121Me (40 mg, yield 15%).
$^1$H-NMR Spectrum (300MHz, DMSO-$d_6$):

δ (ppm): 2.44 (3H, s), 3.68 (3H, s), 6.49 (1H, s), 6.90 (2H, d, J=8.4Hz), 7.52 (1H, dd, J=8.1Hz, 1.5Hz), 7.61 (1H, s), 7.67 (2H, d, J=8.4Hz), 7.80 (1H, d, J=8.1Hz), 9.69 (1H, s)

Example 13: Synthesis of the compound represented by the structural formula (15) (compound J-157)

[Formula 16]

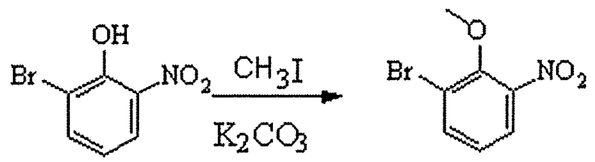


Formula (O-1) Formula (O-2)

To a mixture of the compound represented by the structural formula (O-1) (19.6 g, 90.0 mmol) and potassium carbonate (24.9 g, 180 mmol) in acetone (400 mL), iodomethane (25.6 g, 180 mmol) was added. The mixture was refluxed overnight. After cooling, the mixture was filtered, and washed with ethyl acetate (100 mL). The filtrate was concentrated in vacuo. The residue was dissolved in ethyl acetate, the solution was washed with brine, dried over $Na_2SO_4$, and concentrated to give 1-bromo-2-methoxy-3-nitrobenzene represented by the structural formula (O-2) as yellow solid (20.2 g, yield 97%).
$^1$H-NMR Spectrum (300MHz, DMSO-$d_6$):

δ (ppm): 3.92 (s, 3H), 7.32 (d, 1H, J=8.1Hz), 7.94-8.03 (m, 2H)

A mixture of 1-bromo-2-methoxy-3-nitrobenzene (20.1 g, 86.6 mmol) and iron powder (33.4 g, 598 mmol) in acetic acid/water (1:1, 600 mL) was heated to 80°C for 1.5 hours. After the mixture was cooled and filtered, the solid was washed with ethyl acetate and water. The organic layer was washed with saturated aqueous $NaHCO_3$, dried, and concentrated. The residue was purified by column chromatography (petroleum ether:ethyl acetate = 20:1 to 10:1) to give 3-bromo-2-methoxyphenylamine as yellow solid (17.4 g, yield 99%).
$^1$H-NMR Spectrum (300MHz, DMSO-$d_6$):

δ (ppm): 3.66 (s, 3H), 5.23 (s, 2H), 6.64-6.76 (m, 3H)

A solution of 3-bromo-2-methoxyphenylamine (16.8 g, 83.2 mmol) and 2-oxetanone (8.40 g, 99.8 mmol) in toluene was stirred at 140°C for 6 hours. The solution was cooled to room temperature, and stirred overnight. The reaction mixture was concentrated in vacuo. The residue was purified by column chromatography (petroleum ether:ethyl acetate = 10:1 to 3:1) to give N-(3-bromo-2-methoxyphenyl)-3-oxobutanamide as white solid (15.6 g, yield 66%).
$^1$H-NMR Spectrum (300MHz, DMSO-$d_6$):

δ (ppm): 2.20 (s, 3H), 3.72 (s, 2H), 3.74 (s, 3H), 7.05 (d, 1H, J=8.1Hz), 7.35 (dd, 1H, J=8.1, 1.5Hz), 8.05 (dd, 1H, J=8.4, 1.8Hz), 9.77 (s, 1H)

A mixture of N-(3-bromo-2-methoxyphenyl)-3-oxobutanamide (5.72 g, 20.0 mmol) and $CF_3SO_3H$ (75.0 g, 50.0 mmol) in dichloromethane (60 mL) was stirred overnight at room temperature. The mixture was poured into ice water, and the resulting mixture was filtered. The residue was purified by column chromatography (petroleum ether:ethyl acetate = 5:1) to give 7-bromo-8-methoxy-4-methylhydroquinolin-2-one as white solid (4.13 g, yield 77%).
$^1$H-NMR Spectrum (300MHz, DMSO-$d_6$):

δ (ppm): 2.40 (d, 3H, J=1.2Hz), 3.81 (s, 3H), 6.46 (s, 1H), 7.37-7.44 (m, 2H), 11.23 (brs, 1H)

A mixture of 7-bromo-8-methoxy-4-methylhydroquinolin-2-one (3.60 g, 13.4 mmol), cyclopropylboronic acid (2.31 g, 26.9 mmol), anhydrous cuprous acetate (2.44 g, 13.4 mmol), N,N-dimethyl-4-aminopyridine (DMAP, 4.92 g, 40.3 mmol), lithium bis(trimethylsilyl)amide (LiHMDS, 20 mL, 1 N), and toluene (100 mL) was stirred overnight at 95°C in the air. After cooling, the mixture was concentrated in vacuo. The residue was extracted with ethyl acetate (3 x 50 mL), and the organic layer was washed with brine, dried, and concentrated. The residue was purified by column chromatography (petroleum ether:ethyl acetate = 7:1) to give 7-bromo-1-cyclopropyl-8-methoxy-4-methylhydroquinolin-2-one as white solid (418 mg, yield 10%).
$^1$H-NMR Spectrum (300MHz, $CDCl_3$):

δ (ppm): 0.56-0.58 (m, 2H), 1.16-1.23 (m, 2H), 2.37 (s, 3H), 3.36-3.43 (m, 1H), 3.72 (s, 3H), 6.47 (d, 1H, J=1.2Hz), 7.24-7.27 (m, 1H), 8.09 (d, 1H, J=8.7Hz)

A suspension of 7-bromo-1-cyclopropyl-8-methoxy-4-methylhydroquinolin-2-one (110 mg, 0.36 mmol) and $SeO_2$ (396 mg, 3.60 mmol) in xylene (20 mL) was stirred overnight at 150°C. Then, $SeO_2$ (396 mg, 3.60 mmol) was added to the reaction mixture, and the resulting mixture was stirred overnight at 150°C. The mixture was cooled to room temperature, then water (50 mL) was added to the mixture, and the resulting mixture was extracted with ethyl acetate (3 x 50 mL). The combined organic layer was washed with brine, dried, and concentrated to give 7-bromo-1-cyclopropyl-8-methoxy-2-oxohydroquinoline-4-carboaldehyde as brown solid (115 mg, yield 100%).

To an ice-cooled solution of 7-bromo-1-cyclopropyl-8-methoxy-2-oxohydroquinoline-4-carboaldehyde (115 mg, 1.21 mmol) in methanol (20 mL), sodium borohydride (92 mg, 2.42 mmol) was added portionwise. After the addition, the reaction mixture was stirred at room temperature for 0.5 hours. The reaction mixture was concentrated, and the residue was purified by preparative TLC using ethyl acetate to give 7-bromo-1-cyclopropyl-4-(hydroxymethyl)-8-methoxyhydroquinolin-2-one as yellow solid (60 mg, yield 52%).
$^1$H-NMR Spectrum (300MHz, $CDCl_3$):

δ (ppm): 0.49-0.55 (m, 2H), 1.15-1.25 (m, 2H), 3.37-3.42 (m, 1H), 3.72 (s, 3H), 4.85 (s, 2H), 6.78 (s, 1H,), 7.24-7.27 (m, 1H), 7.42 (d, 1H, J=8.7Hz)

A mixture of 7-bromo-1-cyclopropyl-4-(hydroxymethyl)-8-methoxyhydroquinolin-2-one (145 mg, 0.45 mmol), 4-hydroxyphenylboronic acid (125 mg, 0.90 mmol), $PdCl_2$(dppf) (45 mg), and potassium carbonate (125 mg, 0.67 mmol) in dioxane/water (20 mL/2 mL) was stirred overnight at 80°C under a nitrogen gas atmosphere. The reaction mixture was filtered, and concentrated. The residue was purified by column chromatography (dichloromethane to dichloromethane/methanol = 10:1) to give the compound J-157, 1-cyclopropyl-8-methoxyl-4-(hydroxymethyl)-7-(4-hydroxyphenyl)hydroquinolin-2-one, as black solid (89 mg, yield 59%).
$^1$H-NMR Spectrum (300MHz, DMSO-$d_6$):

δ (ppm): 0.45-0.46 (m, 2H), 1.07-1.15 (m, 2H), 3.23 (s, 3H), 3.29-3.33 (s, 1H), 4.68 (s, 2H), 5.46 (brs, 1H), 6.50 (s, 1H), 6.85 (d, 2H, J=8.7Hz), 7.17 (d, 1H, J=8.1Hz), 7.40-7.44 (m, 3H)
MS Calcd.: 337
MS Found: 338 ([M+1])

Example 14: Synthesis of the compound represented by the structural formula (17) (compound J-144)

[Formula 17]

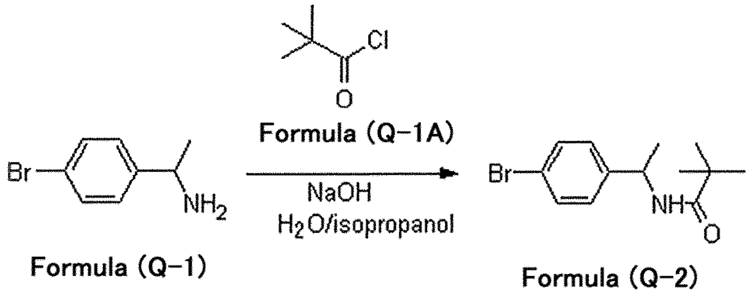

To a mixture of the compound represented by the structural formula (Q-1) (5.0 g, 25 mmol) and NaOH (2.0 g, 50 mmol) in water (18 mL) and isopropanol (18 mL), the compound represented by the structural formula (Q-1A) (3.31 g, 27.5 mmol) was added dropwise at 20°C, and the resulting mixture was stirred at the same temperature for 0.5 hour. Then, the mixture was cooled to 10°C, water (50 mL) was added to the mixture, and the resulting mixture was filtered to give white solid. The resulting solid was dissolved in EA (150 mL), and the solution was washed with brine (50 mL), dried over anhydrous sodium sulfate, filtered, and concentrated to give N-[(4-bromophenyl)ethyl]-2,2-dimethylpropanamide as white solid (6.27 g, yield 88%).
$^1$H-NMR Spectrum (300MHz, $CDCl_3$):

δ (ppm): 7.43-7.47 (m, 2H), 7.14-7.19 (m, 2H), 5.75 (brs, 1H), 5.02-5.07 (m, 1H), 1.40-1.46 (m, 3H), 1.21 (s, 9H)

To a solution of N-[(4-bromophenyl)ethyl]-2,2-dimethylpropanamide (2.0 g, 7.0 mmol) in THF (10 mL), phenyllithium (2.0 M solution in butoxybutane, 10.5 mL, 21.0 mmol) was added dropwise at -5°C, and the resulting mixture was stirred at the same temperature for 4 hours under a nitrogen gas atmosphere. Paraformaldehyde (1.06 g) was added to the mixture, and the resulting mixture was stirred at 5°C for 1 hour. Then, water (20 mL) was added to the mixture, the resulting mixture was extracted with ethoxyethane (50 mL), the organic layer was washed with water (20 mL) and brine (20 mL), dried over anhydrous sodium sulfate, filtered, and concentrated. The residue was purified by silica gel column chromatography (eluted with ethyl acetate/petroleum ether (0:100 to 1:2)) to give N-{[4-bromo-2-(hydroxymethyl)phenyl]ethyl}-2,2-dimethylpropanamide as yellow solid (1.74 g, yield 79%).
$^1$H-NMR Spectrum (300MHz, $CDCl_3$):

δ (ppm): 7.40-7.48 (m, 2H), 7.10-7.12 (m, 1H), 6.10 (brs, 1H), 5.17-5.21 (m, 1H), 4.92-4.97 (m, 1H), 4.65-4.68 (m, 1H), 4.38-4.43 (m, 1H), 1.42-1.47 (m, 3H), 1.16 (s, 9H)

To a solution of N-{[4-bromo-2-(hydroxymethyl)phenyl]ethyl}-2,2-dimethylpropanamide (1.74 g, 5.54 mmol) and triethylamine (TEA, 672 mg, 6.60 mmol) in dichloromethane (20 mL), methanesulfonyl chloride (MsCl, 698 mg, 6.10 mmol) was added dropwise at 0°C, and the resulting mixture was stirred for 1 hour. Water (10 mL) was added to the mixture, the mixture was adjusted to pH 2.0 with 2 M HCl (aq), and extracted with dichloromethane (20 mL), and the organic layer was dried over anhydrous sodium sulfate, filtered, and concentrated to give {2-[(2,2-dimethylpropanoylamino)ethyl]-5-bromophenyl}methyl methylsulfonate as brown solid (2.02 g, yield 93%).
$^1$H-NMR Spectrum (300MHz, $CDCl_3$):

δ (ppm): 7.47-7.55 (m, 2H), 7.19-7.21 (m, 1H), 5.89 (brs, 1H), 5.57-5.61 (m, 1H), 5.23-5.30 (m, 1H), 5.11-5.16 (m, 1H), 3.04 (s, 3H), 1.46-1.50 (m, 3H), 1.18 (s, 9H)

To a solution of {2-[(2,2-dimethylpropanoylamino)ethyl]-5-bromophenyl}methyl methylsulfonate (2.02 g, 5.15 mmol) in diethylene glycol dimethyl ether (20 mL), sodium tert-butoxide (t-BuONa, 544 mg, 5.67 mmol) was added at 0°C, and the resulting mixture was stirred at 5°C for 2 hours. Water (20 mL) was added to the mixture, the resulting mixture was extracted with ethoxyethane (100 mL), and the organic layer was dried over anhydrous sodium sulfate, filtered, and concentrated to give 1-(5-bromo-1-methylisoindolin-2-yl)-2,2-dimethylpropan-1-one as yellow solid (1.52 g, yield 100%).
$^1$H-NMR Spectrum (300MHz, $CDCl_3$):

δ (ppm): 7.38-7.44 (m, 2H), 7.10-7.12 (m, 1H), 5.37-5.40 (m, 1H), 4.98-5.03 (m, 1H), 4.84-4.89 (m, 1H), 1.43-1.45 (m, 3H), 1.33 (s, 9H)

A mixture of 1-(5-bromo-1-methylisoindolin-2-yl)-2,2-dimethylpropan-1-one (1.52 g, 5.1 mmol) in 6 M HCl (aq, 12 mL) was heated to reflux for 4 hours. The reaction mixture was cooled to room temperature, adjusted to pH 10 with 5 M NaOH (aq), and extracted with dichloromethane (60 mL), and the organic layer was dried over anhydrous sodium sulfate, filtered, and concentrated to give 5-bromo-1-methylisoindoline as red oil (870 mg, yield 80%).
1H-NMR Spectrum (300MHz, $CDCl_3$):

δ (ppm): 7.33-7.36 (m, 2H), 7.03-7.06 (m, 1H), 4.37-4.39 (m, 1H), 4.10-4.24 (m, 2H), 1.99 (brs, 1H), 1.40-1.44 (m, 3H)

A mixture of 5-bromo-1-methylisoindoline (870 mg, 4.10 mmol), tert-butyl (tert-butoxycarbonyloxy)formate (1.34 g, 6.16 mmol), and TEA (829 mg, 8.20 mmol) in dichloromethane (10 mL) was stirred overnight at room temperature. The reaction mixture was concentrated, and the residue was purified by silica gel column chromatography (eluted with ethyl acetate/petroleum ether (0:100 to 1:10)) to give tert-butyl 5-bromo-1-methylisoindoline-2-carboxylate as colorless

oil (1.19 g, yield 93%).
$^1$H-NMR Spectrum (300MHz, $CDCl_3$):

δ (ppm): 7.35-7.41 (m, 2H), 7.02-7.09 (m, 1H), 4.95-5.04 (m, 1H), 4.54-4.75 (m, 2H), 1.52-1.59 (m, 12H)

[Formula 18]

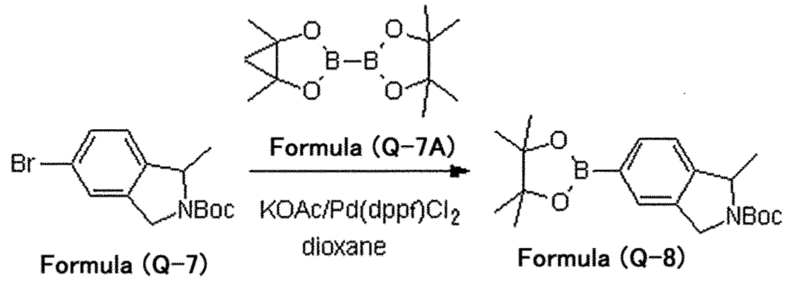


Formula (Q-7) Formula (Q-8)

A mixture of tert-butyl 5-bromo-1-methylisoindoline-2-carboxylate (250 mg, 0.8 mmol), the compound represented by the structural formula (Q-7A) (305 mg, 1.2 mmol), KOAc (235 mg, 2.4 mmol), and Pd(dppf)$Cl_2$ (65 mg, 0.08 mmol) in dioxane (10 mL) was stirred at 80°C for 1 hour under a nitrogen gas atmosphere. The reaction mixture was concentrated, the residue was dissolved in ethyl acetate (100 mL), and the solution was washed with water (50 mL) and brine (50 mL), dried over anhydrous sodium sulfate, filtered, and concentrated to give tert-butyl 5-bromo-1-methylisoindoline-2-carboxylate as red oil (506 mg, yield 100%).

[Formula 19]

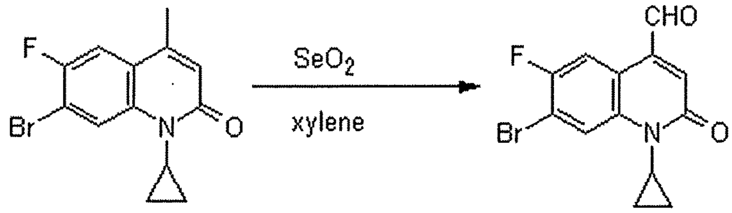


Formula (Q-9) Formula (Q-10)

A suspension of the compound represented by the structural formula (Q-9) (1.94 g, 6.57 mmol) and $SeO_2$ (4.37 g, 39.4 mmol) in xylene (40 mL) was heated to 150°C, and the resulting mixture was stirred at the same temperature for 36 hours. The mixture was cooled to room temperature, then filtered, and concentrated, water (100 mL) was added to the residue, and the resulting mixture was extracted with ethyl acetate (200 mL). The combined organic layer was washed with brine (100 mL), dried over anhydrous sodium sulfate, filtered, and concentrated. The residue was purified by silica gel column chromatography (eluted with ethyl acetate/petroleum ether (1:4) to dichloromethane/methanol (50:1)) to give the compound represented by the structural formula (Q-10) as yellow solid (1.97 g, yield 97%).
$^1$H-NMR Spectrum (300MHz, $CDCl_3$):

δ (ppm): 10.03 (s, 1H), 8.64 (d, J=9.3Hz, 1H), 8.12 (d, J=6.0Hz, 1H), 7.14 (s, 1H), 2.96-3.00 (m, 1H), 1.41-1.45 (m, 2H), 0.91-0.96 (m, 2H)

[Formula 20]

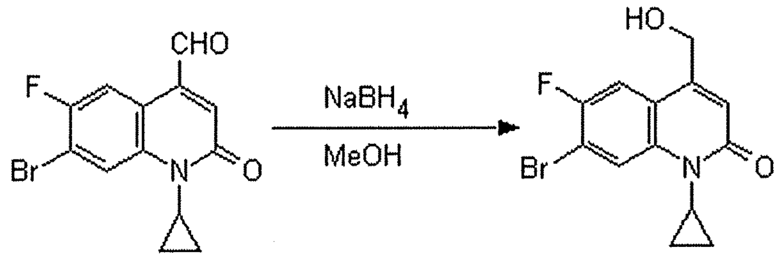


Formula (Q-10) Formula (Q-11)

To an ice-cooled solution of the compound represented by the structural formula (Q-10) (1.97 g, 6.35 mmol) in methanol (40 mL), sodium borohydride (483 mg, 12.71 mmol) was added portionwise. After the addition, the reaction mixture was stirred at room temperature for 30 minutes. Ammonium chloride (50 mL) was added to the mixture, and the resulting mixture was extracted with ethyl acetate (200 mL). The organic layer was washed with brine (100 mL), dried over anhydrous sodium sulfate, filtered, and concentrated to give the compound represented by the structural formula (Q-11) as white solid (1.94 g, yield 98%).
1H-NMR Spectrum (300MHz, $CDCl_3$):

δ (ppm): 8.06 (d, J=6.0Hz, 1H), 7.42 (d, J=8.7Hz, 1H), 6.80 (s, 1H), 4.82-4.84 (m, 2H), 2.87-2.92 (m, 1H), 2.41-2.45 (m, 1H), 1.35-1.42 (m, 2H), 0.86-0.92 (m, 2H)

[Formula 21]

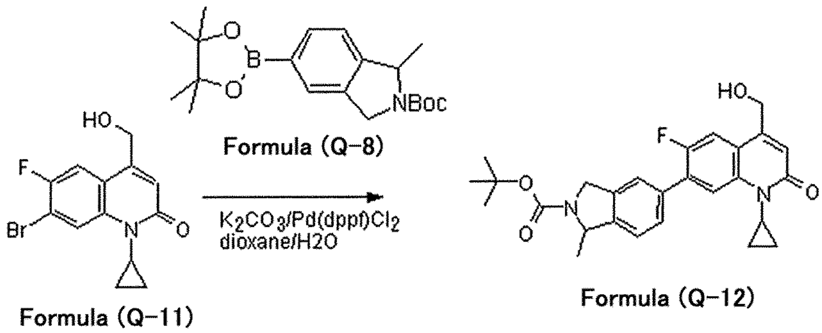


Formula (Q-11) Formula (Q-12)

A mixture of the compound represented by the structural formula (Q-11) (200 mg, 0.64 mmol), the compound represented by the structural formula (Q-8) (287 mg, 0.80 mmoL), Pd(dppf)$Cl_2$ (52 mg, 0.06 mmol), and potassium carbonate (132 mg, 0.96 mmol) in a solvent (dioxane/water = 10:1, 11 mL) was heated to 80°C under a nitrogen atmosphere, and stirred overnight. The mixture was cooled to room temperature, and diluted with ethyl acetate (50 mL), and a solution of $NH_4Cl$ (50 mL). The organic layer was separated, and the aqueous layer was extracted with ethyl acetate (50 mL). The combined organic layer was washed with water (50 mL) and brine (50 mL), and dried over magnesium sulfate, and the solvent was removed in vacuo. The residue was purified by TLC using ethyl acetate/petroleum ether (2:3) to give the compound represented by the structural formula (Q- 12) as yellow solid (183 mg, yield 62%).
$^1$H-NMR Spectrum (300MHz, $CDCl_3$):

δ (ppm): 7.90-7.92 (m, 1H), 7.43-7.47 (m, 3H), 7.26-7.28 (m, 1H), 6.81 (s, 1H), 5.10-5.14 (m, 1H), 4.67-4.89 (m, 4H), 2.90-2.97 (m, 1H), 1.50-1.54 (m, 12H), 1.37-1.40 (m, 3H), 1.26-1.27 (m, 2H)

[Formula 22]

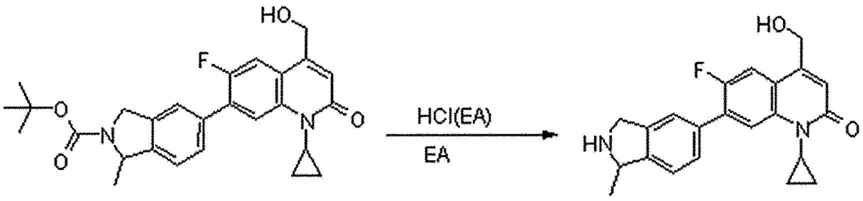


Formula (Q-12) Formula (17)

To a solution of the compound represented by the structural formula (Q-12) (183 mg, 0.39 mmol) in ethyl acetate (2 mL), 4 M HCl in ethyl acetate (2 mL, 8.0 mmol) was added at room temperature, and the resulting mixture was stirred for 1 hour. The mixture was filtered, then the solid was dissolved in methanol (5 mL), the solution was adjusted to pH 8 with NaOH, filtered, and concentrated to give yellow solid. This solid was purified by HPLC to give the compound represented by the structural formula (17), the compound J-144, 1-cyclopropyl-6-fluoro-4-(hydroxymethyl)-7-(1-methylisoindolin-5-yl)hydroquinolin-2-one, as brown solid (43 mg, yield 30%).
$^1$H-NMR Spectrum (300MHz,$CD_3OD$):

δ (ppm): 8.06 (d, J=6.9Hz, 1H), 7.52-7.61 (m, 3H), 7.37-7.40 (m, 1H), 6.79 (s, 1H), 4.85-4.87 (m, 2H), 4.49-4.51 (m, 1H), 4.16-4.30 (m, 2H), 3.04-3.09 (m, 1H), 1.50 (d, j=6.6Hz, 3H), 1.35-1.42 (m, 2H), 0.87-0.93 (m, 2H)
MS Calcd.: 364
MS Found: 365 ([M+1]$^+$)

Example 15: Synthesis of the compound represented by the structural formula (18) (compound J-131E)

[Formula 23]

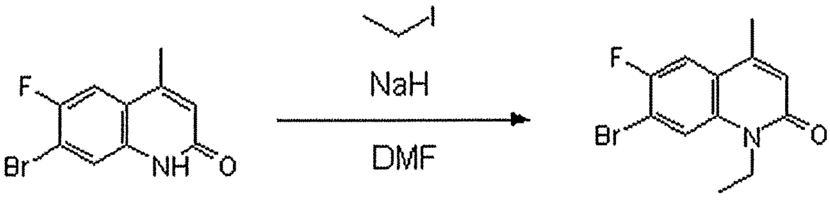


Formula (R-1) Formula (R-2)

To a suspension of the compound represented by the structural formula (R-1) (1.50 g, 5.88 mmol) in DMF (15 mL), NaH (443 mg, 17.6 mmol, 60%) was added at 0°C, and the resulting mixture was stirred at 40°C for 1 hour. The mixture was cooled to room temperature, then iodoethane (1.92 g, 11.8 mmol) was added to the mixture, and the resulting mixture was stirred at room temperature for 0.5 hour, and then at 60°C at overnight. The mixture was poured into water, and the resulting mixture was extracted with ethyl acetate (20 mL x 3). The combined organic layer was washed with brine, dried over $Na_2SO_4$, and concentrated in vacuo. The residue was purified by column chromatography (petroleum ether:ethyl acetate = 5:1) to give 7-bromo-1-ethyl-6-fluoro-4-methylhydroquinolin-2-one represented by the structural formula (R-2) as white solid (530 mg, yield 32%).
$^1$H-NMR Spectrum (300MHz, DMSO-$d_6$):

δ (ppm): 1.16 (t, J=7.2Hz, 3H), 2.39 (d, J=1.2Hz, 3H), 4.23 (q, J=7.2Hz, 2H), 6.59 (s, 1H), 7.74 (d, J=9.9Hz, 1H), 7.89 (d, J=5.7Hz, 1H)

A suspension of 7-bromo-1-ethyl-6-fluoro-4-methylhydroquinolin-2-one (530 mg, 1.87 mmol) and $SeO_2$ (1.25 g, 11.2 mmol) in xylene (15 mL) was stirred at 145°C for 3 days. The reaction mixture was cooled to room temperature, water (50 mL) was added to the mixture, and the resulting mixture was extracted with ethyl acetate (50 mL x 3). The combined organic layer was washed with brine, dried, and concentrated to give 7-bromo-1-ethyl-6-fluoro-2-oxohydroquinoline-4-carboaldehyde as brown solid (700 mg, yield 100%).

To an ice-cooled solution of 7-bromo-1-ethyl-6-fluoro-2-oxohydroquinoline-4-carboaldehyde (700 mg, 1.9 mmol) in methanol (20 mL), sodium borohydride (145 mg, 3.80 mmol) was added portionwise. After the addition, the reaction mixture was stirred at room temperature for 0.5 hours. The reaction mixture was filtered to give 7-bromo-1-ethyl-6-fluoro-4-(hydroxymethyl)hydroquinolin-2-one as white solid (310 mg, yield 56%).
$^1$H-NMR Spectrum (300MHz, DMSO-$d_6$):

δ (ppm): 1.17 (t, J=7.2Hz, 3H), 4.27 (q, J=7.2Hz, 2H), 4.70 (dd, J=5.7,1.2Hz, 2H), 5.56 (t, J=5.7Hz, 1H), 6.71 (s, 1H), 7.72 (d, J=9.6Hz, 1H), 7.93 (d, J=6.3Hz, 1H)

A mixture of 7-bromo-1-ethyl-6-fluoro-4-(hydroxymethyl)hydroquinolin-2-one (300 mg, 1.01 mmol), 4-hydroxyphenylboronic acid (180 mg, 1.31 mmol), Pd(dppf)$Cl_2$ (85 mg), and potassium carbonate (207 mg, 1.51 mmol) in dioxane/water (20 mL/2 mL) was stirred overnight at 80°C under a nitrogen gas atmosphere. The reaction mixture was filtered and concentrated, and the residue was purified by preparative HPLC to give the compound J-131E, 1-ethyl-6-fluoro-4-(hydroxymethyl)-7-(4-hydroxyphenyl)hydroquinolin-2-one, as white solid (140 mg, yield 40%).
$^1$H-NMR Spectrum (300MHz, DMSO-$d_6$):

δ (ppm): 1.21 (t, J=6.9Hz, 3H), 4.34 (q, J=6.9Hz, 2H), 4.73 (d, J=4.2Hz, 2H), 5.55 (t, J=5.7Hz, 1H), 6.67 (s, 1H), 6.91 (d, J=6.6Hz, 2H), 7.51 (d, J=6.6Hz, 2H), 7.63 (d, J=11.7Hz, 1H), 9.79 (s, 1H)
MS Calcd.: 313
MS Found: 314 ([M+1]$^+$)

Example 16: Synthesis of the compound represented by the structural formula (19) (compound J-121Cp)

[Formula 24]

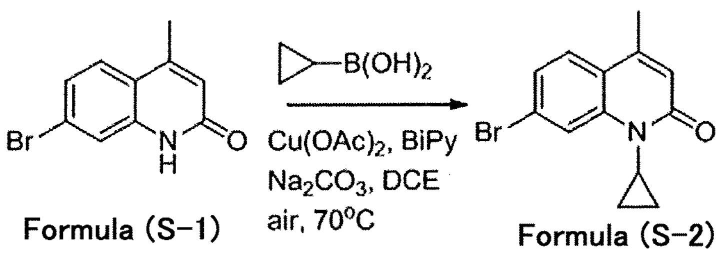


Formula (S-1) Formula (S-2)

A mixture of the compound represented by the structural formula (S-1) (2.40 g, 10.1 mmol), cyclopropylboronic acid (1.72 g, 23.9 mmol), 2,2'-bipyridine (1.56 g, 0.01 mol), sodium carbonate (2.12 g, 0.02 mol), anhydrous cuprous acetate (1.72 g, 0.01 mol), and 1,2-dichloroethane (50 mL) was stirred overnight at 70°C in the air. The mixture was cooled to room temperature, then a saturated ammonium chloride solution (100 mL) was added to the mixture, and the resulting mixture was filtered. The filtrate was extracted with dichloromethane (3 x 100 mL). The combined organic layer was concentrated, and the residue was purified by column chromatography (petroleum ether:ethyl acetate = 8:1 to dichloromethane:methanol = 1:0 to 200:1) to give 7-bromo-1-cyclopropyl-4-methyl-1H-quinolin-2-one as brown solid (950 mg, yield 34%).
$^1$H-NMR Spectrum (300MHz, $CDCl_3$):

δ (ppm): 0.86-0.93 (m, 2H), 1.34-1.41 (m, 2H), 2.4 (s, 3H), 2.88-2.92 (m, 1H), 6.50 (d, 1H, J=0.9Hz), 7.32-7.36 (m, 1H), 7.48-7.51 (m, 1H), 8.01-8.02 (m, 1H)

A suspension of 7-bromo-1-cyclopropyl-4-methyl-1H-quinolin-2-one (950 mg, 3.42 mmol) and $SeO_2$ (1.14 g, 10.2 mmol) in xylene (20 mL) was stirred at 150°C for 4 hours. Then, $SeO_2$ (1.14 g, 10.2 mmol) was added to the reaction mixture, and the resulting mixture was stirred overnight at 150°C. The reaction mixture was cooled to room temperature, water (50 mL) was added to the reaction mixture, and the resulting mixture was extracted with ethyl acetate (3 x 50 mL). The combined organic layer was washed with brine, dried, and concentrated to give 7-bromo-1-cyclopropyl-2-oxo-1,2-dihydroquinoline-4-carboaldehyde as brown solid (1.17 g, yield 100%).
$^1$H-NMR Spectrum (300MHz, $CDCl_3$):

δ (ppm): 0.89-0.95 (m, 2H), 1.41-1.48 (m, 2H), 2.95-2.99 (m, 1H), 7.08 (s, 1H), 7.41-7.44 (m, 1H), 8.07 (d, 1H, J=2.1Hz), 8.65 (d, 1H, J=8.7Hz), 10.06 (s, 1H)

To an ice-cooled solution of 7-bromo-1-cyclopropyl-2-oxo-1,2-dihydroquinoline-4-carboaldehyde (999 mg, 3.42

mmol) in methanol (20 mL), sodium borohydride (261 mg, 6.88 mmol) was added portionwise. After the addition, the reaction mixture was stirred at room temperature for 0.5 hours. The reaction mixture was filtered to give 7-bromo-1-cyclopropyl-4-hydroxymethyl-1H-quinolin-2-one as pink solid (700 mg, yield 69%).
$^1$H-NMR Spectrum (300MHz, DMSO-$d_6$):

δ (ppm): 0.71-0.77 (m, 2H), 1.24-1.32 (m, 2H), 2.93-2.98 (m, 1H), 4.71 (dd, 2H, J=1.2,4.2Hz), 5.50 (t, 1H, J=5.7Hz), 6.58 (s, 1H), 7.40-7.44 (m, 1H), 7.64 (d, 1H, J=8.4Hz), 8.02 (d, 1H, J=1.2Hz)

A mixture of 7-bromo-1-cyclopropyl-4-hydroxymethyl-1H-quinolin-2-one (400 mg, 1.36 mmol), 4-hydroxyphenylboronic acid (244 mg, 1.77 mmol), $PdCl_2$(dppf)(100 mg), and potassium carbonate (282 mg, 2.04 mmol) in dioxane/water (20 mL/2 mL) was stirred overnight at 80°C under a nitrogen gas atmosphere. The reaction mixture was filtered, and concentrated, and the residue was purified by column chromatography (dichloromethane:methanol = 50:1 to 20:1) to give the compound J-121Cp, 1-cyclopropyl-4-hydroxymethyl-7-(4-hydroxy-phenyl)-1H-quinolin-2-one, as white solid (300 mg, yield 72%).
$^1$H-NMR Spectrum (300MHz, DMSO-$d_6$):

δ (ppm): 0.75-0.80 (m, 2H), 1.29-1.36 (m, 2H), 2.99-3.04 (m, 1H), 4.74 (d, 2H, J=5.4Hz), 5.48 (t, 1H, J=5.7Hz), 6.53 (s, 1H), 6.90-6.94 (m, 2H), 7.45-7.49 (m, 1H), 7.61-7.63 (m, 2H), 7.70-7.72 (m, 1H), 8.02 (d, 1H, J=1.2Hz), 9.71 (s, 1H)
MS Calcd.: 307
MS Found: 308 ($[M+H]^+$)

Example 17: Synthesis of the compound represented by the structural formula (20) (compound J-132CP)

[Formula 25]

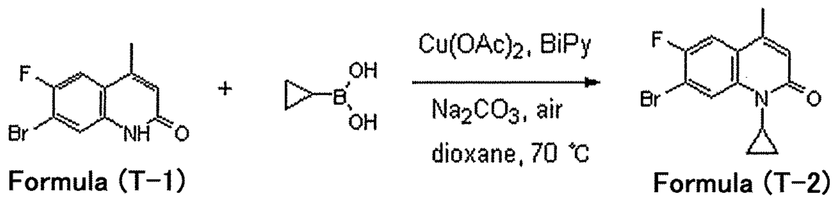


Formula (T-1) Formula (T-2)

A mixture of the compound represented by the structural formula (T-1) (1.0 g, 3.9 mmol), cyclopropylboronic acid (672 mg, 7.80 mmol), 2,2'-bipyridine (609 mg, 3.90 mmol), sodium carbonate (828 mg, 7.80 mmol), anhydrous cuprous acetate (709 mg, 3.90 mmol), and dioxane (24 mL) was heated to 70°C, and stirred at the same temperature for 4 hours in the air. The reaction mixture was cooled to room temperature, and then a saturated ammonium chloride solution (50 mL) was added to the mixture, and the resulting mixture was extracted with dichloromethane (2 x 50 mL). The combined organic layer was washed with water (2 x 50 mL) and brine, dried over anhydrous sodium sulfate, filtered, and concentrated give under reduced pressure. The residue was purified by silica gel column chromatography (eluted with ethyl acetate/petroleum ether (1:10 to 1:2)) to give 7-bromo-1-cyclopropyl-6-fluoro-4-methylhydroquinolin-2-one as yellow solid (687 mg, yield 60%).
$^1$H-NMR Spectrum (300MHz, DMSO-$d_6$):

δ (ppm): 0.68-0.73 (m, 2H), 1.22-1.29 (m, 2H), 2.48 (s, 3H), 2.89-2.94 (m, 1H), 6.48 (s, 1H), 7.70 (d, J=9.3Hz, 1H), 8.06 (d, J=6.0Hz, 1H)

A a suspension of 7-bromo-1-cyclopropyl-6-fluoro-4-methylhydroquinolin-2-one(750 mg, 2.80 mmol) and $SeO_2$ (1.50 g, 13.9 mmol) in xylene (10 mL) was heated to 150°C, and the mixture was stirred at the same temperature for 36 hours. The mixture was cooled to room temperature, and then water (50 mL) was added to the mixture, the resulting mixture was extracted with ethyl acetate (2 x 50 mL). The combined organic layer was washed with brine (100 mL), dried over anhydrous sodium sulfate, filtered, and concentrated under reduced pressure. The residue was ground in pure petroleum ether to give a crude product of 7-bromo-1-cyclopropyl-6-fluoro-2-oxohydroquinoline-4-carboaldehyde as yellow solid (760 mg).
To an ice-cooled solution of 7-bromo-1-cyclopropyl-6-fluoro-2-oxohydroquinoline-4-carboaldehyde (760 mg, 2.80 mmol) in methanol (40 mL), sodium borohydride (158 mg, 4.20 mmol) was added portionwise. After the addition, the reaction mixture was stirred at room temperature for 30 minutes. Ammonium chloride (50 mL) was added to the

mixture, and the resulting mixture was extracted with ethyl acetate (2 x 50 mL). The organic layer was washed with brine, dried over anhydrous sodium sulfate, filtered, and concentrated to give 7-bromo-1-cyclopropyl-6-fluoro-4-(hydroxymethyl)hydroquinolin-2-one as off white solid (500 mg, yield 63%), which was used for the next step without purification.
$^1$H-NMR Spectrum (300MHz, DMSO-$d_6$):

δ (ppm): 0.69-0.75 (m, 2H), 1.23-1.29 (m, 2H), 2.90-2.97 (m, 1H), 4.65 (d, J=4.8Hz, 2H), 5.52 (t, J=5.4Hz, 1H), 6.60 (s, 1H), 7.66 (d, J=9.9Hz, 1H), 8.08 (d, J=6.3Hz, 1H)

A mixture of 7-bromo-1-cyclopropyl-6-fluoro-4-(hydroxymethyl)hydroquinolin-2-one (100 mg, 0.32 mmol), 4-hydroxyphenylboronic acid (67 mg, 0.38mmoL), Pd(dppf)$Cl_2$ (26 mg, 0.032 mmol), and potassium carbonate (88 mg, 0.64 mmol) in a solvent (dioxane:water = 10:1, (10 mL)) was heated to 80°C for 16 hours under a nitrogen atmosphere. The mixture was cooled to room temperature, and diluted with ethyl acetate (50 mL), and a solution of $NH_4Cl$ (50 mL). The organic layer was separated, and the aqueous layer was extracted with ethyl acetate (50 mL). The combined organic layer was washed with water (50 mL) and brine (50 mL), and dried over magnesium sulfate, and the solvent was removed in vacuo. The resulting dark color oil was filtered through a short pad of silica gel, and the filtrate was concentrated to give the compound J-132CP, 7-(4-aminophenyl)-1-cyclopropyl-6-fluoro-4-(hydroxymethyl)hydroquinolin-2-one, as yellow solid (42 mg, yield 40%).
$^1$H-NMR Spectrum (300MHz, $CD_3OD$):

δ (ppm): 0.88-0.90 (m, 2H), 1.35-1.40 (m, 2H), 3.03-3.05 (m, 1H), 4.83 (s, 2H), 6.74 (s, 1H), 6.79-6.82 (m, 2H), 7.40-7.43 (m, 2H), 7.49-7.53 (m, 1H), 7.99-8.01 (m, 1H)
MS Calcd.: 324
MS Found: 325 ($[M+H]^+$)

Example 18: Synthesis of the compound represented by the structural formula (10) (compound J-135)

A mixture of 7-bromo-1-cyclopropyl-6-fluoro-4-(hydroxymethyl)hydroquinolin-2-one (100 mg, 0.320 mmol), 4-nitrophenylboronic acid (67 mg, 0.40 mmol), Pd(dppf)$Cl_2$ (26 mg, 0.032 mmol), and potassium carbonate (88 mg, 0.64 mmol) in a solvent (dioxane:water = 10:1, 10 mL) was heated to 80°C for 16 hours under a nitrogen atmosphere. The mixture was cooled to room temperature, and diluted with ethyl acetate (50 mL), and a solution of $NH_4Cl$ (50 mL). The organic layer was separated, and the aqueous layer was extracted with ethyl acetate (50 mL). The combined organic layer was washed with water (50 mL) and brine (50 mL), and dried over magnesium sulfate, and the solvent was removed to give a crude product of 1-cyclopropyl-6-fluoro-4-(hydroxymethyl)-7-(4-nitrophenyl)hydroquinolin-2-one as black solid (110 mg, yield 97%).

To a mixture of 1-cyclopropyl-6-fluoro-4-(hydroxymethyl)-7-(4-nitrophenyl)hydroquinolin-2-one (50 mg, 0.14 mmol) and propanedioic acid (15 mg, 0.14 mol) in DMF (5 mL), DCC (35 mg, 0.17 mmol) was added, and then the resulting mixture was stirred for 1 hour. The solvent was removed from the mixture, and the residue was purified by preparative TLC (dichloromethane:methanol = 10:1) to give 2-({[1-cyclopropyl-6-fluoro-7-(4-nitrophenyl)-2-oxo-4-hydroquinolyl]methyl}oxycarbonyl)acetic acid as blue solid (45 mg, yield 73%).
$^1$H-NMR Spectrum (300MHz, DMSO-$d_6$):

δ (ppm): 0.80-0.82 (m, 2H), 1.28-1.31 (m, 2H), 3.04-3.08 (m, 1H), 3.11 (s, 2H), 5.31 (s, 2H), 6.85 (s, 1H), 7.78-7.82 (m, 1H), 7.95-8.03 (m, 3H), 8.38-8.41 (m, 2H)

A mixture of 2-({[1-cyclopropyl-6-fluoro-7-(4-nitrophenyl)-2-oxo-4-hydroquinolyl]methyl}oxycarbonyl)acetic acid (110 mg, 0.250 mmol) and Raney-Nickel catalyst (Raney-Ni, 100 mg) in DMF (10 mL) was stirred at room temperature for 2 hours under a $H_2$ atmosphere. The mixture was filtered, the solvent was removed, and the residue was purified by preparative HPLC to give the compound J-135, 2-({[7-(4-aminophenyl)-1-cyclopropyl-6-fluoro-2-oxo-4-hydroquinolyl]methyl}oxycarbonyl)acetic acid as yellow solid (22 mg, yield 21%).
$^1$H-NMR Spectrum (300MHz, DMSO-$d_6$):

δ (ppm): 0.79 (s, 2H), 1.24-1.31 (m, 2H), 3.01-3.03 (m, 1H), 3.56 (s, 2H), 5.39 (s, 2H), 6.59 (s, 1H), 6.69-6.71 (m, 2H), 7.36-7.38 (m, 2H), 7.55-7.59 (m, 1H), 7.86-7.88 (m, 1H)
MS Calcd.: 410
MS Found: 411 ($[M+H]^+$)

Example 19: Synthesis of the compound represented by the structural formula (6) (compound J-136)

To a mixture of 1-cyclopropyl-6-fluoro-4-(hydroxymethyl)-7-(4-nitrophenyl)hydroquinolin-2-one (354 mg, 1.00 mmol) and butanedioic acid (472 mg, 4.00 mol) in DMF (20 mL), N,N'-dicyclohexylcarbodiimide (824 mg, 4.00 mmol) was added, and then the resulting mixture was stirred for 1 hour. The solvent was removed from the mixture, and the residue was purified by preparative TLC (dichloromethane:methanol = 10:1) to give 3-({[1-cyclopropyl-6-fluoro-7-(4-nitrophenyl)-2-oxo-4-hydroquinolyl]methyl}oxycarbonyl)propanoic acid as yellow solid (110 mg, yield 24%).
$^1$H-NMR Spectrum (300MHz, DMSO-$d_6$):

δ (ppm): 0.79-0.84 (m, 2H), 1.27-1.31 (m, 2H), 2.52-2.56 (m, 2H), 2.65-2.70 (m, 2H), 3.03-3.05 (m, 1H), 5.38 (s, 2H), 6.65 (s, 1H), 7.75-7.79 (m, 1H), 7.95-8.05 (m, 3H), 8.39-8.42 (m, 2H)

A mixture of 3-({[1-cyclopropyl-6-fluoro-7-(4-nitrophenyl)-2-oxo-4-hydroquinolyl]methyl}oxycarbonyl)propanoic acid (100 mg, 0.220 mmol) and Raney-Ni (100 mg) in DMF (10 mL) was stirred at room temperature for 2 hours under a $H_2$ atmosphere. The mixture was filtered, the solvent was removed, and the residue was purified by preparative HPLC to give the compound J-136, 3-({[7-(4-aminophenyl)-1-cyclopropyl-6-fluoro-2-oxo-4-hydroquinolyl] methyl}oxycarbonyl)propanoic acid, as yellow solid (21 mg, yield 23%).
$^1$H-NMR Spectrum (300MHz, $CD_3OD$):

δ (ppm): 0.88-0.94 (m, 2H), 1.35-1.42 (m, 2H), 2.63-2.67 (m, 2H), 2.70-2.76 (m, 2H), 3.04-3.09 (m, 1H), 5.38-5.40 (m, 2H), 6.71 (s, 1H), 7.26-7.30 (m, 2H), 7.58-7.63 (m, 1H), 7.68-7.72 (m, 2H), 8.05-8.08 (m, 1H)
MS Calcd.: 424
MS Found: 425 ($[M+H]^+$)

Example 20: Synthesis of the compound represented by the structural formula (16) (compound J-138)

To a solution of 1-cyclopropyl-6-fluoro-4-(hydroxymethyl)-7-(4-nitrophenyl)hydroquinolin-2-one (100 mg, 0.283 mmol) and TEA (86 mg, 0.85 mmol) in DMF (5 mL), propanoyl chloride (52 mg, 0.56 mmol) was added at room temperature, and then the resulting mixture was stirred for 3 hours. The solvent was removed from the mixture to give a crude product of [1-cyclopropyl-6-fluoro-7-(4-nitrophenyl)-2-oxo-4-hydroquinolyl] methyl propanoate as black oil.

A mixture of [1-cyclopropyl-6-fluoro-7-(4-nitrophenyl)-2-oxo-4-hydroquinolyl]methyl propanoate (100 mg, 0.244 mmol) and Raney-Ni (100 mg) in DMF (10 mL) was stirred at room temperature for 2 hours under a $H_2$ atmosphere. The mixture was filtered, the solvent was removed, and the residue was purified by preparative HPLC to give the compound J-138, [7-(4-aminoplienyl)-1-cyclopropyl-6-fluoro-2-oxo-4-hydroquinolyl]methyl propanoate, as yellow solid (40 mg, yield 37%).
$^1$H-NMR Spectrum (300MHz, $CD_3OD$):

δ (ppm): 0.95-1.01 (m, 2H), 1.19-1.26 (m, 3H), 1.39-1.46 (m, 2H), 2.46-2.53 (m, 2H), 3.01-3.06 (m, 1H), 5.30-5.31 (m, 2H), 6.81-6.85 (m, 3H), 7.34-7.37 (m, 1H), 7.45-7.49 (m, 2H), 7.96-7.98 (m, 1H)
MS Calcd.: 380
MS Found: 381 ($[M+H]^+$)

Example 21: Synthesis of the compound represented by the structural formula (22) (compound J-139)

A mixture of 7-bromo-1-cyclopropyl-6-fluoro-4-(hydroxymethyl)hydroquinolin-2-one (150 mg, 0.500 mmol), $NaN_3$ (39 mg, 0.60mmoL), and triphenylphosphine ($PPh_3$, 276 mg, 1.05 mmol) in a solvent ($CCl_4$:DMF = 1:4, 5 mL) was stirred at 90°C for 1 hour. The mixture was cooled to room temperature, water (2.5 mL) was added to the mixture, and the resulting mixture was stirred at room temperature for 2 hours. The resulting mixture was extracted with dichloromethane (50 mL), the organic layer was dried over $Na_2SO_4$, and the solvent was removed to give a crude product of 4-(aminomethyl)-7-bromo-1-cyclopropyl-6-fluorohydroquinolin-2-one as yellow solid (326 mg, yield 100%).

A mixture of 4-(aminomethyl)-7-bromo-1-cyclopropyl-6-fluorohydroquinolin-2-one (156 mg, 0.501 mmol), 4-hydroxyphenylboronic acid (83 mg, 0.60mmoL), Pd(dppf)$Cl_2$ (41 mg, 0.050 mmol), and potassium carbonate (104 mg, 0.750 mmol) in a solvent (dioxane:water = 10:1, 5.5 mL) was heated at 80°C for 16 hours under a nitrogen atmosphere. The mixture was cooled to room temperature, and then ethyl acetate (50 mL) was added to the mixture, the resulting mixture was washed with water (50 mL) and brine (50 mL), and dried over magnesium sulfate, and the solvent was removed to give a crude product. This product was purified by preparative HPLC to give the compound J-139, 4-(aminomethyl)-1-cyclopropyl-6-fluoro-7-(4-hydroxyphenyl)hydroquinolin-2-one, as white solid (28 mg, yield 17%).
$^1$H-NMR Spectrum (300MHz, DMSO-$d_6$):

δ (ppm): 0.76-0.77 (m, 2H), 1.31-1.33 (m, 2H), 3.06-3.09 (m, 1H), 4.35 (s, 2H), 6.64 (s, 1H), 6.92-6.95 (m, 2H), 7.50-7.53 (m, 2H), 7.74-7.78 (m, 1H), 7.91-7.94 (m, 1H), 8.36-8.38 (m, 2H), 9.85 (s, 1H)
MS Calcd.: 324
MS Found: 325 ($[M+H]^+$)

Example 22: Synthesis of the compound represented by the structural formula (21) (compound J-149)

A solution of 2-bromo-1,3-difluorobenzene (25.0 g, 130 mmol) in concentrated $H_2SO_4$ (52 mL, 98%) was vigorously stirred at room temperature. Then, concentrated nitric acid (9 mL, 70%) was added dropwise to the solution, and the internal temperature was maintained to be lower than 55°C. After the addition, the mixture was further stirred for 30 minutes, and poured on ice. $CH_2Cl_2$ (200 mL x 2) was added to the mixture for extraction of the desired compound. The combined organic layer was washed with saturated $Na_2CO_3$ and brine, dried over $Na_2SO_4$, and concentrated to give 2-bromo-1,3-difluoro-4-nitrobenzene as yellow solid (23.0 g, yield 74%).
$^1$H-NMR Spectrum (300MHz, $CDCl_3$):

δ (ppm): 7.10-7.16 (m, 1H), 8.09-8.16 (m, 1H)

A solution of 2-bromo-1,3-difluoro-4-nitrobenzene (10.5 g, 44.1 mmol) in DMSO (80 mL) was stirred at room temperature. Then, a solution of KOH (12.4 g, 221 mmol) in water (22 mL) was added dropwise over 15 minutes. After the addition, the mixture was stirred overnight at 30°C. Completion of the reaction was determined by TLC. The mixture was acidified with 2 N HCl to pH 4, and ethyl acetate (100 mL x 2) was added to the mixture for extraction of the desired compound. The combined organic layer was washed with water and brine, dried over $Na_2SO_4$, and concentrated to give 2-bromo-3-fluoro-6-nitrophenol as yellow solid (10.5 g, yield >100%).
$^1$H-NMR Spectrum (300MHz, $CDCl_3$):

δ (ppm): 6.82-6.86 (m, 1H), 8.15-8.22 (m, 1H), 11.41 (brs, 1H)

To a mixture of 2-bromo-3-fluoro-6-nitrophenol (5.00 g, 21.2 mmol), $K_2CO_3$ (8.78 g, 63.6 mmol), and NaI (1.59 g, 10.6 mmol) in acetone (150 mL), chloroacetone (3.92 g, 42.4 mmol) was added with stirring. Then, the resulting mixture was heated overnight to reflux, and stirred. The mixture was concentrated in vacuo, and ethyl acetate was added to the mixture for extraction of the desired compound. The organic layer was washed with water and brine, dried over $Na_2SO_4$, and concentrated. The residue was purified by column chromatography (petroleum ether:ethyl acetate = 20:1) to give 1-(2-bromo-3-fluoro-6-nitrophenoxy)acetone as yellow solid (5.3 g, yield 85%).
$^1$H-NMR Spectrum (300MHz, $CDCl_3$):

δ (ppm): 2.38 (s, 3H), 4.73 (s, 2H), 7.11 (dd, J=9.3,7.2Hz, 1H), 7.95 (dd, J=9.3,7.2Hz, 1H)

[Formula 26]

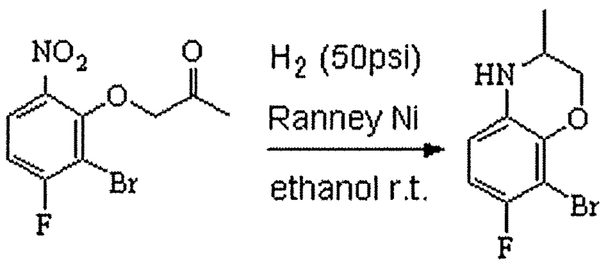


Formula (U-4) Formula (U-5)

A mixture of the compound represented by the structural formula (U-4) (5.30 g, 18.1 mmol) and Raney-Ni(1 g) in ethanol (30 mL) was stirred at room temperature for 4 hours under a $H_2$ atmosphere (50 psi). The mixture was filtered, and the filtrate was concentrated. The residue was purified by column chromatography (petroleum ether:ethyl acetate = 8:1) to give the compound represented by the structural formula (U-5) as brown solid (3.9 g, yield 87%).
$^1$H-NMR Spectrum (300MHz, $CDCl_3$):

δ (ppm): 1.15 (d, J=8.1Hz, 3H), 3.48-3.52 (m, 1H), 3.79-3.86 (m, 1H), 4.30-4.34 (m, 1H), 6.45-6.50 (m, 1H), 6.58 (d, J=8.4Hz, 3H)

[Formula 27]

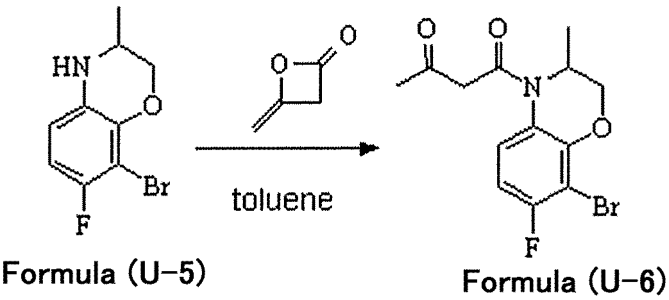


Formula (U-5) Formula (U-6)

A mixture of the compound represented by the structural formula (U-5) (2.5 g, 10.2 mmol) and diketene (1.02 g, 12.2 mmol) in toluene (50 mL) was refluxed overnight. The mixture was concentrated, and the residue was purified by column chromatography (petroleum ether:ethyl acetate = 15:1) to give the compound represented by the structural formula (U-6) as yellow solid (2.2 g, yield 65%).
$^1$H-NMR Spectrum (300MHz, $CDCl_3$):

δ (ppm): 1.05 (d, J=5.7Hz, 1H), 1.97 (s, 1.2H), 2.21 (s, 1.8H), 3.74 (s, 2H), 4.15-4.38 (m, 2H), 4.88-5.37 (m, 1H), 6.70-6.77 (m, 1H), 6.92-7.36 (m, 1H)

[Formula 28]

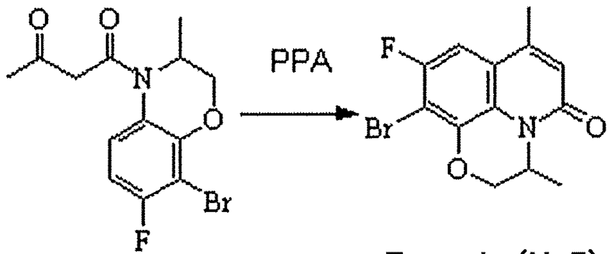


Formula (U-7)

Formula (U-6)

PPA (15 g) in toluene (20 mL) was heated to 80°C with stirring. Then, the compound represented by the structural formula (U-6) (2.20 g, 6.70 mmol) was added to the mixture, and the resulting mixture was stirred at 115°C for 3 hours. After cooling, the mixture was poured on ice. Ethyl acetate (100 mL x 2) was added to the mixture for extraction of the desired compound. The combined organic layer was washed with water and brine, dried over $Na_2SO_4$, and concentrated. The residue was recryctallized from petroleum ether and ethyl acetate (ratio = 2:3) to give the compound represented by the structural formula (U-7) as pale yellow solid (0.82 g, yield 41%).
$^1$H-NMR Spectrum (300MHz, $CDCl_3$):

δ (ppm): 1.39 (d, J=6.6Hz, 3H), 2.42 (s, 3H), 4.14-4.19 (m, 1H), 4.53 (dd, J=11.4,1.2Hz, 1H), 5.10-5.13 (m, 1H), 6.62 (s, 1H), 7.08 (d, J=9.0Hz, 1H)

[Formula 29]

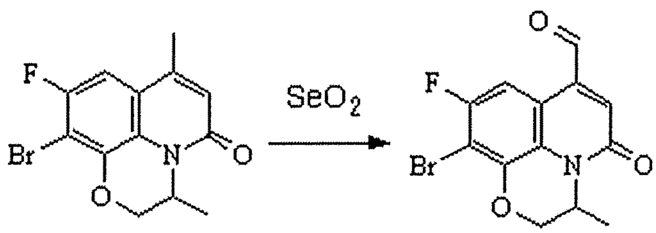


Formula (U-7) Formula (U-8)

A suspension of the compound represented by the structural formula (U-7) (200 mg, 0.643 mmol) and $SeO_2$ (143 mg, 1.29 mmol) in xylene (20 mL) was stirred overnight at 150°C. Then, the mixture was cooled to room temperature, water (50 mL) was added to the mixture, and the resulting mixture was extracted with ethyl acetate (3 x 50 mL). The combined organic layer was washed with brine, dried over $Na_2SO_4$, and concentrated to give the compound represented by the structural formula (U-8) (204 mg, yield 98%), which was used for the following step without purification.

[Formula 30]

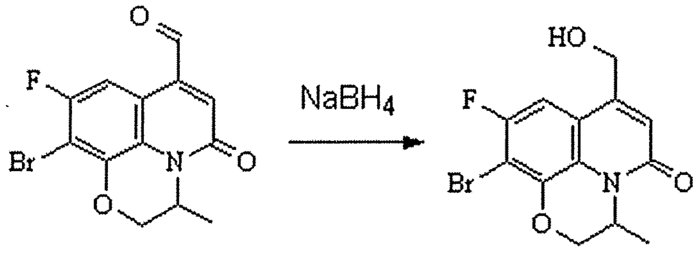


Formula (U-8)    Formula (U-9)

To an ice-cooled solution of the compound represented by the structural formula (U-8) (204 mg, 0.630 mmol) in methanol (20 mL), sodium borohydride (49 mg, 1.3 mmol) was added portionwise. After the addition, the reaction mixture was stirred at room temperature for 1 hour. The solid was collected by filtration to give the compound represented by the structural formula (U-9) as pink solid.
$^1$H-NMR Spectrum (300MHz, DMSO-$d_6$):

δ (ppm): 1.21 (d, J=6.9Hz, 3H), 4.20 (dd, J=11.1,2.4Hz, 1H), 4.59 (d, J=11.1Hz, 1H), 4.69 (d, J=5.4Hz, 2H), 4.94 (dd, J=6.6, 1.2Hz, 1H), 5.58 (t, J=5.7Hz, 1H), 6.73 (s, 1H), 7.36 (d, J=9.9Hz, 1H)

[Formula 31]

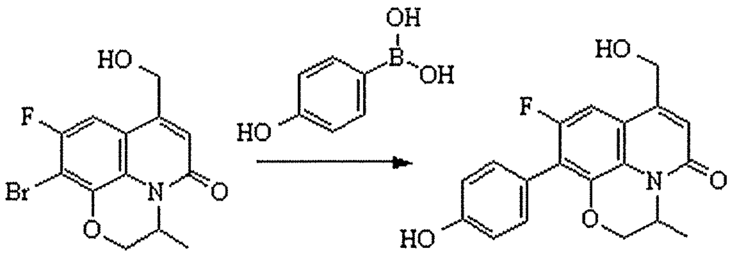

Formula (U-9)    Formula (21)

A mixture of the compound represented by the structural formula (U-9) (110 mg, 0.340 mmol), 4-hydroxyphenylboronic acid (61.0 mg, 0.440 mmol), $PdCl_2$(dppf) (50 mg), and potassium carbonate (94 mg, 0.68 mmol) in dioxane (20 mL) and water (2 mL) was stirred at overnight at 80°C under a nitrogen gas atmosphere. The mixture was filtered, and concentrated. The residue was purified by preparative HPLC to give the compound represented by the structural formula (21) J-149, 9-fluoro-7-(hydroxymethyl)-10-(4-hydroxyphenyl)-3-methyl-2H-[1,4]oxazino[2,3,4-ij]quinolin-5(3H)-one as white solid (72 mg, yield 62%).
$^1$H-NMR Spectrum (300MHz, DMSO-$d_6$):

δ (ppm): 1.23 (d, J=6.6Hz, 3H), 4.20 (d, J=10.2Hz, 1H), 4.37 (d, J=11.4Hz, 1H), 4.71 (d, J=4.2Hz, 2H), 4.89 (dd, J=6.0,2.4Hz, 1H), 5.52-5.56 (m, 1H), 6.69 (s, 1H), 6.84 (d, J=11.1Hz, 2H), 7.23-7.27 (m, 3H), 9.62 (s, 1H)
MS Calcd.: 341
MS Found: 342 ([M+1]$^+$)

Example 23: Synthesis of the compound represented by the structural formula (23) (compound J-150)

To a suspension of 3-bromo-4-fluoroaniline (2.00 g, 10.5 mmol) in toluene (15 mL), methyl 3-oxopentanoate (2.74 g, 21.1 mmol) was added, and the resulting mixture was stirred overnight at 110°C. The mixture was cooled to

room temperature, and then concentrated in vacuo. The residue was purified by column chromatography (petroleum ether:ethyl acetate = 4:1) to give N-(3-bromo-4-fluorophenyl)-3-oxopentanamide as white solid (1.6 g, yield 54%).
$^1$H-NMR Spectrum (300MHz, $CDCl_3$):

δ (ppm): 1.09 (t, J=7.2Hz, 3H), 2.60 (d, J=7.2Hz, 2H), 3.56 (s, 2H), 7.03 (t, J=8.4Hz, 1H), 7.39-7.44 (m, 1H), 7.85-7.88 (m, 1H), 9.32 (brs, 1H)

A solution of N-(3-bromo-4-fluorophenyl)-3-oxopentanamide (1.5 g, 5.2 mmol) in concentrated sulfuric acid (15 mL) was heated at 95°C for 2 hours. Then, the mixture was cooled to room temperature, and poured on ice. The solid collected by filtration, and washed with water to give 7-bromo-4-ethyl-6-fluorohydroquinolin-2-one as white solid (720 mg, yield 48%).
$^1$H-NMR Spectrum (300MHz, $CDCl_3$):

δ (ppm): 1.16 (t, J=7.5Hz, 3H), 2.74 (q, J=12.0Hz, 2H), 6.41 (s, 1H), 7.55 (d, J=5.4Hz, 1H), 7.72 (d, J=9.9Hz, 1H), 11.67 (brs, 1H)

A mixture of 7-bromo-4-ethyl-6-fluorohydroquinolin-2-one (720 mg, 2.67 mmol), cyclopropylboronic acid (459 mg, 5.34 mmol), 2,2'-bipyridine (416 mg, 2.67 mmol), sodium carbonate (566 mg, 5.34 mmol), anhydrous cuprous acetate (485 mg, 2.67 mmol), and 1,2-dichloroethane (15 mL) was stirred overnight at 70°C. The mixture was cooled to room temperature, then saturated ammonium chloride solution (100 mL) was added to the mixture, and the resulting mixture was filtered. The filtrate was extracted with dichloromethane (100 mL x 3). The combined organic layer was concentrated, and the residue was purified by chromatography (petroleum ether:ethyl acetate = 8:1 to 1:1) to give 7-bromo-1-cyclopropyl-4-ethyl-6-fluorohydroquinolin-2-one as yellow solid (260 mg, yield 31%).

A mixture of 7-bromo-1-cyclopropyl-4-ethyl-6-fluorohydroquinolin-2-one (260 mg, 0.840 mmol), 4-hydroxyphenylboronic acid (150 mg, 1.09 mmol), $PdCl_2$(dppf) (70 mg), and potassium carbonate (174 mg, 1.26 mmol) in dioxane/water (15 mL/1.5 mL) was stirred overnight at 80°C under a nitrogen gas atmosphere. The mixture was filtered and concentrated, and then the residue was purified by preparative HPLC to give the compound J-150, 1-cyclopropyl-4-ethyl-6-fluoro-7-(4-hydroxyphenyl)hydroquinolin-2-one, as white solid (35 mg, yield 13%). $^1$H-NMR Spectrum (300MHz, DMSO-$d_6$):

δ (ppm): 0.72 (m, 2H), 1.18-1.28 (m,5H), 2.74 (q. J=12.0Hz, 2H), 2.93-2.94 (m, 1H), 6.39 (s, 1H), 6.88 (d, J=8.4Hz, 1H), 7.46 (d, J=7.2Hz, 2H), 7.62 (d, J=12.0Hz, 1H), 7.84 (d, J=7.5Hz, 1H), 9.76 (brs, 1H)
MS Calcd.: 323
MS Found: 324 ($[M+1]^+$)

To a solution of 3-bromo-4-methylaniline (5.00 g, 26.9 mmol) in xylene (50 mL), methyl acetoacetate (6.20 g, 53.8 mmol) and pyridine (4.30 g, 53.8 mmol) were added. The mixture was heated to 135°C overnight. Consumption of the compound represented by the structural formula (X-1) was shown by LCMS. Then, the mixture was cooled to room temperature, and diluted with water, aqueous HCl was added to the mixture, and the resulting mixture was extracted with ethyl acetate (3 x 100 mL). The organic layer was dried over anhydrous $Na_2SO_4$, and concentrated, and the residue was purified by flash silica gel chromatography (petroleum ether:ethyl acetate = 20:1 to 5:1) to give N-(3-bromo-4-methylphenyl)-3-oxobutanamide as white solid (4.10 g, yield 56%).
$^1$H-NMR Spectrum (300MHz, $CDCl_3$):

δ (ppm): 2.33 (s, 3H), 2.35 (s, 3H), 3.58 (s, 2H), 7.16 (d, J=8.4Hz, 1H), 7.36 (dd, J=8.4, 2.1Hz, 1H), 7.81 (d, J=2.1Hz, 1H), 9.14 (br, 1H)

A solution of N-(3-bromo-4-methylphenyl)-3-oxobutanamide (4.10 g, 15.2 mmol) in concentrated sulfuric acid (50 mL) was heated to 95°C for 2 hours. Then, the mixture was cooled to room temperature, and poured on ice. The solid was collected by filtration, and washed with water to give 7-bromo-4,6-dimethylhydroquinolin-2-one as yellow solid (3.60 g, yield 95%).
$^1$H-NMR Spectrum (300MHz, DMSO-$d_6$):

δ (ppm): 2.33 (s,6H), 6.38 (s, 1H), 7.50 (s, 1H), 7.66 (s, 1H), 11.54 (brs, 1H)

A mixture of 7-bromo-4,6-dimethylhydroquinolin-2-one (2.00 g, 7.94 mmol), cyclopropylboronic acid (1.30 g, 15.4 mmol), 2,2'-bipyridine (1.30 g, 7.94 mmol), sodium carbonate (1.60 g, 15.38 mmol), anhydrous cuprous acetate (1.60 g, 7.94 mmol), and 1,2-dichloroethane (100 mL) was stirred overnight at 70°C under an air atmosphere. The

mixture was cooled to room temperature, then saturated ammonium chloride solution (100 mL) was added to the mixture, the resulting mixture was filtered, and the filtrate was extracted with dichloromethane (100 mL x 3). The combined organic layer was concentrated, and the residue was purified by chromatography (petroleum ether:ethyl acetate = 8:1 to dichloromethane:methanol = 1:0 to 200:1) to give 7-bromo-1-cyclopropyl-4,6-dimethylhydroquinolin-2-one as yellow solid (179 mg, yield 8%).
$^1$H-NMR Spectrum (300MHz, DMSO-$d_6$):

$\delta$ (ppm): 0.67-0.73 (m, 2H), 1.21-1.27 (m, 2H), 2.35 (s, 3H), 2.40 (s, 3H), 2.87-2.92 (m, 1H), 6.40 (s, 1H), 7.68 (s, 1H), 7.80 (s, 1H)

A suspension of 7-bromo-1-cyclopropyl-4,6-dimethylhydroquinolin-2-one (175 mg, 0.600 mmol) and $SeO_2$ (133 mg, 1.20 mmol) in xylene (20 mL) was stirred overnight at 150°C. The reaction mixture was cooled to room temperature, water (50 mL) was added to the mixture, and the resulting mixture was extracted with ethyl acetate (50 mL x 3). The combined organic layer was washed with brine, dried, and concentrated to give 7-bromo-1-cyclopropyl-6-methyl-2-oxohydroquinoline-4-carboaldehyde, and this product was used for the following step as it was.

To an ice-cooled solution of 7-bromo-1-cyclopropyl-6-methyl-2-oxohydroquinoline-4-carboaldehyde (180 mg, 0.590 mmol) in methanol (20 mL), sodium borohydride (45.0 mg, 1.18 mmol) was added portionwise. After the addition, the reaction mixture was stirred at room temperature for 0.5 hours. The solid was collected by filtration to give 7-bromo-1-cyclopropyl-4-(hydroxymethyl)-6-methylhydroquinolin-2-one as pink solid (125 mg, yield 69%).
$^1$H-NMR Spectrum (300MHz, DMSO-$d_6$):

$\delta$ (ppm): 0.71-0.75 (m, 2H), 1.23-1.30 (m, 2H), 2.40 (s, 3H), 2.92-2.94 (m, 1H), 4.70 (d, J=2.7Hz, 2H), 5.49 (t, J=2.7Hz, 1H), 6.55 (s, 1H), 7.65 (s, 1H), 8.03 (s, 1H)

A mixture of in 7-bromo-1-cyclopropyl-4-(hydroxymethyl)-6-methylhydroquinolin-2-one (125 mg, 0.405 mmol), 4-hydroxyphenylboronic acid (71.0 mg, 0.510 mmol), $PdCl_2$(dppf)(50 mg), and potassium carbonate (108 mg, 0.780 mmol) in dioxane/water (30 mL/3 mL) was stirred overnight at 80°C under a nitrogen gas atmosphere. The mixture was filtered, and concentrated, and the residue was purified by chromatography (dichloromethane:methanol = 50:1 to 20:1) to give the compound J-140, 1-cyclopropyl-4-(hydroxymethyl)-7-(4-hydroxyphenyl)-6-methylhydroquinolin-2-one, as gray solid (85 mg, yield 68%).
$^1$H-NMR Spectrum (300MHz, DMSO-$d_6$):

$\delta$ (ppm): 0.71-0.77 (m, 2H), 1.18-1.25 (m, 2H), 2.28 (s, 3H), 2.29-2.93 (m, 1H), 4.74 (d, J=4.2Hz, 2H), 5.45 (t, J=4.2Hz, 1H), 6.52 (s, 1H), 6.87 (d, J=9.6Hz, 2H), 7.25 (d, J=9.6Hz, 2H), 7.55 (s, 1H), 7.63 (s, 1H), 9.59 (brs, 1H)
MS Calcd.: 321
MS Found: 322 ($[M+1]^+$)

Example 25: Synthesis of the compound represented by the structural formula (25) (compound J-154)

A suspension of 3-bromo-2-fluorobenzoic acid (21.9 g, 100 mmol), DPPA (33.0 g, 120 mmol), and triethylamine (17 mL, 120 mmol) in toluene (1,000 mL) was stirred at 120°C. After the mixture was heated to reflux for 2 hours, t-BuOH was added to the mixture, and the mixture was heated to reflux for further 2 hours. The solvent was removed from the mixture, the residue was purified by silica gel column chromatography using petroleum ether to give tert-butoxy-N-(3-bromo-2-fluorophenyl)carboxamide as colorless oil (17.7 g, yield 61%).
$^1$H-NMR Spectrum (300MHz, $CDCl_3$):

$\delta$ (ppm): 1.52 (s, 3H), 6.72 (brs, 1H), 6.93-6.99 (m, 1H), 7.12-7.18 (m, 1H), 8.04 (t, J=7.5Hz, 1H)

A solution of tert-butoxy-N-(3-bromo-2-fluorophenyl)carboxamide (17.7 g, 59.5 mmol) in 5 N HCl/1,4-dioxane (500 mL) was stirred at room temperature for 18 hours. The mixture was filtered, the filtration cake was adjusted to pH 8 to 9 with 4 M NaOH, and extracted with ethyl acetate (2 x 50 mL). The combined organic layer was dried over $Na_2SO_4$, and concentrated in vacuo to give 3-bromo-2-fluorophenylamine as colorless oil (10.4 g, yield 94%).
$^1$H-NMR Spectrum (300MHz, DMSO-$d_6$):

$\delta$ (ppm): 6.83-6.70 (m, 3H), 5.43 (s, 2H)

To a suspension of 3-bromo-2-fluorophenylamine (10.5 g, 55.5 mmol) in toluene (100 mL), methyl 3-oxobutanoate (7.7 g, 66.7 mmol) was added, and the resulting mixture was stirred overnight at 110°C. The mixture was cooled

to room temperature, and then the mixture was concentrated in vacuo. The residue was purified by column chromatography (petroleum ether:ethyl acetate = 12:1 -> 8:1 -> 3:1) to give N-(3-bromo-2-fluorophenyl)-3-oxobutanamide as white solid (5.5 g, yield 36%).
$^1$H-NMR Spectrum (300MHz, $CDCl_3$):

δ (ppm): 2.33 (s, 3H), 3.63 (s, 2H), 7.03 (t, J=8.1Hz, 1H), 7.24-7.29 (m, 1H), 8.22 (t, J=8.1Hz, 1H), 9.52 (brs, 1H)

A solution of N-(3-bromo-2-fluorophenyl)-3-oxobutanamide (300 mg, 1.1 mmol) in $CF_3SO_3H$ (2.50 g, 16.5 mmol) was stirred overnight at room temperature. The mixture was poured on ice, and then solid was collected by filtration, and washed with water to give 7-bromo-8-fluoro-4-methylhydroquinolin-2-one as white solid (110 mg, yield 39%).
$^1$H-NMR Spectrum (300MHz, DMSO-$d_6$):

δ (ppm): 2.49 (s, 3H), 6.49 (s, 1H), 7.41-7.50 (m, 2H), 11.7 (brs, 1H)

A mixture of 7-bromo-8-fluoro-4-methylhydroquinolin-2-one (150 mg, 0.59 mmol), cyclopropylboronic acid (101 mg, 1.18 mmol), 2,2'-bipyridine (92 mg, 0.59 mmol), sodium carbonate (125 mg, 1.18 mmol), anhydrous cuprous acetate (107 mg, 0.59 mmol), and 1,2-dichloroethane (6 mL) was stirred overnight at 70°C. The mixture was cooled to room temperature, and then saturated ammonium chloride solution (30 mL) was added to the mixture, then the resulting mixture was filtered, and the filtrate was extracted with dichloromethane (3 x 30 mL). The combined organic layer was concentrated, and the residue was purified by chromatography (petroleum ether:ethyl acetate = 10:1 -> 8:1 -> 5:1) to give 7-bromo-1-cyclopropyl-8-fluoro-4-methylhydroquinolin-2-one as white solid (60 mg, yield 47%).
$^1$H-NMR Spectrum (300MHz, $CDCl_3$):

δ (ppm): 0.66-0.73 (m, 2H), 1.20-1.27 (m, 2H), 2.37 (d, J=1.2Hz, 3H), 3.36-3.42 (m, 1H), 6.49 (s, 1H), 7.25-7.29 (m, 1H), 7.35-7.40 (m, 1H)

A suspension of 7-bromo-1-cyclopropyl-8-fluoro-4-methylhydroquinolin-2-one (310 mg, 1.05 mmol) and $SeO_2$ (1.17 g, 10.5 mmol) in xylene (12 mL) was stirred at 145°C for 3 days. The mixture was cooled to room temperature, then water (50 mL) was added to the reaction mixture, and the resulting mixture was extracted with ethyl acetate (50 mL x 3). The combined organic layer was washed with brine, dried, and concentrated to give 7-bromo-1-cyclopropyl-8-fluoro-2-oxohydroquinoline-4-carboaldehyde as brown oil (350 mg, yield 100%).

To an ice-cooled solution of 7-bromo-1-cyclopropyl-8-fluoro-2-oxohydroquinoline-4-carboaldehyde (350 mg, 1.13 mmol) in methanol (8 mL), sodium borohydride (127 mg, 3.39 mmol) was added portionwise. After the addition, the reaction mixture was stirred at room temperature for 0.5 hours. The reaction mixture was filtered to give 7-bromo-1-cyclopropyl-8-fluoro-4-(hydroxymethyl)hydroquinolin-2-one as red solid (182 mg, yield 56%).
$^1$H-NMR Spectrum (300MHz, $CDCl_3$):

δ (ppm): 0.66-0.72 (m, 2H), 1.21-1.28 (m, 2H), 2.20 (t, J=6.3Hz, 1H), 3.36-3.42 (m, 1H), 4.83 (d, J=6.0Hz, 2H), 6.77 (s, 1H), 7.24-7.29 (m, 1H), 7.35-7.40 (m, 1H)

A mixture of 7-bromo-1-cyclopropyl-8-fluoro-4-(hydroxymethyl)hydroquinolin-2-one (182 mg, 0.59 mmol), 4-hydroxyphenylboronic acid (106 mg, 0.77 mmol), Pd(dppf)$Cl_2$ (70 mg), and potassium carbonate (122 mg, 0.90 mmol) in dioxane/water (10 mL/1 mL) was stirred overnight at 80°C under a nitrogen gas atmosphere. The reaction mixture was filtered, and concentrated, and the residue was purified by silica gel column chromatography using petroleum ether/ethyl acetate (10:1 -> 3:1 -> 1:1 -> 1: 3) to give the compound J-154, 1-cyclopropyl-8-fluoro-4-(hydroxymethyl)-7-(4-hydroxyphenyl)hydroquinolin-2-one, as yellow solid (150 mg, yield 79%).
$^1$H-NMR Spectrum (300MHz, DMSO-$d_6$):

δ (ppm): 0.58 (brs, 2H), 1.09-1.15 (m, 2H), 3.27-3.33 (m, 1H), 4.67 (d, J=6.0Hz, 2H), 5.42 (t, J=5.7Hz, 1H), 6.54 (s, 1H), 6.86-6.89 (m, 2H), 7.23-7.29 (m, 1H), 7.40-7.50 (m, 3H), 9.70 (s, 1H)
MS Calcd.: 325
MS Found: 326 ($[M+1]^+$)

Example 26

The following compounds were synthesized in the same manners as those of Examples 1 to 25.

Compound J-155 represented by the structural formula (26)

$^1$H-NMR (DMSO-$d_6$, 300MHz): δ 0.57-0.63 (m, 2H), 1.04-1.10 (m, 2H), 3.23-3.29 (m, 1H), 4.60-4.66 (m, 2H), 5.50-5.54 (m, 1H), 6.59 (s, 1H), 6.88-6.92 (m, 2H), 7.28-7.32 (m, 2H), 7.43-7.46 (m, 1H), 9.79 (brs, 1H)

Compound J-156 represented by the structural formula (27)

$^1$H-NMR (300MHz, DMSO-$d_6$): 0.46-0.47 (m, 2H), 1.04-1.06 (m, 2H), 1.23-1.26 (m, 1H), 3.24 (s, 3H), 4.63-4.65 (m, 2H), 5.50 (t, 1H, J=5.7Hz), 6.55 (s, 1H), 6.86-6.89 (m, 2H), 7.26-7.34 (m, 3H), 9.66 (s, 1H)

Compound J-166 represented by the structural formula (28)

$^1$H-NMR ($CDCl_3$, 300MHz) δ: 0.76-0.80 (m, 2H), 1.26-1.30 (m, 2H), 2.98-3.02 (m, 1H), 4.70 (s, 2H), 5.50 (s, 1H), 6.28 (s, 2H), 6.57 (s, 2H), 7.56-7.80 (m, 1H), 7.66-7.71 (m, 1H), 7.86-7.88 (m, 1H), 8.22-8.23 (m, 1H)

Compound J-173 represented by the structural formula (29)

$^1$H-NMR (300MHz, DMSO-$d_6$): δ 8.22-8.20 (m, 1H), 7.94-7.88 (m, 2H), 7.70 (d, J=11.4Hz, 1H), 7.55-7.45 (m, 2H), 6.92-6.90 (m, 2H), 6.62 (s, 1H), 3.07-3.00 (m, 1H), 1.31-1.24 (m, 2H), 0.77-0.73 (m, 2H)

Compound J-175 represented by the structural formula (30)

$^1$H-NMR (300MHz, DMSO-$d_6$): δ 9.78 (s, 1H), 7.88 (d, J=7.2Hz, 1H), 7.58 (d, J=7.2Hz, 1H), 7.49 (dd, J=8.7,1.8Hz, 2H), 6.92 (m, 2H), 6.55 (s, 1H), 4.64 (d, J=1.2Hz, 2H), 3.39 (s, 3H), 3.03-2.98 (m, 1H), 1.32-1.23 (m, 2H), 0.82-0.76 (m, 2H)

Compound J-131ACP represented by the structural formula (31)

$^1$H-NMR (DMSO-$d_6$, 300MHz): δ 0.75-0.77 (m, 2H), 1.26-1.28 (m, 2H), 2.13 (s, 3H), 2.95-3.01 (m, 1H), 5.30 (s, 1H), 6.52 (s, 1H), 6.91 (d, J=6.6Hz, 2H), 7.48 (d, J=6.6Hz, 3H), 7.62 (d, J=11.7Hz, 1H), 7.89 (d, J=6.9Hz, 1H), 9.80 (s, 1H)

Compound J-176 represented by the structural formula (32)

$^1$H-NMR (300MHz, DMSO-$d_6$): 0.75 (s, 2H), 1.26 (d, 2H, J=6.9Hz), 4.68 (s, 2H), 6.55 (s, 1H), 6.83 (d, 1H, J=7.8Hz), 6.91 (d, 1H, J=8.1Hz), 7.04 (s, 1H), 7.53 (d, 1H, J=9.0Hz), 7.53 (d, 1H, J=9.0Hz), 7.83 (d, 1H, J=6.9Hz)

Compound J-149A represented by the structural formula (33)

$^1$H-NMR (300MHz, DMSO-$d_6$): δ 9.66 (s, 1H), 7.6 (m, 3H), 6.87 (d, J=8.4Hz, 2H), 6.70 (s, 1H), 5.58 (m, 1H), 4.92 (d, J=6.4Hz, 1H), 4.73 (d, J=5.2Hz, 1H), 4.40 (d, J=11.2Hz, 1H), 4.11 (d, J=11.2Hz, 1H), 1.25 (d, J=6.4Hz, 3H)

Compound J-149d represented by the structural formula (34)

$^1$H-NMR (300MHz, DMSO-$d_6$): δ 9.66 (s, 1H), 7.32 (d, J=8.4Hz, 2H), 6.87 (d, J=8.4Hz, 2H), 6.59 (s, 1H), 4.90 (d, J=5.6Hz, 1H), 4.40 (d, J=11.2Hz, 1H), 4.09 (d, J=10Hz, 1H), 2.42 (s, 3H), 1.24 (d, J=6.8Hz, 3H)

Compound J-165 represented by the structural formula (35)

$^1$H-NMR (300MHz, DMSO-$d_6$): δ 8.37 (d, J=2.1Hz, 1H), 8.00 (d, J=1.5Hz, 1H), 7.65-7.71 (m, 2H), 7.47 (dd, J=8.4,1.8Hz, 1H), 6.51 (s, 1H), 6.22-6.24 (m, 1H), 5.44 (t, J=5.7Hz, 1H), 4.73-4.75 (m, 2H), 2.98-3.02 (m, 1H), 2.91 (d, J=4.5Hz, 3H), 2.15 (s, 3H), 1.31-1.35 (m, 2H), 0.74-0.79 (m, 2H)

Compound J-158 represented by the structural formula (36)

$^1$H-NMR (300MHz, DMSO-$d_6$): 0.63-0.66 (m, 2H), 1.25-1.31 (m, 2H), 3.50-3.54 (m, 1H), 4.86 (s, 2H), 6.07 (t, 1H, J=74.7Hz), 6.74 (s, 1H), 6.90 (d, 2H, J=8.7Hz), 7.36 (d, 1H, J=8.4Hz), 7.46 (d, 2H, J=8.7Hz), 7.67 (d, 1H, J=8.4Hz)

Compound J-162 represented by the structural formula (37)

$^1$H-NMR ($CDCl_3$, 300MHz) δ: 0.74-0.79 (m, 2H), 1.23-1.25 (m, 2H), 2.95-2.97 (m, 1H), 4.72-4.74 (m, 1H), 5.52-5.55 (m, 1H), 6.58 (s, 1H), 6.88-6.92 (m, 2H), 7.35-7.38 (m, 2H), 7.79-7.82 (m, 2H), 9.75 (s, 1H)

Compound J-161 represented by the structural formula (38)

$^1$H-NMR ($CD_3OD$, 300MHz) δ: 7.52 (d, J=9.6Hz, 1H), 7.21 (d, J=7.8Hz, 2H), 6.91 (dd, J=6.6,2.1Hz, 2H), 6.77 (s, 1H), 4.79 (s, 2H), 3.62 (m, 1H), 1.25-1.23 (m, 2H), 0.60-0.58

(m, 2H)

Compound J-167 represented by the structural formula (39)

$^1$H-NMR (300MHz, DMSO-$d_6$): δ 8.24 (brs, 2H), 7.92 (d, J=6.9Hz, 1H), 7.63-7.71 (m,5H), 6.62 (s, 1H), 5.57 (brs, 1H), 4.72 (s, 2H), 4.09 (s, 2H), 2.99-3.04 (m, 1H), 1.26-1.28 (m, 2H), 0.78-0.82 (m, 2H)

Compound J-168 represented by the structural formula (40)

$^1$H-NMR (300MHz, DMSO-$d_6$): δ 8.73-8.75 (m, 2H), 8.01 (d, J=6.9Hz, 1H), 7.57-7.72 (m, 3H), 6.65 (s, 1H), 5.54 (t, J=5.4Hz, 1H), 4.72-4.74 (m, 2H), 3.01-3.04 (m, 1H), 1.26-1.31 (m, 2H), 0.78-0.80 (m, 2H)

Compound J-169 represented by the structural formula (41)

$^1$H-NMR (300MHz, DMSO-$d_6$): δ 9.88 (s, 1H), 7.78-7.70 (m, 2H), 7.46-7.43 (m, 2H), 6.89 (d, J=5.4Hz, 2H), 4.55 (s, 2H), 2.94-2.91 (m, 1H), 1.28-1.23 (m, 2H), 0.74-0.71 (m, 2H) Compound J-174 represented by the structural formula (42)

$^1$H-NMR (300MHz, DMSO-$d_6$): δ 7.88 (d, J=6.9Hz, 1H), 7.63 (d, J=12.3Hz, 1H), 7.51-7.48 (m, 2H), 6.96-6.91 (m, 2H), 6.57 (s, 1H), 6.34 (s, 2H), 4.78 (s, 2H), 3.04-2.97 (m, 1H), 1.32-1.25 (m, 2H), 0.81-0.75 (m, 2H)

Compound J-179 represented by the structural formula (43)

$^1$H-NMR (300MHz, DMSO-$d_6$): 7.77 (d, J=7.2Hz, 1H), 7.49-7.41 (m, 3H), 6.88 (d, J=8.7Hz, 2H), 5.59 (s, 2H), 4.96 (s, 1H), 4.63 (m, 2H), 3.10-3.06 (m, 1H), 1.32-1.25 (m, 2H), 0.79-0.74 (m, 2H)

Compound J-144M represented by the structural formula (44)

$^1$H-NMR (300MHz, $CD_3OD$): 0.88-0.93 (m, 2H), 1.36-1.39 (m, 2H), 1.54 (d,3H, J=6.6Hz), 1.75 (d,3H, J=6.6Hz), 3.03-3.08 (m, 1H), 3.34 (s, 1H), 4.69-4.70 (m, 2H), 5.09-5.12 (m, 1H), 5.22-5.24 (m, 1H), 6.83 (s, 1H), 7.54 (d, 1H, J=6.3Hz), 7.71-7.80 (m, 3H), 8.08 (d, 1H, J=6.3Hz)

Test Example 1-1

Antibacterial activity of the compound represented by the structural formula (1) (compound J-131CP) against *Staphylococcus aureus* was examined by the following methods.

<Preparation of compound J-131CP and quinolones>

The compound J-131CP was dissolved in DMSO at a concentration of 1,024 mg/L. Cation-adjusted MHB (CAMBH) was separately prepared by adding magnesium and calcium to MHB (Mueller Hinton Broth) according to CLSI (Clinical and Laboratory Standards Institute), and the solution of the compound J-131CP was diluted 4 times with CAMHB to prepare a solution at a concentration of 256 mg/L. This solution was serially 2-fold-diluted 11 times in total to a concentration of 0.125 mg/L.

Solutions of norfloxacin (NOR, produced by Sigma-Aldrich), ofloxacin (OFX, produced by Sigma-Aldrich), levofloxacin (LVX, produced by LKT Laboratories, Inc.), ciprofloxacin (CIP, produced by MP Biomedicals, LLC), tosufloxacin (TSX, produced by Toyama Chemical), and sparfloxacin (SPX, produced by Dainippon Sumitomo Pharma), which are quinolones, were prepared at 1,024 mg/L with sterilized water or sterilized 0.1 M aqueous sodium hydroxide, and then diluted to a concentration of 0.125 mg/L in the same manner as that used for the compound J-131CP.

<Measurement of MIC of compound J-131CP for *Staphylococcus aureus*>

*S. aureus* Mu50 (refer to Non-patent documents 1 and 3), *S. aureus* FDA209P (ATCC 6538P), and *S. aureus* Mu50 NR1-1 (refer to Non-patent document 3) were each cultured overnight at 37°C in 4 mL of TSB (Tryptical Soy Broth, produced by Becton Dickinson) with shaking. After completion of the culture, the cells were collected, and suspended in fresh TSB to prepare a cell suspension showing an absorbance of 0.3 at 578 nm. Then, the cell suspension was diluted 500 times with CAMHB mentioned above.

CAMHB containing the compound J-131CP at each concentration or each quinolone at each concentration was added to each well of a 96-well plate in a volume of 50 μL/well, the aforementioned cell suspension diluted 500 times was added to the wells in a volume of 50 μL/well, and stationary culture was carried out overnight at 37°C. The final concentrations of the compound J-131CP and the quinolones at the time of culture were 128 to 0.0625 mg/L.

After completion of the culture, whether the bacterium proliferated or not was determined on the basis of visually observed turbidity of the culture in the well, and MIC values (mg/L) of the compound J-131CP and the quinolones for the strains were determined. The results are shown in Table 1. The ratios of MIC for *S. aureus* Mu50 and MIC for *S. aureus* FDA209P (ATCC 6538P) (MIC for *S. aureus* Mu50/MIC for *S. aureus* FDA209P (ATCC 6538P)) for the compounds are shown in Table 2.

[Table 1]

| | | MIC (mg/L) | | |
|---|---|---|---|---|
| Strain | | Mu50 | FDA209P | Mu50 NR1-1 |
| QRDR* mutation | ParC | S80F | 80S | S80F |
| | GyrA | S84L | 84S | 84S |
| J-131CP | | ≤0. 06 | 0. 25 | 0. 25 |
| NOR | | 128 | 0. 25 | 16 |
| OFX | | 16 | 0. 125 | 2 |
| LVX | | 8 | ≤0. 06 | 1 |
| CIP | | 32 | 0. 125 | 2 |
| TSX | | >128 | ≤0. 06 | 0. 5 |
| SPX | | 16 | ≤0. 06 | 0. 25 |
| DMSO | | >128 | >128 | >128 |

*QRDR: Quinolone resistance determination resion

[Table 2]

| | MIC ratio ( Mu50 MIC / FDA209P MIC ) |
|---|---|
| J-131CP | ≤0. 25 |
| NOR | 512 |
| OFX | 128 |
| LVX | ≥128 |
| CIP | 256 |
| TSX | ≥2048 |
| SPX | ≥256 |

*S. aureus* FDA209P (ATCC 6538P) is a methicillin-susceptible *Staphylococcus aureus* (MSSA).

*S. aureus* Mu50 was clinically separated (refer to Non-patent documents 1 and 3). *S. aureus* Mu50 is a quinolone-resistant MRSA having amino acid substitution mutations in the two genes *parC* and *gyrA* existing in the quinolone resistance determination region (QRDR), namely, the mutation from serine to phenylalanine for the 80th amino acid residue of the subunit of DNA topoisomerase IV encoded by the *parC* gene (S80F), and the mutation from serine to leucine for the 84th amino acid residue of DNA gyrase encoded by the *gyrA* gene (S84L) (refer to Non-patent document 1).

*S. aureus* Mu50 NR1-1 is a strain obtained by stepwise selection of *S. aureus* Mu50 using deoxynybomycin (refer to J. Antimicrob. Agents., 39(6), pp.478-485, 2012). *S. aureus* Mu50 NR1-1 is a mutant strain in which quinolone-susceptibility is restored, and the 84th amino acid residue of the DNA gyrase encoded by *gyrA* is reverted to serine by reverse mutation (refer to Non-patent document 3).

As seen in the results shown in Table 1, MIC of the compound J-131CP for *S. aureus* FDA209P was 0.25 mg/L, and thus it showed an outstanding antibacterial activity. MIC of the compound J-131CP for *S. aureus* Mu50 is 0.06 mg/L or lower, and thus it showed an antibacterial activity 4 times or more higher than that for *S. aureus* FDA209P (Table 2). It was demonstrated that the compound J-131CP has a characteristic of a reverse antibiotics of showing higher antibacterial activity against quinolone-resistant bacteria compared with that for quinolone-susceptible bacteria.

MIC of the compound J-131CP for *S. aureus* Mu50 NR1-1 was 0.25 mg/L, and was the same as MIC for quinolone-susceptible *S. aureus* FDA209P.

On the other hand, MICs of the quinolones, NOR, OFX, LVX, CIP, TSX, and SPX, for the quinolone-resistant MRSA, *S. aureus* Mu50, were 8 to 128 mg/L or higher, and therefore they showed only weaker antibacterial activity compared with the compound J-131CP.

The results described above suggested that the compound J-131CP is extremely useful as an antibacterial agent for *Staphylococcus aureus,* especially a quinolone-resistant *Staphylococcus aureus.* Moreover, since MIC of the compound J-131CP for *S. aureus* Mu50 NR1-1 having the reverse mutation of *gyrA* was the same as MIC for quinolone-susceptible *S. aureus* FDA209P, it was suggested that the action site of the compound J-131CP is *gyrA.*

Test Example 1-2

Antibacterial activities of the compounds represented by the structural formulas (2) to (25) for *Staphylococcus aureus* were examined by the following methods.

The compounds represented by the structural formulas (2) to (25) were each prepared in the same manner as that for the compound J-131CP described in Test Example 1-1. *S. aureus* Mu50 and *S. aureus* FDA209P (ATCC 6538P) were each prepared in the same manner as that described in Test Example 1-1. MICs (mg/L) were determined in the same manner as that described in Test Example 1-1, except that the compounds represented by the structural formulas (2) to (25) were used as the test compounds, and *S. aureus* Mu50 and *S. aureus* FDA209P (ATCC 6538P) were used as the bacteria. The results are shown in Table 3 together with ratios of MICs for *S. aureus* Mu50 and MICs for *S. aureus* FDA209P (ATCC 6538P) (MIC for *S. aureus* Mu50/MIC for *S. aureus* FDA209P (ATCC 6538P)).

[Table 3]

| **Compound** | MIC (mg/L) | | **MIC ratio** ( Mu50 MIC/FDA209P MIC ) |
|---|---|---|---|
| | Mu50 | FDA209P | |
| **Formula** (2) (J-103) | 16 | 128 | 0.125 |
| **Formula** (3) (J-125a) | 16 | 128 | 0.125 |
| **Formula** (4) (J-2.1.2) | 16 | 128 | 0.125 |
| **Formula** (5) (J-2.1.1) | 16 | 32 | 0.5 |
| **Formula** (6) (J-136) | 8 | 32 | 0.25 |
| **Formula** (7) (J-147) | 16 | 32 | 0.5 |
| **Formula** (8) (J-146) | 16 | 16 | 1 |
| **Formula** (9) (J-132) | 8 | 128 | 0.0625 |
| **Formula** (10) (J-135) | 8 | 8 | 1 |
| **Formula** (11) (J-121) | 4 | 128 | 0.0313 |
| **Formula** (12) (J-121Me) | 4 | 128 | 0.0313 |
| **Formula** (13) (J-131ACp) | 4 | 64 | 0.0625 |
| **Formula** (14) (J-131) | 4 | 16 | 0.25 |
| **Formula** (15) (J-157) | 2 | 4 | 0.5 |
| **Formula** (16) (J-138) | 2 | 2 | 1 |
| **Formula** (17) (J-144) | 1 | 0.5 | 2 |
| **Formula** (18) (J-131E) | 0.5 | 2 | 0.25 |
| **Formula** (19) (J-121Cp) | 0.5 | 1 | 0.5 |
| **Formula** (20) (J-132CP) | 0.5 | 0.5 | 1 |
| **Formula** (21) (J-149) | 0.5 | 0.5 | 1 |

(continued)

| **Compound** | MIC (mg/L) | | **MIC ratio** ( Mu50 MIC/FDA209P MIC ) |
|---|---|---|---|
| | Mu50 | FDA209P | |
| **Formula** (22) (J-139) | 0.5 | 0.5 | 1 |
| **Formula** (23) (J-150) | 0.25 | 4 | 0.0625 |
| **Formula** (24) (J-140) | 0.25 | 1 | 0.25 |
| **Formula** (25) (J-154) | 0.25 | 0.5 | 0.5 |

The results shown in Table 3 suggested that the compounds represented by the structural formulas (2) to (25) are also extremely useful as antibacterial agents for *Staphylococcus aureus,* especially quinolone-resistant *Staphylococcus aureus,* like the compound represented by the structural formula (1).

Moreover, since the ratios of MIC for the quinolone-resistant MRSA, *S. aureus* Mu50, and the MIC for the quinolone-susceptible *S. aureus* FDA209P of the compounds represented by the structural formulas (1) to (4), (6), (9), (11) to (14), (18), (23), and (24) were 0.25 or smaller as seen in the results shown in Tables 2 and 3, it was suggested that, by using them together with a quinolone as a reverse antibiotics, effect of suppressing emergence of quinolone-resistant bacteria can be expected.

The results of Test Examples 1-1 and 1-2 revealed that the compounds represented by the structural formulas (1) to (25) have an antibacterial activity for quinolone-resistant *Staphylococcus aureus* equivalent to or higher than that for quinolone-susceptible *Staphylococcus aureus.* The antibacterial activity equivalent to that for quinolone-susceptible *Staphylococcus aureus* means that the ratio of MIC for quinolone-resistant *Staphylococcus aureus* and MIC for quinolone-susceptible *Staphylococcus aureus* (MIC for quinolone-resistant *Staphylococcus aureus*/MIC for quinolone-susceptible *Staphylococcus aureus*) is not smaller than 1/2 and not larger than 2.

Test Example 2

In order to further verify that the action site of the compound J-131CP is *gyrA,* there were examined antibacterial activities against the MRSA, *S. aureus* MR5867, and stepwise-mutated strains (one-step mutant strain to three-step mutant strain) of *S. aureus* MR5867 in which mutation was introduced into QRDR, as well as the MSSA, *S. aureus* MS5952, and stepwise-mutated strains (one-step mutant strain to three-step mutant strain) of *S. aureus* MS5952 in which mutation was introduced into QRDR (those strains mentioned above are described in Antimicrob. Agents Chemother., 42(8), pp.1917-1922, 1998) by the following methods. The strains used for this test example are available from the inventors of the present invention.

The compound J-131CP and quinolones were prepared in the same manner as that of Test Example 1-1.

Each of *S. aureus* MR5867, and stepwise-mutated strains (one-step mutant strain to three-step mutant strain) of *S. aureus* MR5867 in which mutation was introduced into QRDR, as well as *S. aureus* MS5952, and stepwise-mutated strains (one-step mutant strain to three-step mutant strain) of *S. aureus* MS5952 in which mutation was introduced into QRDR was cultured overnight at 37°C in 4 mL of TSB(produced by Becton Dickinson) with shaking. After completion of the culture, the cells were collected, and suspended in fresh TSB to prepare a cell suspension showing an absorbance of 0.3 at 578 nm. Then, the cell suspension was diluted 500 times with CAMHB mentioned above.

CAMHB containing each of the compound J-131CP and quinolones at each concentration was added to each well of a 96-well plate in a volume of 50 μL/well, the aforementioned cell suspension diluted 500 times was added to the well in a volume of 50 μL/well, and stationary culture was carried out overnight at 37°C in the same manner as that of Test Example 1-1. The final concentrations of the compound J-131CP and quinolones at the time of culture were 128 to 0.0625 mg/L. After completion of the culture, whether the bacterium proliferated or not was determined on the basis of visually observed turbidity of the culture in the well, and MIC values (mg/L) of the compound J-131CP and quinolones against the strains were determined. The results are shown in Tables 4 and 5.

[Table 4]

| Strain | | MIC against MR5867 and stepwise mutants thereof (mg/L) | | | |
|---|---|---|---|---|---|
| | | MR5867 | One-step mutant | Two-step mutant | Three-step mutant |
| QRDR | ParC | 80Ser<br>84Glu | 80Ser<br>84Lys# | 80Ser<br>84Lys# | 80Phe#<br>84Lys# |
| | GyrA | 84Ser | 84Ser | 84Leu# | 84Leu# |
| | J-131CP | 0.25 | 0.25 | ≤0.06 | ≤0.06 |
| | NOR | 1 | 16 | 64 | >128 |
| | OFX | 0.25 | 1 | 16 | 64 |
| | LVX | 0.125 | 1 | 8 | 16 |
| | CIP | 0.25 | 2 | 32 | 128 |
| | TSX | <0.06 | 0.25 | 8 | >128 |
| | SPX | <0.06 | <0.06 | 8 | 16 |
| | DMSO | >128 | >128 | >128 | >128 |
| *The underlines indicate QRSR mutations introduced into the strain MR5867. | | | | | |

[Table 5]

| Strain | | MIC against MR5952 and stepwise mutants thereof (mg/L) | | | |
|---|---|---|---|---|---|
| | | MS5952 | One-step mutant | Two-step mutant | Three-step mutant |
| QRDR | ParC | 80Ser<br>116Ala | 80Tyr#<br>116Ala | 80Tyr#<br>116Ala | 80Tyr#<br>116Val# |
| | GyrA | 84Ser | 84Ser | 84Leu# | 84Leu# |
| | J-131CP | 0.25 | 0.25 | 0.125 | 0.125 |
| | NOR | 1 | 16 | 16 | 128 |
| | OFX | 0.25 | 1 | 8 | 64 |
| | LVX | 0.25 | 0.5 | 4 | 32 |
| | CIP | 0.25 | 2 | 8 | 64 |
| | TSX | 0.25 | 2 | 8 | >128 |
| | SPX | <0.06 | 8 | 16 | 128 |
| | DMSO | >128 | >128 | >128 | >128 |
| *The underlines indicate QRSR mutations introduced into the strain MR5952. | | | | | |

As seen from the results shown in Tables 4 and 5, it was observed that when mutations were successively introduced into QRDR of MRSA, *S. aureus* MR5867, and MSSA, *S. aureus* MS5952, MICs of quinolones against the stepwise-mutated strains introduced with the mutations correspondingly increased.

On the other hand, MIC of the compound J-131CP decreased for the two-step mutant strain in which a mutation was introduced into the *gyrA* gene encoding DNA gyrase. Therefore, it was strongly suggested that the target protein of the compound J-131CP is DNA gyrase.

Test Example 3

The antibacterial activities of the compound J-131CP against *Staphylococcus aureus* strains belonging to the five categories of *Staphylococcus aureus,* i.e., MSSA, MRSA, community-acquired-infection type MRSA (CA-MRSA), VISA, and VRSA, were investigated.

CA-MRSA is a new MRSA clone that emerged in the 2000s, shows potent pathogenicity and propagation power, and causes community acquired infection (Clin. Microbiol., 40 (11), pp.4289-4294, 2002). All the *Staphylococcus aureus* strains belonging to MSSA, MRSA, CA-MRSA, VISA, and VRSA used for this test example are available from

National Collection of Type Cultures (NCTC), or the inventors of the present invention.

The compound J-131CP and quinolones were prepared in the same manner as that of Test Example 1-1.

Cell suspensions were prepared in the same manner as that of Test Example 1-1 described in the section of measurement of MIC in Test Example 1-1, except that the strains mentioned in Table 6 were used as *Staphylococcus aureus,* and MICs of the compound J-131CP and quinolones as the control were determined for the strains in the same manner as that of Test Example 1-1. The results are shown in Table 7. In Table 6, the amino acid substitution mutations in QRDR of the strains are underlined.

[Table 6]

| Strain | Category | QRDR amino acid residue | | | |
|---|---|---|---|---|---|
| | | ParC | | GyrA | |
| ATCC29213 | MSSA | 80Ser | 84Glu | 84Ser | 88Glu |
| NCTC8325 | MSSA | 80Ser | 84Glu | 84Ser | 88Glu |
| MW2 | CA-MRSA | 80Ser | 84Glu | 84Ser | 88Glu |
| USA300FPR | CA-MRSA | 80Tyr | 84Glu | 84Leu | 88Glu |
| KSA36 | MRSA | 80Tyr | 84Glu | 84Leu | 88Gly |
| BR2 | VISA | 80Phe | 84Glu | 84Leu | 88Glu |
| MI | VISA | 80Phe | 84Glu | 84Leu | 88Glu |
| VRS1 | VRSA | 80Phe | 84Glu | 84Leu | 88Glu |
| VRS3a | VRSA | 80Tyr | 84Lys | 84Leu | 88Glu |
| VRS5 | VRSA | 80Tyr | 84Gly | 84Leu | 88Lys |

[Table 7]

| Strain | Category | MIC (mg/L) | | | | | | | |
|---|---|---|---|---|---|---|---|---|---|
| | | J-131CP | NOR | OFX | LVX | CIP | TSX | SPX | DMSO |
| ATCC29213 | MSSA | 0.25 | 1 | 0.25 | 0.125 | 0.25 | ≤0.06 | ≤0.06 | >128 |
| NCTC8325 | MSSA | 0.125 | 1 | 0.25 | 0.25 | 0.25 | ≤0.06 | 0.125 | >128 |
| MW2 | CA-MRSA | 0.125 | 0.5 | 0.5 | 0.125 | 0.25 | ≤0.06 | ≤0.06 | >128 |
| USA300FPR | CA-MRSA | ≤0.06 | 32 | 16 | 4 | 16 | 16 | 8 | >128 |
| KSA36 | MRSA | ≤0.06 | >128 | >128 | >128 | 128 | >128 | 128 | >128 |
| BR2 | VISA | ≤0.06 | 64 | 64 | 8 | 4 | 32 | 32 | >128 |
| MI | VISA | ≤0.06 | 128 | 128 | 32 | 16 | 16 | 32 | 128 |
| VRS1 | VRSA | ≤0.06 | >128 | 32 | 16 | 128 | 4 | 8 | >128 |
| VRS3a | VRSA | ≤0.06 | 128 | 32 | 16 | 64 | >128 | 16 | >128 |
| VRS5 | VRSA | ≤0.06 | >128 | >128 | >128 | >128 | >128 | 128 | >128 |

As shown in Table 7, MICs of the compound J-131CP were ≤0.06 mg/L for the quinolone-resistant *Staphylococcus aureus* strains having a mutation in QRDR (USA300FPR, KSA36, BR2, MI, VRS1, VRS3a, and VRS5), and thus it was demonstrated that it has an extremely potent antibacterial activity. MICs of the compound J-131CP were 0.125 to 0.25 mg/L for the quinolone-susceptible wild-type *Staphylococcus aureus* strains having QRDR (ATCC 29213, NCTC 8325, and MW2), namely, in comparison with quinolones, it showed an antibacterial activity stronger than that of NOR, and equivalent to those of OFX, LVX, and CIP, although it was slightly weaker than those of SPX and TSX.

Test Example 4-1

Antibacterial activities of the compound J-131CP against *Enterococcus* bacteria (*Enterococcus faeeium* and *Enterococcus faecalis*) were examined by the following methods.

All of the vancomycin-resistant *Enterococcus* bacteria (VRE) and the other *Enterococcus* bacteria used in this test example are available from National Collection of Type Cultures (NCTC), or the inventors of the present invention.

The compound J-131CP and quinolones were prepared in the same manner as that of Test Example 1-1.

Cell suspensions were prepared in the same manner as that of Test Example 1-1 described in the section of

measurement of MIC in Test Example 1-1, except that the *Enterococcus* bacterium strains mentioned in Table 8 were used instead of *Staphylococcus aureus,* and MICs of the compound J-131CP and quinolones as control were determined for the strains in the same manner as that of Test Example 1-1 described in the section of measurement of MIC in Test Example 1-1. The results are shown in Table 9. In Table 8, the amino acid substitution mutations in QRDR of the strains are underlined.

[Table 8]

| Strain | Species | Category | Amino acid residue in QRDR | | |
|---|---|---|---|---|---|
| | | | ParC | GyrA | |
| NCTC12202 | *E. faecium* | VRE | 82Ser | 84Ser | 88Glu |
| NCTC12204 | *E. faecium* | VRE | 82Ser | 84Ser | 88Glu |
| RPR10 | *E. feecium* | | 82Ser | 84Ser | 88Glu |
| RPR2 | *E. faecium* | | 82Ile | 84Ile | 88Lys |
| NCTC12201 | *E. faecalis* | VRE | 80Ser | 84Ser | |
| NCTC12203 | *E. faecalis* | VRE | 80Ser | 84Ser | |
| 36-15747 | *E. faecalis* | | 80Ile | 84Ser | |
| 36-15722 | *E. faecalis* | | 80Ile | 84Ile | |

[Table 9]

| Strain | Species | Category | MIC(mg/L) | | | | | | | |
|---|---|---|---|---|---|---|---|---|---|---|
| | | | J-131CP | NOR | LVX | OFX | CIP | TFX | SPX | DMSO |
| NCTC12202 | *E. faecium* | VRE | 8 | 2 | 1 | 1 | 0. 5 | 0. 25 | 0. 25 | 128 |
| NCTC12204 | *E. faecium* | VRE | 8 | 4 | 2 | 4 | 4 | 4 | 1 | 128 |
| RPR10 | *E. faecium* | | 4 | 2 | 1 | 2 | 1 | 4 | 0. 13 | 128 |
| RPR2 | *E faecium* | | 4 | >128 | >128 | >128 | >128 | >128 | >128 | 128 |
| NCTC12201 | *E. faecalis* | VRE | 0. 5 | 2 | 1 | 2 | 1 | 0.25 | 0. 5 | 128 |
| NCTC12203 | *E. faecalis* | VRE | 2 | 4 | 2 | 4 | 4 | 4 | 1 | 128 |
| 36-15747 | *E. faecalis* | | 0. 5 | 16 | 2 | 4 | 2 | 2 | 0. 5 | 128 |
| 36-15722 | *E. faecalis* | | 1 | 128 | 36 | 64 | 64 | >128 | 64 | 128 |

As shown in Table 9, the antibacterial activities of the compound J-131CP against the VRE strains belonging to *E. faecium* (NCTC 12202, NCTC 11104) and the *E. faecium* strain (RPR10), which did not have any mutation in QRDR, were lower than the antibacterial activities of quinolones. On the other hand, against the *E. faecium* strain having a mutation in GyrA (RPR2), whereas MICs of all the quinolones were >128 mg/L, MIC of the compound J-131CP was 4 mg/L, and thus it showed antibacterial activity higher than those of the quinolones.

The compound J-131CP showed MICs equivalent to or lower than those of the quinolones for the strains belonging to *E. faecalis,* except for MIC of TFX for NCTC 12201, and MIC of SPX for NCTC 12203. In particular, the compound J-131CP has a mutation in GyrA, and showed a low MIC as low as 1 mg/L for 36-15722, which showed advanced resistance to quinolones (MICs of quinolones are ≥64 mg/L).

Test Example 4-2

Antibacterial activities of the compounds represented by the structural formulas (20), (22), (17), (21), and (25) against *Enterococcus* bacteria (*Enterococcus faecium* and *Enterococcus faecalis*) were examined by the following method.

The compounds represented by the structural formulas (20), (22), (17), (21), and (25) were prepared in the same manner as that for the compound J-131CP mentioned in Test Example 1-1.

Cell suspensions were prepared in the same manner as that of Test Example 4-1 described in the section of measurement of MIC in Test Example 4-1, and MICs of the compounds for the strains were measured in the same manner as that of Test Example 4-1 described in the section of measurement of MIC in Test Example 4-1, except that the compounds represented by the structural formulas (20), (22), (17), (21), and (25) were used, and the strains mentioned

in Table 10 were used. The results are shown in Table 10. In Table 10, MICs of LVX measured in Test Example 4-1 are also shown.

[Table 10]

| Compound | MIC (mg/L) | | | |
|---|---|---|---|---|
| | *E. faecium* | | *E. faecalis* | |
| | NCTC12202 | RPR2 | NCTC12201 | 36-15722 |
| Formula(20) (J-132CP) | 16 | 8 | 2 | 2 |
| Formula(22) (J-139) | 8 | 8 | 1 | 4 |
| Formula(17) (J-144) | 8 | 4 | 0.5 | 0.5 |
| Formula(21) (J-149) | 128 | 16 | 0.5 | 0.5 |
| Formula(25) (J-154) | 16 | 8 | 4 | 2 |
| LVX | 1 | >128 | 1 | 36 |

As shown in Table 10, the compounds represented by the structural formulas (20), (22), (17), (21), and (25) showed stronger antibacterial activities against the quinolone-resistant *E. faecium* (RPR2) compared with those against the quinolone-susceptible *E. faecium* (NCTC 12202). It was also demonstrated that they had favorable antibacterial activities against *E. faecalis* (NCTC 12201 and 36-15722) irrespective of whether the strains were quinolone-susceptible or resistant.

Test Example 5-1

Antibacterial activity of the compound J-131CP against pneumococcus (*Streptococcus pneumoniae*) was examined by the following method. The *S. pneumoniae* strains used for this test belong to any one of the categories of penicillin-susceptible pneumococcus (penicillin-susceptible *Streptococcus pneumoniae,* PSSP), penicillin-intermediate pneumococcus (penicillin-intermediate *Streptococcus pneumoniae,* PISP), and penicillin-resistant pneumococcus (penicillin-resistant *Streptococcus pneumoniae,* PRSP).

PSSP, PISP, and PRSP are defined by MICs of penicillin G not higher than 0.063 mg/L, higher than 0.063 mg/L and not higher than 1 mg/L, and not lower than 2 mg/L, respectively. The *S. pneumoniae* strains used for this test example can be obtained from the inventors of the present invention (described in Journal of the Japanese Society of Chemotherapy, 56(1), pap.16-20, 2008).

The compound J-131CP and quinolones were prepared in the same manner as that of Test Example 1-1, except that CAMHB containing 5 mass % of lysed horse blood (LHB) was used for the dilution instead of CAMHB. The lysed horse blood was prepared by adding sterilized water to horse sterile defibered blood (produced by Nippon Bio-Supp. Center) at a ratio of 1:1, subjecting the mixture to freezing and thawing 5 times, then centrifuging the mixture twice at 10,000 rpm for 20 minutes, and filtering the supernatant through a filter.

The *S. pneumoniae* strains mentioned in Table 11 were each cultured overnight at 37°C on a blood agar medium as stationary culture. The blood agar medium was prepared by adding 5 mass % ovine sterile defibered blood (produced by Nippon Bio-Supp. Center) to a heart infusion agar medium (produced by EIKEN CHEMICAL). Each *S. pneumoniae* strain cultured overnight at 37°C as stationary culture was further subcultured overnight at 37°C on fresh blood agar medium as stationary culture. After completion of the culture, cells of each pneumococcus strain were collected with a swab, and suspended in physiological saline, and the suspension was adjusted with physiological saline to show absorbance of 0.08 to 0.13 at 625 nm as measured with an absorbance meter (produced by Amersham), and further diluted 200 times with CAMHB containing 5 mass % LHB.

CAMHB containing the compound J-131CP at each concentration or each quinolone at each concentration, and 5 mass % LHB was added to each well of a 96-well plate in a volume of 50 μL/well, each cell suspension diluted 200 times was added to the well in a volume of 50 μL/well, and stationary culture was carried out overnight at 37°C. The final concentrations of the compound J-131CP and the quinolones at the time of culture were 128 to 0.0625 mg/L.

After completion of the culture, whether the bacterium proliferated or not was determined on the basis of visually observed turbidity of the culture in the well, and MIC values (mg/L) of the compound J-131CP and the quinolones for the strains were determined. The results are shown in Table 12. In Table 11, the amino acid substitution mutations in QRDR of the strains are underlined.

[Table 11]

| Strain | Category | QRDR amino acid residue | | |
|---|---|---|---|---|
| | | ParC | GyrA | |
| KBSP04 | PISP | 79Ser | 81Ser | 85Glu |
| KBSP11 | PRSP | 79Ser | 81Ser | 85Glu |
| BCLSP16 | PSSP | 79Tyr | 81Phe | 85Glu |
| JHSP11 | PSSP | 79Tyr | 81Ser | 85Lys |
| BCLSP37 | PSSP | 79Phe | 81Ser | 85Lys |

[Table 12]

| Strain | Category | MIC (mg/L) | | | | | | | |
|---|---|---|---|---|---|---|---|---|---|
| | | J-131CP | NOR | OFX | LVX | CIP | TSX | SPX | DMSO |
| KBSP04 | PISP | 4 | 4 | 2 | 0.5 | 0.5 | 0.125 | 0.125 | 128 |
| KBSP11 | PRSP | 2 | 4 | 1 | 0.5 | 0.5 | 0.125 | 0.25 | 128 |
| BCLSP16 | PSSP | 4 | >128 | 32 | 16 | >32 | 8 | 16 | 128 |
| JHSP11 | PSSP | 4 | 128 | 64 | 8 | >32 | 8 | >32 | 64 |
| BCLSP37 | PSSP | 4 | 64 | 32 | 32 | >32 | 16 | >32 | 64 |

As shown in Table 12, the antibacterial activities of the compound J-131CP against *S. pneumoniae* strains used for this test were lower than the antibacterial activities of the quinolones used for this test except for NOR for the two strains in which QRDR did not have any mutation (KBSP04 and KBSP11). On the other hand, for the three strains having a mutation in QRDR (BCLSP16, JHSP11, and BCLSP37), the antibacterial activities of the compound J-131CP exceeded the antibacterial activities of all the quinolones used for this test. Any correlation was not observed with the categories of the bacteria based on penicillin G susceptibility.

Test Example 5-2

Antibacterial activities of the compounds represented by the structural formulas (20), (22), (17), (21), and (25) against pneumococci (*Streptococcus pneumoniae*) were examined.
The compounds represented by the structural formulas (20), (22), (17), (21), and (25) were prepared in the same manner as that for the compound J-131CP mentioned in Test Example 5-1.
Cell suspensions were prepared in the same manner as that of Test Example 5-1 described in the section of measurement of MIC in Test Example 5-1, and MICs of the compounds against the strains were determined in the same manner as that of Test Example 5-1 described in the section of measurement of MIC in Test Example 5-1, except that the compounds represented by the structural formulas (20), (22), (17), (21), and (25) were used, and the strains mentioned in Table 13 mentioned below were used. The results are shown in Table 13.

[Table 13]

| Compound | MIC (mg/L) | |
|---|---|---|
| | KBSP11 | BCLSP37 |
| Formula(20) (J-132CP) | 2 | 4 |
| Formula(22) (J-139) | 1 | 4 |
| Formula(17) (J-144) | 0.25 | 0.5 |
| Formula(21) (J-149) | 4 | 8 |
| Formula(25) (J-154) | 8 | 8 |

As shown in Table 13, it was found that the compounds represented by the structural formulas (20), (22), (17), (21), and (25) tended to show stronger antibacterial activities against the quinolone-resistant strain (BCLSP37) compared with the quinolone-susceptible strain (KBSP11).

Test Example 6

In order to confirm that the compound J-131CP targets a mutant DNA gyrase, a DNA cleavage assay using DNA gyrase was performed by the following method with the compound J-131CP (refer to J. Antimicrob. Agents, 39(6), pp.478-485, 2012; Methods Mol., Med., 142, pp.11-23, 2008; and Antimicrob. Agents Chemother., 41, pp.2362-2366, 1997).

The DNA encoding the wild-type GyrA of *Staphylococcus aureus* (henceforth also referred to as "GyrA(wt)") shown as SEQ ID NO: 1 was cloned into the plasmid pMAL-c2 (produced by New England Biolabs) (the resultant is henceforth also referred to as "pMAL-c2-*gyrA*").

The DNA encoding the wild-type GyrB of *Staphylococcus aureus* (henceforth also referred to as "GyrB(wt)") shown as SEQ ID NO: 2 was cloned into the plasmid pMAL-c2 (produced by New England Biolabs) (the resultant is henceforth also referred to as "pMAL-c2-*gyrB*").

*E. coli* BL21(DE3) (produced by Invitrogen) was transformed with pMAL-c2-*gyrA,* pMAL-c2-*gyrB*, or pMAL-c2-SAGAmut84 containing a mutant *gyrA* encoding the amino acid sequence of GyrA(wt) in which serine residue at the 84th position from the amino terminus is substituted with a leucine residue (see, Int. J. Antimicrob. Agents, 39(6), 478-85, 2012). To the culture medium of the resulting transformed *Escherichia coli,* 1 mM isopropyl-β-thiogalactopyranoside (produced by Wako Pure Chemical Industries) was added to induce expression of maltose-binding GyrA(wt), maltose-binding GyrB(wt), or maltose-binding mutant GyrA(S84L), and the bacterium was cultured overnight at 25°C. After completion of the culture, the cells were collected, and suspended in a buffer (20 mM Tris-HCl (pH 8.0), 200 mM sodium chloride, 1 mM ethylenediaminetetraacetic acid (EDTA), 1 mM dithiothreitol (DTT)), a protein extracting reagent (10 x BugBuster (registered trademark), produced by Novagen) in a volume of 1/10 of the volume of the buffer, and 2.5 μL/10 mL of the buffer of Benzonase (registered trademark) nuclease (produced by Novagen) were added, the mixture was left standing at 25°C for 30 minutes, and the cells were disrupted and lysed to obtain a disrupted cell suspension containing the maltose-binding GyrA(wt), maltose-binding GyrB(wt), or maltose-binding mutant GyrA(S84L).

Then, each disrupted cell suspension was added to a column filled with an amylose resin covalently bound with maltose (produced by Novagen). A buffer (20 mM Tris-HCl (pH 8.0), 200 mM sodium chloride, 1 mM EDTA) containing 10 mM maltose was added to the column to elute the maltose-binding GyrA(wt), maltose-binding GyrB(wt), and maltose-binding mutant GyrA(S84L).

To each eluate, 4 units/1 mL of the eluate of protease (Factor Xa, produced by Amersham) was added, and the mixture was left standing at 25°C for 3 hours and 30 minutes to cleave the maltose-binding proteins, GyrA(wt), GyrB(wt), and mutant GyrA(S84L), from the maltose-binding GyrA(wt), maltose-binding GyrB(wt), and maltose-binding mutant GyrA(S84L).

Then, GyrA(wt), GyrB(wt), and mutant GyrA(S84L) were concentrated by using an ultrafiltration unit (YM-50 Filter Unit, produced by Millipore).

The concentrated GyrA(wt), GyrB(wt), and mutant GyrA(S84L) were each suspended in a buffer (35 mM Tris-HCl (pH 7.5), 24 mM potassium chloride, 6 mM magnesium chloride, 1.8 mM spermidine, 0.36 mg/mL bovine serum albumin, 5 mM DTT, 50 volume % glycerol) at a concentration of 1 μg/μL for GyrA(wt), 2 μg/μL for GyrB(wt), or 1 μg/μL for mutant GyrA(S84L).

A substrate plasmid DNA, pTWV228 (0.4 μg, produced by Takara Bio), and levofloxacin (LVFX) or the compound J-131CP were added to a buffer (35 mM Tris-HCl (pH 7.5), 24 mM potassium chloride, 6 mM magnesium chloride, 1.8 mM spermidine, 0.36 mg/mL bovine serum albumin, 5 mM DTT), and 0.15 μL of the enzyme solution of GyrA(wt) and 0.2 μL of the enzyme solution of GyrB(wt) were added to the mixture to prepare a reaction mixture in a total volume of 20 μL. Levofloxacin was added at a final concentration of 16 μg/mL as control for an index of the enzymatic activity. The compound J-131CP was added at a final concentration of 0, 0.125, 0.25, 0.5, 1, 2, or 4 μg/mL.

The reaction solution was left standing at 25°C for 30 minutes to allow the reaction. Subsequently, a 6 mass % solution of sodium laurylsulfate (2 μL) and a 5 mg/mL solution of Proteinase K (2 μL, produced by Wako Pure Chemical Industries) were added to the reaction solution, and the mixture was left standing at 37°C for 30 minutes to inactivate GyrA(wt) and GyrB(wt) bound to DNA.

Then, the whole volume of the reaction solution treated with Proteinase K was subjected to electrophoresis on 1 mass % agarose gel to visualize the resultants of the reaction. The results are shown in Fig. 1

Intensities of the bands in the visualized images shown in Fig. 1 were quantified by using software (ImageJ v. 10.2, National Institutes of Health, littp://rsb.info.nih.gov/ij/), and $IC_{50}$ values of the compound J-131CP were calculated by the method described in Antimicrob. Agents Chemother., 43(5), 1129-36, 1999. Specifically, the intensities of the bands of "S", "L", and "N" were measured for each concentration of levofloxacin (LVX) or the compound J-131CP by using the aforementioned software. Then, the total value of the intensities of the bands of "S", "L", and "N" was calculated for each concentration, and the result was used as "total DNA amount" (equation 1 mentioned below). Further, the total value of the intensities of the bands of "L" and "N" was also calculated, and the result was used as "amount of cleaved DNA" (equation 2 mentioned below). Then, the ratio (%) of the amount of the substrate plasmid DNA to the total DNA

amount was calculated in accordance with the following equation 3, and the ratio (%) of the amount of the cleaved DNA to the total DNA amount was calculated in accordance with the following equation 4. $IC_{50}$ value of levofloxacin or the compound J-131CP was calculated from the ratio of the cleaved DNA. The results are shown in Table 14.

$$\text{Total DNA amount} = S + L + N \text{ ... (Equation 1)}$$

$$\text{Amount of cleaved DNA} = L + N \text{ ... (Equation 2)}$$

$$\text{Ratio of substrate plasmid DNA (\%)} = S/(S + L + N) \times 100 \text{ ... (Equation 3)}$$

$$\text{Ratio of cleaved DNA amount (\%)} = (L + N)/(S + L + N) \times 100 \text{ ... (Equation 4)}$$

The assay was performed with the mutant GyrA(S84L) and GyrB(wt) in the same manner as that of the cleavage of the plasmid DNA with GyrA(wt) and GyrB(wt) mentioned above, provided that 0.15 μL of an enzyme solution of mutant GyrA(S84L) and 0.2 μL of the enzyme solution of GyrB(wt) were added instead of 0.15 μL of the enzyme solution of GyrA(wt) and 0.2 μL of the enzyme solution of GyrB(wt). The results of the agarose gel electrophoresis are shown in Fig. 2. In Fig. 2, the symbols "S", "L", and "N" have the same meanings as those explained for Figs 1. $IC_{50}$ value of levofloxacin or the compound J-131CP was also calculated from the visualized images shown in Figs. 1 and 2. The results are shown in Table 15.

[Table 14]

| Compound | Concentration [μg/mL] | GyrA(wt)+GyrB(wt) | | |
|---|---|---|---|---|
| | | Ratio of substrate plasmid DNA (%) | Ratio of cleaved DNA (%) | $IC_{50}$ (μg/mL) |
| J-131CP | 0 | 93 | 7 | 2 |
| | 0.125 | 88 | 12 | |
| | 0.25 | 81 | 19 | |
| | 0.5 | 77 | 23 | |
| | 1 | 53 | 47 | |
| | 2 | 23 | 77 | |
| | 4 | 19 | 81 | |
| Levofloxacin | 16 | 22 | 78 | Not determined |

[Table 15]

| Compound | Concentration [μg/mL] | GyrA(S84L)+GyrB(wt) | | |
|---|---|---|---|---|
| | | Ratio of substrate plasmid DNA (%) | Ratio of cleaved DNA (%) | $IC_{50}$ (μg/mL) |
| J-131CP | 0 | 95 | 5 | 1 |
| | 0.125 | 93 | 7 | |
| | 0.25 | 88 | 12 | |
| | 0.5 | 52 | 48 | |
| | 1 | 35 | 65 | |
| | 2 | 12 | 88 | |
| | 4 | 8 | 92 | |
| Levofloxacin | 16 | 92 | 8 | Not determined |

The DNA cleavage assay is a method for evaluating an inhibitory action of an agent for DNA gyrase by detecting whether or not DNAs cleaved by DNA gyrase are recombined. When the DNA gyrase inhibitory activity of the compound J-131CP is observed, the substrate plasmid DNA having the superhelix structure (shown as "S" in Figs. 1 and 2) decreases in a compound J-131CP concentration-dependent manner, and the ratios of the double-stranded substrate plasmid DNA in which one of the strands was cleaved (indicated with "N" in Figs. 1 and 2) and the double-stranded substrate plasmid DNA linearized by cleavage of the both strands (indicated with "L" in Figs. 1 and 2) correspondingly increase.

As seen from the results shown in Figs. 1 and 2, levofloxacin exhibited a potent inhibitory activity against the wild-type DNA gyrase at a concentration of 16 μg/mL (Fig. 1), whilst it exhibited almost no inhibitory activity against the mutant DNA gyrase (Fig. 2). In contrast, the compound J-131CP exhibited inhibitory activity against the wild-type DNA gyrase at a lower concentration compared with levofloxacin in a concentration-dependent manner (Fig. 1). The compound J-131CP exhibited a higher inhibitory activity against the mutant DNA gyrase (GyrA(S84L)) compared with the wild-type DNA gyrase (Fig. 2).

Further, as shown in Tables 14 and 15, the $IC_{50}$ values of the compound J-131CP indicating binding affinity at the time of formation of the complex with the DNA gyrase and the substrate plasmid DNA were 2 μg/mL (6.2 μM) when GyrA(wt) and GyrB(wt) were used as the enzyme solutions, and 1 μg/mL (3.1 μM) when GyrA(S84L) and GyrB(wt) were used. Therefore, it was demonstrated that the compound J-131CP has not only an inhibitory activity against the wild-type DNA gyrase, but also a stronger inhibitory activity against the mutant DNA gyrase (GyrA(S84L)), against which quinolones are ineffective.

Test Example 7

In the same manners as those of Test Examples 1 to 5, antibacterial activities and combination with PaβN, which is a drug efflux pump inhibitor, of the compounds represented by the structural formulas (26) to (44) were evaluated. The results are shown in Tables 16 and 17. The numerical values in the tables indicate MIC (mg/L), Res means resistant bacterium, and Sus means susceptible bacterium. When the compounds were used in combination with Phe-Arg-β-naphthylamide dihydrochloride (PAβN), which is a drug efflux pump inhibitor, for performing the antibacterial activity test with *Escherichia coli,* a 1,024 mg/L solution of flavone or quinolone was diluted twice with CAMHB mentioned above to adjust the concentration to 512 mg/L. This solution was diluted twice with CAMHB containing 160 mg/L of PaβN (produced Sigma-Aldrich) to a concentration of 256 mg/L, and this solution was diluted 11 times by 2-fold serial dilution with CAMHB containing 80 mg/L of PAβN to a concentration of 0.125 mg/L.

[Table 16]

| | | MIC(mg/L) | | | | | | | | | | | |
|---|---|---|---|---|---|---|---|---|---|---|---|---|---|
| | | Staphylococcus | aureus | Enterococcus faecalis | | Enterococcus faecium | | S. pneumoniae | | E. coli | | E coli plus PAβN 40mg/L | |
| | | Quinolone Res | Quinolone Sus | Quinolone Res | Quinolone Sus | Quinolone Res | Quinolone Sus | Quinolone Res | Quinolone Sus | Quinolone Res | Quinolone Sus | Quinolone Res | Quinolone Sus |
| | | Mu50 | FDA209P | 36-15722 | NCTC12201 | RPR2 | NCTC12202 | BCLSP37 | KBSP11 | strain 1708 | strain 1709 | strain 1708 | strain 1709 |
| J-131CP | | ≤0.06 | 0.25 | 1 | 0.5 | 4 | 8 | 4 | 2 | 128 | 8 | 0.25 | ≤0.06 |
| J-155 | 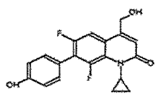 | 0.13 | 0.25 | 1 | 1 | 8 | 16 | 1 | 2 | 128 | 8 | 0.5 | ≤0.06 |
| J-156 | 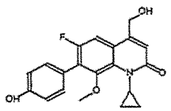 | 0.5 | 0.5 | 2 | 2 | nt | nt | 2 | 4 | 128 | 128 | 2 | 0.1 |
| J-166 | 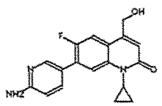 | 1 | 1 | 2 | 1 | 8 | 16 | 2 | 2 | 128 | 8 | 0.5 | ≤0.06 |
| J-173 | 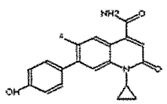 | 2 | 8 | nt | nt | nt | nt | 8 | 8 | 128 | 128 | nt | nt |
| J-175 | 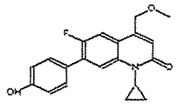 | 0.5 | 1 | nt | nt | nt | nt | 8 | 32 | 128 | 128 | nt | nt |
| J-131ACP | 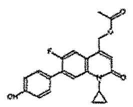 | 4 | 64 | nt | nt | nt | nt | nt | nt | 128 | 128 | nt | nt |
| J-176 | 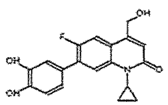 | 1 | 2 | 2 | 2 | 4 | 8 | 8 | 4 | 128 | 32 | nt | nt |

(continued)

| | | MIC(mg/L) | | | | | | | | | | | |
|---|---|---|---|---|---|---|---|---|---|---|---|---|---|
| | | Staphylococcus | aureus | Enterococcus faecalis | | Enterococcus faecium | | S. pneumoniae | | E. coli | | E coli plus PAβN 40mg/L | |
| | | Quinolone Res | Quinolone Sus | Quinolone Res | Quinolone Sus | Quinolone Res | Quinolone Sus | Quinolone Res | Quinolone Sus | Quinolone Res | Quinolone Sus | Quinolone Res | Quinolone Sus |
| | | Mu50 | FDA209P | 36-15722 | NCTC12201 | RPR2 | NCTC12202 | BCLSP37 | KBSP11 | strain 1708 | strain 1709 | strain 1708 | strain 1709 |
| J-131CP | | ≤0.06 | 0.25 | 1 | 0.5 | 4 | 8 | 4 | 2 | 128 | 8 | 0.25 | ≤0.06 |
| J-149A (or J-149L) | 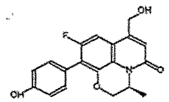 | 0.5 | 0.25 | nt | nt | nt | nt | nt | nt | 128 | 16 | nt | nt |
| J-149d | 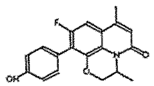 | 0.5 | 1 | nt | nt | nt | nt | nt | nt | 128 | 128 | nt | nt |

[Table 17]

| | | MIC(mg/L) | | | | | | | | | | | |
|---|---|---|---|---|---|---|---|---|---|---|---|---|---|
| | | Staphylococcus aureus | | Enterococcus faecalis | | Enterococcus faecium | | S. pneumoniae | | | E. Coli | E. coli plus | PAβN 40mg/L |
| | | Quinolone Res | Quinolone Sus | Quinolone Res | Quinolone Sus | Quinolone Res | Quinolone Sus | Quinolone Res | Quinolone Sus | Quinolone Res | Quinolone Sus | Quinolone Res | Quinolone Sus |
| | | Mu50 | FDA209P | 36-15722 | NCTC12201 | RPR2 | NCTC12302 | BCLSP37 | KBSP11 | strain 1708 | Strain 1709 | strain 1708 strain | strain 1709 strain 1708 |
| J-131CP | | ≤0.06 | 0.25 | 1 | 0.5 | 4 | 8 | 4 | 2 | 128 | 8 | 0.25 | ≤0.06 |
| J-165 | 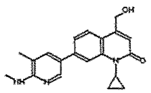 | 8 | 4 | nt | nt | nt | nt | nt | nt | 128 | 128 | nt | nt |
| J-158 | 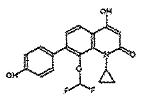 | 4 | 8 | nt | nt | nt | nt | nt | nt | 128 | 128 | nt | nt |
| J-162 | 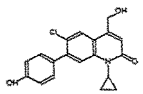 | 0.25 | 1 | nt | nt | nt | nt | nt | nt | 128 | 128 | nt | nt |
| J-161 | 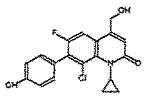 | 0.5 | 0.25 | 1 | 1 | 8 | 16 | 4 | 2 | 128 | 16 | 1 | 0.13 |
| J-167 | 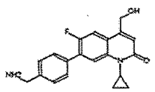 | 4 | 0.5 | 1 | 0.5 | 8 | 8 | 0.25 | 0.25 | 128 | 4 | 2 | 0.25 |
| J-168 | 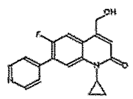 | 2 | 2 | nt | nt | nt | nt | nt | nt | 128 | 16 | 0.5 | ≤0.06 |
| J-169 | 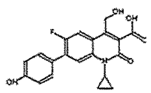 | 8 | 8 | nt | nt | nt | nt | nt | nt | 128 | 16 | nt | nt |
| J-174 | 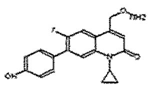 | 1 | 2 | 8 | 4 | nt | nt | nt | nt | 128 | 64 | 4 | 0.5 |

(continued)

| | | MIC(mg/L) | | | | | | | | | | | |
|---|---|---|---|---|---|---|---|---|---|---|---|---|---|
| | | Staphylococcus aureus | | Enterococcus faecalis | | Enterococcus faecium | | S. pneumoniae | | | E. Coli | E. coli plus | PAβN 40mg/L |
| | | Quinolone Res | Quinolone Sus | Quinolone Res | Quinolone Sus | Quinolone Res | Quinolone Sus | Quinolone Res | Quinolone Sus | Quinolone Res | Quinolone Sus | Quinolone Res | Quinolone Sus |
| | | Mu50 | FDA209P | 36-15722 | NCTC12201 | RPR2 | NCTC12302 | BCLSP37 | KBSP11 | strain 1708 | Strain 1709 | strain 1708 strain | strain 1709 strain 1708 |
| J-131CP | | ≤0.06 | 0.25 | 1 | 0.5 | 4 | 8 | 4 | 2 | 128 | 8 | 0.25 | ≤0.06 |
| J-179 | 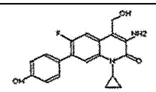 | 1 | 0.5 | nt | nt | nt | nt | nt | nt | 128 | 128 | nt | nt |
| J-144M | 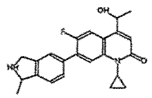 | 4 | 0.5 | 2 | 1 | nt | nt | nt | nt | 128 | 4 | nt | nt |

Test Example 8

Antibacterial activities againts *Escherichia coli* of the compounds shown Table 19 were examined by the following method.

*E. coli* 1708 having *parC* gene and *gyr*A gene encoding topoisomerase IV and DNA gyrase, respectively, of which amino acid sequences have the mutations shown in Table 16 (Miroku Medical Laboratory, 659-2, Innai, Saku-shi, Nagano-ken), and *E. coli* 1709 of which amino acid sequences of topoisomerase IV and DNA gyrase are the same as those of the prototype strain (K-12 MG1655) (Miroku Medical Laboratory) were each cultured overnight at 37°C in 4 mL of TBS (produced by Dickinson) with shaking. After completion of the culture, the cells were suspended in fresh TBS to prepare a cell suspension showing an absorbance of 0.3 at 578 nm. Then, the cell suspension was diluted 1,000 times with CAMHB prepared in Test Example 1. In Table 18, the positions of the amino acids counted from the amino terminus of the topoisomerase IV or DNA gyrase and amino acid sequences thereof are shown. For example, the indication "80Ser(AGT)" for *parC* of the prototype strain of *Escherichia coli,* K-12 MG1655, means that the 80th amino acid of the topoisomerase IV counted from the amino terminus is Ser (AGT).

[Table 18]

| Strain | Country | Topoisomerase IV (gene: *parC*) | | DNA gyrase (gene: *gyrA*) | |
|---|---|---|---|---|---|
| K-12 MG1655 * | | 80Ser(AGC) | 84Glu(GAA) | 83Ser(TCG) | 187Asp(GAC) |
| 1708 | Japan | 80Ile(ATT) | 84Val(GTA) | 83Leu(TTG) | 87Asn(AAC) |
| 1709 | Japan | 80Ser(AGT) | 84Glu(GAA) | 83Ser(TCG) | 87Asp(GAC) |
| * Authentic Escherichia coli strain | | | | | |

[Table 19]

| | | S. aureus | | E.faecalis | | E. faecium | | S. pneumoniae | | Escherihia coli | | | |
|---|---|---|---|---|---|---|---|---|---|---|---|---|---|
| | | | | | | | | | | Without | it PAβN | With | PAβN |
| | Product | QR Mu50 | QS 209P | QR 36-15722 P(S80I), G(S84I) | QS NCTC1220 1 wild type | QR RPR2 P(S821), G(S84I, E88K) | QS NCTC1220 2 wild type | QR BCLSP37 P(S79F), G(E85K) | QS KBSP11 wild type | QR E. coli (#1708) | QS E.coli (#1709) | QR E. coli (#1708) | QS E.coli (#1709) |
| J-139 | 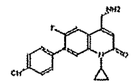 | 0.5 | 0.5 | 4 | 1 | 8 | 8 | 4 | 1 | 128 | 1 | 0.5 | ≤0.06 |
| J-144 | 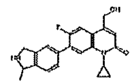 | 1 | 0.5 | 0.5 | 0.5 | 4 | 8 | 0.5 | 0.25 | 32 | 1 | 0.5 | ≤0.06 |
| J-132CP | 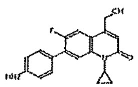 | 0.5 | 0.5 | 2 | 2 | 8 | 16 | 4 | 2 | 128 | 8 | 0.5 | ≤0.06 |
| J-154 | 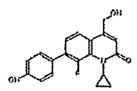 | 0.25 | 0.5 | 2 | 4 | 8 | 16 | 8 | 8 | 128 | 16 | 0.5 | ≤0.06 |

Industrial Applicability

The compounds represented by the general formula (I) and salts thereof of the present invention are useful as an active ingredient of a medicament for prophylactic and/or therapeutic treatment of various kinds of infectious diseases, especially infectious diseases caused by quinolone-resistant bacteria. The medicament of the present invention containing a combination of a compound represented by the general formula (I) or a salt thereof, and a quinolone has an advantage that it can exhibit antibacterial activity irrespective of type of bacterium that causes infectious diseases, and can also suppress emergence of quinolone-resistant bacteria, since the compound represented by the general formula (I) or a salt thereof acts on quinolone-resistant bacteria, and the quinolone mainly acts on quinolone-susceptible bacteria.

## Claims

**1.** A compound represented by the following general formula (I):

[Formula 1]

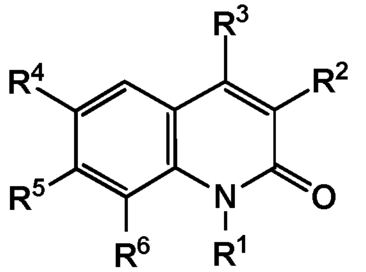

(I)

wherein, in the formula, $R^1$ represents a C1-C6 alkyl group which may have a substituent, a C1-C6 alkenyl group which may have a substituent, or an aryl group which may have a substituent, provided that $R^1$ may bind with $R^6$ to form a partially unsaturated or saturated heterocyclic ring containing one or two or more ring-constituting heteroatoms (the ring may have a substituent); $R^2$ represents hydrogen atom, carboxyl group, or amino group; $R^3$ represents hydroxy group, or a C1-C6 alkyl group which may have a substituent; $R^4$ represents hydrogen atom, a halogen atom, a C1-C6 alkyl group which may have a substituent, or a C1-C6 alkoxy group which may have a substituent, provided that $R^4$ may bind with $R^5$ to form a partially unsaturated or saturated heterocyclic ring containing one or two or more ring-constituting heteroatoms (the ring may have a substituent); $R^5$ represents a C1-C6 alkyl group which may have a substituent, a C1-C6 alkoxy group which may have a substituent, an aryl group which may have a substituent, a heteroaryl group which may have a substituent, or a partially saturated or saturated heterocyclic group which may have a substituent, provided that $R^5$ may bind with $R^6$ to form a partially unsaturated or saturated heterocyclic ring containing one or two or more ring-constituting heteroatoms (the ring may have a substituent); and $R^6$ represents hydrogen atom, a C1-C6 alkyl group which may have a substituent, a C1-C6 alkoxy group which may have a substituent, or a halogen atom, or a salt thereof.

2. The compound or a salt thereof according to claim 1, wherein $R^1$ is a C1-C6 alkyl group which may have a substituent, provided that $R^1$ may bind with $R^6$ to form a saturated 5- to 7-membered ring containing one or two or more ring-constituting heteroatoms (the ring may have a substituent); $R^2$ is hydrogen atom, carboxyl group, or amino group; $R^3$ is hydroxy group, a C1-C6 alkyl group, a hydroxy(C1-C6 alkyl) group which may have a substituent, or an amino(C1-C6 alkyl) group which may have a substituent; $R^4$ is hydrogen atom, a halogen atom, or a C1-C6 alkyl group which may have a substituent; $R^5$ is an aryl group which may have a substituent, or a partially saturated or saturated heterocyclic group which may have a substituent, provided that $R^5$ may bind with $R^6$ to form a partially unsaturated or saturated 5- to 7-membered ring containing one or two or more ring-constituting heteroatoms (the ring may have a substituent); and $R^6$ is hydrogen atom, a C1-C6 alkyl group which may have a substituent, a C1-C6 alkoxy group which may have a substituent, or a halogen atom.

3. The compound or a salt thereof according to claim 1, wherein $R^1$ is $-CH_3$,$-CH_2CH_3$, or cyclopropyl group, or may bind with $R^6$ to form a saturated 5- or 6-membered heterocyclic ring containing two ring-constituting heteroatoms (the ring may have one C1-C6 alkyl group as a substituent); $R^2$ is hydrogen atom, carboxyl group, or amino group; $R^3$ is hydroxy group, $-CH_3$, $-CH_2CH_3$, $-CH_2OH$, $-CH_2OCH_3$, $-CH_2NH_2$, $-C(O)NH_2$, $-CH_2OCOCH_3$, $-CH_2OCOCH_2CH_3$, $-CH_2OCOCH_2COOH_3$,$-CH_2OCOCH_2CH_2COOH$, $-CH_2OCH_3$, and $-CH_2ONH_2$; $R^4$ is hydrogen atom, fluorine atom, chlorine atom, or $-CH_3$; $R^5$ is phenyl group (the phenyl group may have one or two substituents selected from the group consisting of hydroxy group, amino group, a C1-C6 alkyl group, and aminomethyl group), pyridyl group (the pyridyl group may have one or two substituents selected from the group consisting of hydroxy group, amino group, a C1-C6 alkyl group, and aminomethyl group), piperazinyl group (the piperazinyl group may have one or two substituents selected from the group consisting of hydroxy group, amino group, a C1-C6 alkyl group, and aminomethyl group), piperidinyl group (the piperidinyl group may have one or two substituents selected from the group consisting of hydroxy group, amino group, a C1-C6 alkyl group, and aminomethyl group), or isoindolinyl group (the isoindolinyl group may have one or two substituents selected from the group consisting of hydroxy group, amino group, a C1-C6 alkyl group, and aminomethyl group, and may be partially saturated), or may bind with $R^6$ to form a saturated 5- or 6-membered heterocyclic ring containing two ring-constituting heteroatoms (the ring may have a C1-C6 alkyl group which may have a substituent as a substituent); and $R^6$ is hydrogen atom, a halogen atom, a C1-C6 alkyl group (the alkyl group may be substituted with fluorine atom), or a C1-C6 alkoxy group (the alkoxy group may be substituted with fluorine atom).

4. A medicament for prophylactic and/or therapeutic treatment of an infectious disease, which contains the compound or a salt thereof according to any one of claims 1 to 3 as an active ingredient.

5. The medicament according to claims 4, which is for prophylactic and/or therapeutic treatment of an infectious disease caused by a quinolone-resistant bacterium.

6. A medicament containing a combination of the compound or a salt thereof according to any one of claims 1 to 3, and a quinolone.

Fig. 1

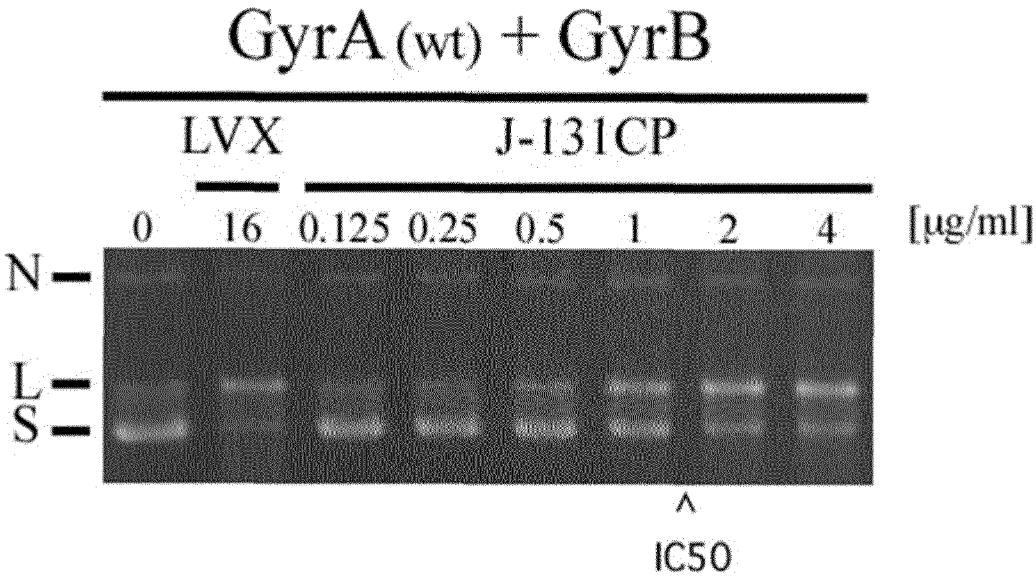
GyrA (wt) + GyrB
LVX
J-131CP
0
16
0.125
0.25
0.5
1
2
4
[μg/ml]
N
L
S
^
IC50

Fig. 2

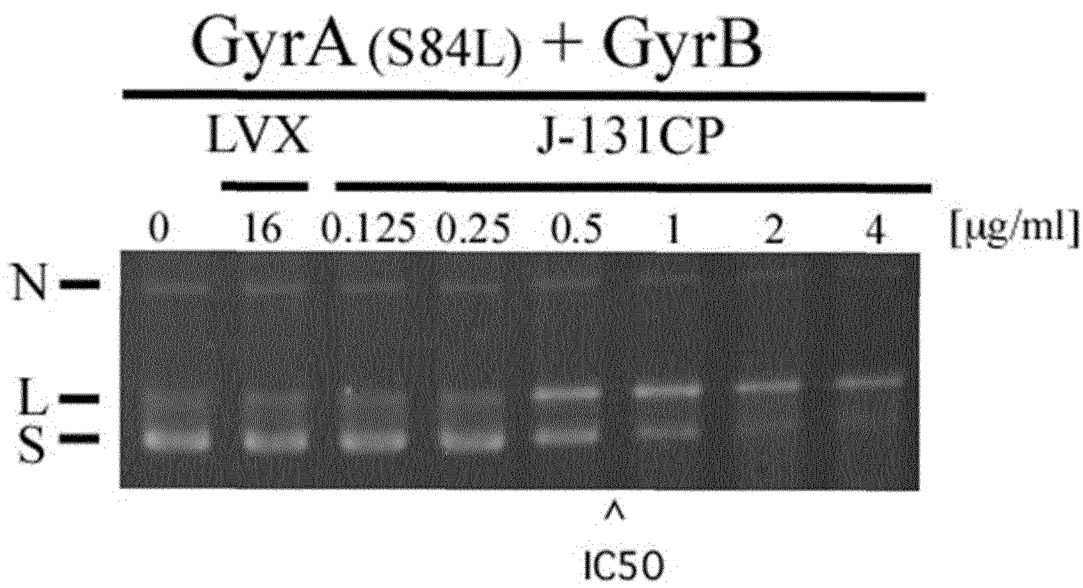
GyrA (S84L) + GyrB
LVX
J-131CP
0
16
0.125
0.25
0.5
1
2
4
[μg/ml]
N
L
S
^
IC50

**INTERNATIONAL SEARCH REPORT**

International application No.
PCT/JP2014/061004

A. CLASSIFICATION OF SUBJECT MATTER
*C07D215/22*(2006.01)i, *A61K31/4704*(2006.01)i, *A61K45/00*(2006.01)i, *A61P31/04*(2006.01)i, *A61P43/00*(2006.01)i, *C07D401/04*(2006.01)i, *C07D403/04*(2006.01)i, *C07D491/056*(2006.01)i, *C07D498/04*(2006.01)i,
According to International Patent Classification (IPC) or to both national classification and IPC

B. FIELDS SEARCHED

Minimum documentation searched (classification system followed by classification symbols)
C07D215/22, A61K31/4704, A61K45/00, A61P31/04, A61P43/00, C07D401/04, C07D403/04, C07D491/056, C07D498/04, C07D498/06, C12N9/99

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | MAITI, Arup et al., Synthesis of Casimiroin and Optimization of Its Quinone Reductase 2 and Aromatase Inhibitory Activities, Journal of Medicinal Chemistry, 2009, Vol.52, No.7, pp. 1873-1884 | 1-3 |
| X | MEISELS, Alex et al., The constituents of Casimiroa edulis. III. The structure of casimiroin, Journal of the American Chemical Society, 1957, Vol.79, pp.6328-6333 | 1-3 |
| X | CHILIN, Adriana et al., 4-Hydroxymethyl- and 4-methoxymethylfuro[2,3-h]quinolin-2(1H)-ones: synthesis and biological properties, Bioorganic & Medicinal Chemistry, 2003, Vol.11, No.7, pp. 1311-1318 | 1-3 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 July, 2014 (03.07.14) | 15 July, 2014 (15.07.14) |
| Name and mailing address of the ISA/ Japanese Patent Office | Authorized officer |
| Facsimile No. | Telephone No. |

Form PCT/ISA/210 (second sheet) (July 2009)

**INTERNATIONAL SEARCH REPORT**

International application No.
PCT/JP2014/061004

C (Continuation). DOCUMENTS CONSIDERED TO BE RELEVANT

| Category* | Citation of document, with indication, where appropriate, of the relevant passages | Relevant to claim No. |
|---|---|---|
| X | MARZANO, Cristina et al., 1,4,8-Trimethylfuro [2,3-H]quinolin-2(1H)-one, a new furocoumarin bioisoster, European Journal of Medicinal Chemistry, 2004, Vol.39, No.5, pp.411-419 | 1-3 |
| X | MOHAMED, E. A. et al., Synthesis of some 1,10-phenanthroline derivatives, Journal of the Indian Chemical Society, 1995, Vol.72, No.2, pp.93-97 | 1-3 |
| X | WO 2006/137485 A1 (Toyama Chemical Co., Ltd.), 28 December 2006 (28.12.2006), entire text; particularly, claims; examples 1, 2, 6, 7, 14 to 18, 38, 39, 52 to 57, 98, 100 to 105, 118 to 129, 142, 143, 148 to 155, 190; referential examples 2 to 4, 10, 11, 15, 52, 84, 85, 93 to 98, 100, 101, 103, 104, 111, 120 to 122, 124, 125, 129, 130, 141, 164, 165, 198, 199<br>& US 2010/0168418 A1 & US 2012/0214990 A1<br>& EP 1900732 A1 & EP 2468743 A1 | 1,2,4-6 |
| X | PATHAK, L. P. et al., 2-nitro-6,11-dioxo-6,11-dihydro-5H-dibenzo[b,e]azepin-4-yl-alkyl-arylamides/imides as possible hypoglycemic agents, Indian Journal of Heterocyclic Chemistry, 2008, Vol.17, No.4, pp.373-374 | 1,2 |
| X | JP 2013-43859 A (Microbial Chemistry Research Foundation), 04 March 2013 (04.03.2013), entire text; particularly, claims; structural formulae (1) to (3)<br>(Family: none) | 1,4-6 |
| X | WO 2011/058923 A1 (Microbial Chemistry Research Foundation), 19 May 2011 (19.05.2011), entire text; particularly, claims; structural formulae (1), (2)<br>& US 2012/0258980 A1 & EP 2500022 A1 | 1,4-6 |
| X | NADZAN, Alex M. et al., Hydroxynybomycin: isolation, structure and bioactivity, Journal of Antibiotics, 1977, Vol.30, No.6, pp.523-524 | 1,4-6 |
| X | WO 2012/108376 A1 (Daiichi Sankyo Co., Ltd.), 16 August 2012 (16.08.2012), entire text; particularly, claims; example 38; referential example 93<br>& EP 2674430 A1 & TW 201309689 A | 1,4-6 |

Form PCT/ISA/210 (continuation of second sheet) (July 2009)

**INTERNATIONAL SEARCH REPORT**

International application No.
PCT/JP2014/061004

C (Continuation). DOCUMENTS CONSIDERED TO BE RELEVANT

| Category* | Citation of document, with indication, where appropriate, of the relevant passages | Relevant to claim No. |
|---|---|---|
| X | EBLE, T. E. et al., Nybomycin: isolation, properties, and derivatives, Antibiotics and Chemotherapy (Washington, D. C.), 1958, Vol.8, pp.627-630 | 1,4-6 |
| X | JP 2003-146972 A (Teikoku Hormone Mfg. Co., Ltd.), 21 May 2003 (21.05.2003), entire text; particularly, examples 1-027, 1-028, 1-036 to 1-061, 1-065, 1-066, 1-069, 1-072, 2-007 to 2-010, 2-016 to 2-018, 4-013, 5-001 to 5-005, 9-010, 9-083, 9-084, 9-091 to 9-116, 9-122 to 9-129, 10-013, 10-250, 10-251, 10-261 to 307 (Family: none) | 1 |
| X | EP 2130831 A1 (INTERMED DISCOVERY GMBH), 09 December 2009 (09.12.2009), entire text; particularly, examples 1, 2, 4 (Family: none) | 1 |
| X | JP 9-507879 A (Molecular Probes, Inc.), 12 August 1997 (12.08.1997), entire text; particularly, example 2 & US 5658751 A & EP 740689 A & WO 1996/013552 A2 & CA 2179284 A | 1 |
| X | US 2003/0105124 A1 (PFIZER INC.), 05 June 2003 (05.06.2003), entire text; particularly, example 15(ii) (Family: none) | 1 |

Form PCT/ISA/210 (continuation of second sheet) (July 2009)

**INTERNATIONAL SEARCH REPORT**

International application No.
PCT/JP2014/061004

Continuation of A. CLASSIFICATION OF SUBJECT MATTER
(International Patent Classification (IPC))

*C07D498/06*(2006.01)i, *C12N9/99*(2006.01)i

(According to International Patent Classification (IPC) or to both national classification and IPC)

Form PCT/ISA/210 (extra sheet) (July 2009)

INTERNATIONAL SEARCH REPORT

International application No.
PCT/JP2014/061004

Subject to be covered by this search:

Claim 1 relates to compounds represented by general formula (I), and compounds represented by general formula (I) include a very large number of compounds.

However, only a few compounds among the claimed compounds are supported by the description within the meaning of PCT Article 6 and are disclosed within the meaning of PCT Article 5.

Consequently, this international search has been carried out on those supported and disclosed by the description, namely compounds represented by general formula (I) wherein R5 is an optionally substituted aryl group, or an optionally substituted partially saturated or saturated heterocyclic group, or alternatively, R5 and R6 combine together to form a partially unsaturated or saturated five- to seven-membered ring that contains one or more ring member heteroatoms.

Further, the search has been perfectly carried out with respect to compounds set forth in claims 2 and 3.

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008502720 A


### Non-patent literature cited in the description


- *J. Antimicrob. Chemother.,* 1997, vol. 40 (1), 135-136
- *Antimicrob. Agents Chemother.,* 2009, vol. 53 (11), 4580-4587
- *Int. J. Antimicrob. Agents,* 2012, vol. 39 (6), 478-485
- **XIAN-ZHI LI ; HIROSHI NIKAIDO.** *Drugs,* 2009, vol. 69, 1555-1623
- *Antibiotics,* 2013, vol. 2, 28-45
- *Journal of Antimicrobial Chemotherapy,* 2007, vol. 59, 1223-1229
- *Bioorganic & Medicinal Chemistry,* 2011, vol. 19, 7679-7689
- *Antimicrobial Agents and Chemotherapy,* 2004, vol. 48, 4171-4176
- *Journal of Antimicrobial Chemotherapy,* 2007, vol. 59, 1247-1260
- *Antimicrobial Agents and Chemotherapy,* 2008, vol. 52, 3202-3209
- *Journal of the Japanese Society of Chemotherapy,* 2004, vol. 52 (7), 355-360
- *J. Antimicrob. Agents.,* 2012, vol. 39 (6), 478-485
- *Antimicrob. Agents Chemother.,* 1998, vol. 42 (8), 1917-1922
- *Clin. Microbiol.,* 2002, vol. 40 (11), 4289-4294
- *Journal of the Japanese Society of Chemotherapy,* 2008, vol. 56 (1), 16-20
- *J. Antimicrob. Agents,* 2012, vol. 39 (6), 478-485
- *Methods Mol., Med.,* 2008, vol. 142, 11-23
- *Antimicrob. Agents Chemother.,* 1997, vol. 41, 2362-2366
- *Int. J. Antimicrob. Agents,* 2012, vol. 39 (6), 478-85
- *Antimicrob. Agents Chemother.,* 1999, vol. 43 (5), 1129-36

SEQUENCELISTING

<110> JUNTENDO EDUCATIONAL FOUNDATION
<120> Novel Compound having antibacterial actibity
<130> 131895M
<150> JP2013-088008
<151> 2013-04-19
<150> JP2013-184213
<151> 2013-09-05
<160> 2
<170> Patent Inversion 3.5
<210> 1
<211> 2670
<212> DNA
<213> S. aureus
<400> 1

```
atggctgaat tacctcaatc aagaataaat gaacgaaata ttaccagtga aatgcgtgaa      60

tcatttttag attatgcgat gagtgttatc gttgctcgtg cattgccaga tgttcgtgac     120

ggtttaaaac cagtacatcg tcgtatacta tatggattaa atgaacaagg tatgacaccg     180

gataaatcat ataaaaaatc agcacgtatc gttggtgacg taatgggtaa atatcacccc t   240

catggtgact catctattta tgaagcaatg gtacgtatgg ctcaagattt cagttatcgt     300

tatccgcttg ttgatggcca aggtaacttt ggttcaatgg atggagatgg cgcagcagca     360

atgcgttata ctgaagcgcg tatgactaaa atcacacttg aactgttacg tgatattaat     420

aaagatacaa tagattttat cgataactat gatggtaatg aaagagagcc gtcagtctta     480

cctgctcgat tccctaactt gttagccaat ggagcatcag gtatagcggt aggtatggca     540

acgaatattc caccacataa cttaacagaa ttaatcaatg gtgtacttag cttaagtaag     600

aaccctgata tttcaattgc tgagttaatg gaggatattg aaggtcctga tttcccaact     660

gctggactta ttttaggtaa gagtggtatt agacgtgcat atgaaacagg tcgtggttca     720

attcaaatgc gttctcgtgc agttattgaa gaacgtggag gcggacgtca acgtattgtt     780

gtcactgaaa ttcctttcca agtgaataag gctcgtatga ttgaaaaaat tgcagagctc     840

gttcgtgaca agaaaattga cggtatcact gatttacgtg atgaaacaag tttacgtact     900

ggtgtgcgtg tcgttattga tgtgcgtaag gatgcaaatg ctagtgtcat tttaaataac     960

ttatacaaac aaacacctct tcaaacatca tttggtgtga atatgattgc acttgtaaat    1020

ggtagaccga agcttattaa tttaaaagaa gcgttggtac attatttaga gcatcaaaag    1080

acagttgtta gaagacgtac gcaatacaac ttacgtaaag ctaaagatcg tgcccacatt    1140

ttagaaggat tacgtatcgc acttgaccat atcgatgaaa ttatttcaac gattcgtgag    1200

tcagatacag ataaagttgc aatggaaagc ttgcaacaac gcttcaaact ttctgaaaaa    1260

caagctcaag ctattttaga catgcgttta agacgtctaa caggtttaga gagagacaaa    1320

attgaagctg aatataatga gttattaaat tatattagtg aattagaaac aatcttagct    1380

gatgaagaag tattactaca attagttaga gatgaattaa cagaaattcg agatcgtttc    1440
```

ggtgatgatc gtcgtactga aatccaatta ggtggatttg aagatttaga agatgaagat 1500

ctcattccag aagaacaaat tgtaattaca ctaagccata ataactacat taaacgtttg 1560

ccggtatcta catatcgtgc tcaaaaccgt ggtggtcgtg gtgttcaagg tatgaataca 1620

ttggaagaag attttgtcag tcaattggta actttaagta cacatgacca tgtattgttc 1680

tttactaaca aaggtcgtgt atacaaactt aaaggttatg aagtgcctga gttatcaaga 1740

cagtctaaag gtattcctgt agtgaatgct attgaacttg aaaatgatga agtcattagt 1800

acaatgattg ctgttaaaga ccttgaaagt gaagacaact tcttagtgtt tgcaactaaa 1860

cgtggtgtcg ttaaacgttc agcattaagt aacttctcaa gaataaatag aaatggtaag 1920

attgcgattt cgttcagaga agatgatgag ttaattgcag ttcgcttaac aagtggtcaa 1980

gaagatatct tgattggtac atcacatgca tcattaattc gattccctga atcaacatta 2040

cgtcctttag gccgtacagc aacgggtgtg aaaggtatta cacttcgtga aggtgacgaa 2100

gttgtagggc ttgatgtagc tcatgcaaac agtgttgatg aagtattagt agttactgaa 2160

aatggttatg gtaaacgtac gccagttaat gactatcgtt tatcaaatcg tggtggtaaa 2220

ggtattaaaa cagctacgat tactgagcgt aatggtaatg ttgtatgtat cactacagta 2280

actggtgaag aagatttaat gattgttact aatgcaggtg tcattattcg actagatgtt 2340

gcagatattt ctcaaaatgg tcgtgcagca caaggtgttc gcttaattcg cttaggtgat 2400

gatcaatttg tttcaacggt tgctaaagta aaagaagatg cagaagatga aacgaatgaa 2460

gatgagcaat ctacttcaac tgtatctgaa gatggtactg aacaacaacg tgaagcggtt 2520

gtaaatgatg aaacaccagg aaatgcaatt catactgaag tgattgattc agaagaaaat 2580

gatgaagatg gacgtattga agtaagacaa gatttcatgg atcgtgttga agaagatata 2640

caacaatcat cagatgaaga tgaagaataa 2670

<210> 2
<211> 1935
<212> DNA
<213> S. aureus
<400> 2

atggtgactg cattgtcaga tgtaaacaac acggataatt atggtgctgg gcaaatacaa 60

gtattagaag gtttagaagc agtacgtaaa agaccaggta tgtatatagg atcgacttca 120

gagagaggtt tgcaccattt agtgtgggaa attgtcgata atagtatcga tgaagcatta 180

gctggttatg caaataaaat tgaagttgtt attgaaaaag ataactggat taaagtaacg 240

gataacggac gtggtatccc agttgatatt caagaaaaaa tgggacgtcc agctgtcgaa 300

gttattttaa ctgttttaca tgctggtggt aaattcggcg gtggcggata caaagtatct 360

ggtggtttac atggtgttgg ttcatcagtt gtaaacgcat tgtcacaaga cttagaagta 420

tatgtacaca gaaatgagac tatatatcat caagcatata aaaaaggtgt acctcaattt 480

gacttaaaag aagttggcac aactgataag acaggtactg tcattcgttt taaagcagat 540

ggagaaatct tcacagagac aactgtatac aactatgaaa cattacagca gcgtattaga 600

gagcttgctt tcttaaacaa aggaattcaa atcacattaa gagatgaacg tgatgaagaa 660

aacgttagag aagactccta tcactatgag ggcggtatta aatcgtacgt tgagttattg 720

aacgaaaata aagaacctat tcatgatgag ccaatttata ttcatcaatc taaagatgat 780

attgaagtag aaattgcgat tcaatataac tcaggatatg ccacaaatct tttaacttac 840

gcaaataaca ttcatacgta tgaaggtggt acgcatgaag acggattcaa acgtgcatta 900

acgcgtgtct taaatagtta tggtttaagt agcaagatta tgaaagaaga aaaagataga 960

ctttctggtg aagatacacg tgaaggtatg acagcaatta tatctatcaa acatggtgat 1020

cctcaattcg aaggtcaaac gaagacaaaa ttaggtaatt ctgaagtgcg tcaagttgta 1080

gataaattat tctcagagca ctttgaacga tttttatatg aaaatccaca agtcgcacgt 1140

acagtggttg aaaaaggtat tatggcggca cgtgcacgtg ttgctgcgaa aaaagcgcgt 1200

gaagtaacac gtcgtaaatc agcgttagat gtagcaagtc ttccaggtaa attagccgat 1260

tgctctagta aaagtcctga agaatgtgag attttcttag tcgaagggga ctctgccggg 1320

gggtctacaa aatctggtcg tgactctaga acgcaggcga ttttaccatt acgaggtaag 1380

atattaaatg ttgaaaaagc acgattagat agaattttga ataacaatga aattcgtcaa 1440

atgatcacag catttggtac aggaatcggt ggcgactttg atctagcgaa agcaagatat 1500

cacaaaatcg tcattatgac tgatgccgat gtggatggag cgcatattag aacattgtta 1560

ttaacattct tctatcgatt tatgagaccg ttaattgaag caggctatgt gtatattgca 1620

cagccaccgt tgtataaact gacacaaggt aaacaaaagt attatgtata caatgatagg 1680

gaacttgata aacttaaatc tgaattgaat ccaacaccaa aatggtctat tgcacgatac 1740

aaaggtcttg gagaaatgaa tgcagatcaa ttatgggaaa caacaatgaa ccctgagcac 1800

cgcgctcttt tacaagtaaa acttgaagat gcgattgaag cggaccaaac atttgaaatg 1860

ttaatgggtg acgttgtaga aaaccgtaga caatttatag aagataatgc agtttatgca 1920

aacttagact tctaa 1935